# EUROPEAN PATENT SPECIFICATION

(11) **EP 1 379 273 B1**
(45) Date of publication and mention of the grant of the patent: **16.09.2009**
(21) Application number: 01274277.1
(22) Date of filing: 26.11.2001
(51) Int. Cl.: A61K 39/39, A61K 48/00

(54) **NUCLEIC ACID ADJUVANTS**
NUKLEINSÄUREADJUVANTIEN
ADJUVANTS COMPORTANT DES ACIDES NUCLEIQUES

(30) Priority: 27.11.2000 US 724315
(43) Date of publication of application: 14.01.2004
(73) Proprietor: Powderject Vaccines, Inc., New York, NY 10017-5755 (US)
(72) Inventor: HAYNES, Joel, R., Madison, WI 53711 (US); ARRINGTON, Joshua, E., Madison, WI 53711 (US)
(74) Representative: Ricol, David Gerard
(86) International application number: PCT/US2001/043151
(87) International publication number: WO 2003/004055

(56) References cited:
- US-A- 5 980 898
- US-A- 6 056 960
- SCHARTON-KERSTEN T ET AL: "Transcutaneous immunization with bacterial ADP-ribosylating exotoxins, subunits, and unrelated adjuvants" INFECTION AND IMMUNITY, AMERICAN SOCIETY FOR MICROBIOLOGY. WASHINGTON, US, vol. 68, no. 9, September 2000 (2000-09), pages 5306-5313, XP002230158 ISSN: 0019-9567
- GLENN G M ET AL: "Transcutaneous immunization with bacterial ADP-ribosylating exotoxins as antigens and adjuvants" INFECTION AND IMMUNITY, AMERICAN SOCIETY FOR MICROBIOLOGY. WASHINGTON, US, vol. 67, no. 3, March 1999 (1999-03), pages 1100-1106, XP002110054 ISSN: 0019-9567

### SEQUENCE LISTING

<110> HAYNES, Joel R.
   ARRINGTON, Joshua
<120> NUCLEIC ACID ADJUVANTS
<130> APF41.40
<140>
   <141> 2001-11-26
<160> 22
<170> PatentIn Ver. 2.1
<210> 1
   <211> 5500
   <212> DNA
   <213> pPJV2002 plasmid
<400> 1
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agcattgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagcta gtcgacataa atcaatattg gctattggcc attgcatacg 2280
   ttgtatctat atcataatat gtacatttat attggctcat gtccaatatg accgccatgt 2340
   tgacattgat tattgactag ttattaatag taatcaatta cggggtcatt agttcatagc 2400
   ccatatatgg agttccgcgt tacataactt acggtaaatg gcccgcctcg tgaccgccca 2460
   acgacccccg cccattgacg tcaataatga cgtatgttcc catagtaacg ccaataggga 2520
   ctttccattg acgtcaatgg gtggagtatt tacggtaaac tgcccacttg gcagtacatc 2580
   aagtgtatca tatgccaagt ccggccccct attgacgtca atgacggtaa atggcccgcc 2640
   tggcattatg cccagtacat gaccttacgg gactttccta cttggcagta catctacgta 2700
   ttagtcatcg ctattaccat ggtgatgcgg ttttggcagt acaccaatgg gcgtggatag 2760
   cggtttgact cacggggatt tccaagtctc caccccattg acgtcaatgg gagtttgttt 2820
   tggcaccaaa atcaacggga ctttccaaaa tgtcgtaata accccgcccc gttgacgcaa 2880
   atgggcggta ggcgtgtacg gtgggaggtc tatataagca gagctcgttt agtgaaccgt 2940
   cagatcgcct ggagacgcca tccacgctgt tttgacctcc atagaagaca ccgggaccga 3000
   tccagcctcc gcggccggga acggtgcatt ggaacgcgga ttccccgtgc caagagtgac 3060
   gtaagtaccg cctatagact ctataggcac acccctttgg ctcttatgca tgctatactg 3120
   tttttggctt ggggcctata cacccccgct ccttatgcta taggtgatgg tatagcttag 3180
   cctataggtg tgggttattg accattattg accactcccc tattggtgac gatactttcc 3240
   attactaatc cataacatgg ctctttgcca caactatctc tattggctat atgccaatac 3300
   tctgtccttc agagactgac acggactctg tatttttaca ggatggggtc ccatttatta 3360
   tttacaaatt cacatataca acaacgccgt cccccgtgcc cgcagttttt attaaacata 3420
   gcgtgggatc tccacgcgaa tctcgggtac gtgttccgga catgggctct tctccggtag 3480
   cggcggagct tccacatccg agccctggtc ccatgcctcc agcggctcat ggtcgctcgg 3540
   cagctccttg ctcctaacag tggaggccag acttaggcac agcacaatgc ccaccaccac 3600
   cagtgtgccg cacaaggccg tggcggtagg gtatgtgtct gaaaatgagc tcggagattg 3660
   ggctcgcacc gtgacgcaga tggaagactt aaggcagcgg cagaagaaga tgcaggcagc 3720
   tgagttgttg tattctgata agagtcagag gtaactcccg ttgcggtgct gttaacggtg 3780
   gagggcagtg tagtctgagc agtactcgtt gctgccgcgc gcgccaccag acataatagc 3840
   tgacagacta acagactgtt cctttccatg ggtcttttct gcagtcaccg tccaagcttg 3900
   caatcatgga tgcaatgaag agagggctct gctgtgtgct gctgctgtgt ggagcagtct 3960
   tcgtttcggc tagcaatgat gataagttat atcgggcaga ttctagacct cctgatgaaa 4020
   taaagcagtc aggtggtctt atgccaagag gacagagtga gtactttgac cgaggtactc 4080
   aaatgaatat caacctttat gatcatgcaa gaggaactca gacgggattt gttaggcacg 4140
   atgatggata tgtttccacc tcaattagtt tgagaagtgc ccacttagtg ggtcaaacta 4200
   tattgtctgg tcattctact tattatatat atgttatagc cactgcaccc aacatgttta 4260
   acgttaatga tgtattaggg gcatacagtc ctcatccaga tgaacaagaa gtttctgctt 4320
   taggtgggat tccatactcc caaatatatg gatggtatcg agttcatttt ggggtgcttg 4380
   atgaacaatt acatcgtaat aggggctaca gagatagata ttacagtaac ttagatattg 4440
   ctccagcagc agatggttat ggattggcag gtttccctcc ggagcataga gcttggaggg 4500
   aagagccgtg gattcatcat gcaccgccgg gttgtgggaa tgctccaaga tcatcgatga 4560
   gtaatacttg cgatgaaaaa acccaaagtc taggtgtaaa attccttgac gaataccaat 4620
   ctaaagttaa aagacaaata ttttcaggct atcaatctga tattgataca cataatagaa 4680
   ttaaggatga attatgagga tcctcgcaat ccctaggagg attaggcaag ggcttgagct 4740
   cacgctcttg tgagggacag aaatacaatc aggggcagta tatgaatact ccatggagaa 4800
   acccagatct acgtatgatc agcctcgact gtgccttcta gttgccagcc atctgttgtt 4860
   tgcccctccc ccgtgccttc cttgaccctg gaaggtgcca ctcccactgt cctttcctaa 4920
   taaaatgagg aaattgcatc gcattgtctg agtaggtgtc attctattct ggggggtggg 4980
   gtggggcagg acagcaaggg ggaggattgg gaagacaata gcaggcatgc tggggatgcg 5040
   gtgggctcta tggcttctga ggcggaaaga accagctggg gctcgacagc tcgactctag 5100
   aattcactgg ccgtcgtttt acaacgtcgt gactgggaaa accctggcgt tacccaactt 5160
   aatcgccttg cagcacatcc ccctttcgcc agctggcgta atagcgaaga ggcccgcacc 5220
   gatcgccctt cccaacagtt gcgcagcctg aatggcgaat ggcgcctgat gcggtatttt 5280
   ctccttacgc atctgtgcgg tatttcacac cgcatatggt gcactctcag tacaatctgc 5340
   tctgatgccg catagttaag ccagccccga cacccgccaa cacccgctga cgcgccctga 5400
   cgggcttgtc tgctcccggc atccgcttac agacaagctg tgaccgtctc cgggagctgc 5460
   atgtgtcaga ggttttcacc gtcatcaccg aaacgcgcga                       5500
<210> 2
   <211> 5089
   <212> DNA
   <213> pPJV2003 plasmid
<400> 2
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agcattgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagcta gtcgacataa atcaatattg gctattggcc attgcatacg 2280
   ttgtatctat atcataatat gtacatttat attggctcat gtccaatatg accgccatgt 2340
   tgacattgat tattgactag ttattaatag taatcaatta cggggtcatt agttcatagc 2400
   ccatatatgg agttccgcgt tacataactt acggtaaatg gcccgcctcg tgaccgccca 2460
   acgacccccg cccattgacg tcaataatga cgtatgttcc catagtaacg ccaataggga 2520
   ctttccattg acgtcaatgg gtggagtatt tacggtaaac tgcccacttg gcagtacatc 2580
   aagtgtatca tatgccaagt ccggccccct attgacgtca atgacggtaa atggcccgcc 2640
   tggcattatg cccagtacat gaccttacgg gactttccta cttggcagta catctacgta 2700
   ttagtcatcg ctattaccat ggtgatgcgg ttttggcagt acaccaatgg gcgtggatag 2760
   cggtttgact cacggggatt tccaagtctc caccccattg acgtcaatgg gagtttgttt 2820
   tggcaccaaa atcaacggga ctttccaaaa tgtcgtaata accccgcccc gttgacgcaa 2880
   atgggcggta ggcgtgtacg gtgggaggtc tatataagca gagctcgttt agtgaaccgt 2940
   cagatcgcct ggagacgcca tccacgctgt tttgacctcc atagaagaca ccgggaccga 3000
   tccagcctcc gcggccggga acggtgcatt ggaacgcgga ttccccgtgc caagagtgac 3060
   gtaagtaccg cctatagact ctataggcac acccctttgg ctcttatgca tgctatactg 3120
   tttttggctt ggggcctata cacccccgct ccttatgcta taggtgatgg tatagcttag 3180
   cctataggtg tgggttattg accattattg accactcccc tattggtgac gatactttcc 3240
   attactaatc cataacatgg ctctttgcca caactatctc tattggctat atgccaatac 3300
   tctgtccttc agagactgac acggactctg tatttttaca ggatggggtc ccatttatta 3360
   tttacaaatt cacatataca acaacgccgt cccccgtgcc cgcagttttt attaaacata 3420
   gcgtgggatc tccacgcgaa tctcgggtac gtgttccgga catgggctct tctccggtag 3480
   cggcggagct tccacatccg agccctggtc ccatgcctcc agcggctcat ggtcgctcgg 3540
   cagctccttg ctcctaacag tggaggccag acttaggcac agcacaatgc ccaccaccac 3600
   cagtgtgccg cacaaggccg tggcggtagg gtatgtgtct gaaaatgagc tcggagattg 3660
   ggctcgcacc gtgacgcaga tggaagactt aaggcagcgg cagaagaaga tgcaggcagc 3720
   tgagttgttg tattctgata agagtcagag gtaactcccg ttgcggtgct gttaacggtg 3780
   gagggcagtg tagtctgagc agtactcgtt gctgccgcgc gcgccaccag acataatagc 3840
   tgacagacta acagactgtt cctttccatg ggtcttttct gcagtcaccg tccaagcttg 3900
   caatcatgga tgcaatgaag agagggctct gctgtgtgct gctgctgtgt ggagcagtct 3960
   tcgtttcggc tagcacacct caaaatatta ctgatttgtg tgcagaatac cacaacacac 4020
   aaatatatac gctaaatgat aagatatttt cgtatacaga atctctagct ggaaaaagag 4080
   agatggctat cattactttt aagaatggtg caatttttca agtagaagta ccaggtagtc 4140
   aacatataga ttcacaaaaa aaagcgattg aaaggatgaa ggataccctg aggattgcat 4200
   atcttactga agctaaagtc gaaaagttat gtgtatggaa taataaaacg cctcatgcga 4260
   ttgccgcaat tagtatggca aattaaggat cctcgcaatc cctaggagga ttaggcaagg 4320
   gcttgagctc acgctcttgt gagggacaga aatacaatca ggggcagtat atgaatactc 4380
   catggagaaa cccagatcta cgtatgatca gcctcgactg tgccttctag ttgccagcca 4440
   tctgttgttt gcccctcccc cgtgccttcc ttgaccctgg aaggtgccac tcccactgtc 4500
   ctttcctaat aaaatgagga aattgcatcg cattgtctga gtaggtgtca ttctattctg 4560
   gggggtgggg tggggcagga cagcaagggg gaggattggg aagacaatag caggcatgct 4620
   ggggatgcgg tgggctctat ggcttctgag gcggaaagaa ccagctgggg ctcgacagct 4680
   cgactctaga attcactggc cgtcgtttta caacgtcgtg actgggaaaa ccctggcgtt 4740
   acccaactta atcgccttgc agcacatccc cctttcgcca gctggcgtaa tagcgaagag 4800
   gcccgcaccg atcgcccttc ccaacagttg cgcagcctga atggcgaatg gcgcctgatg 4860
   cggtattttc tccttacgca tctgtgcggt atttcacacc gcatatggtg cactctcagt 4920
   acaatctgct ctgatgccgc atagttaagc cagccccgac acccgccaac acccgctgac 4980
   gcgccctgac gggcttgtct gctcccggca tccgcttaca gacaagctgt gaccgtctcc 5040
   gggagctgca tgtgtcagag gttttcaccg tcatcaccga aacgcgcga 5089
<210> 3
   <211> 5488
   <212> DNA
   <213> plasmid pPJV2006
<400> 3
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agcattgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagcta gtcgacataa atcaatattg gctattggcc attgcatacg 2280
   ttgtatctat atcataatat gtacatttat attggctcat gtccaatatg accgccatgt 2340
   tgacattgat tattgactag ttattaatag taatcaatta cggggtcatt agttcatagc 2400
   ccatatatgg agttccgcgt tacataactt acggtaaatg gcccgcctcg tgaccgccca 2460
   acgacccccg cccattgacg tcaataatga cgtatgttcc catagtaacg ccaataggga 2520
   ctttccattg acgtcaatgg gtggagtatt tacggtaaac tgcccacttg gcagtacatc 2580
   aagtgtatca tatgccaagt ccggccccct attgacgtca atgacggtaa atggcccgcc 2640
   tggcattatg cccagtacat gaccttacgg gactttccta cttggcagta catctacgta 2700
   ttagtcatcg ctattaccat ggtgatgcgg ttttggcagt acaccaatgg gcgtggatag 2760
   cggtttgact cacggggatt tccaagtctc caccccattg acgtcaatgg gagtttgttt 2820
   tggcaccaaa atcaacggga ctttccaaaa tgtcgtaata accccgcccc gttgacgcaa 2880
   atgggcggta ggcgtgtacg gtgggaggtc tatataagca gagctcgttt agtgaaccgt 2940
   cagatcgcct ggagacgcca tccacgctgt tttgacctcc atagaagaca ccgggaccga 3000
   tccagcctcc gcggccggga acggtgcatt ggaacgcgga ttccccgtgc caagagtgac 3060
   gtaagtaccg cctatagact ctataggcac acccctttgg ctcttatgca tgctatactg 3120
   tttttggctt ggggcctata cacccccgct ccttatgcta taggtgatgg tatagcttag 3180
   cctataggtg tgggttattg accattattg accactcccc tattggtgac gatactttcc 3240
   attactaatc cataacatgg ctctttgcca caactatctc tattggctat atgccaatac 3300
   tctgtccttc agagactgac acggactctg tatttttaca ggatggggtc ccatttatta 3360
   tttacaaatt cacatataca acaacgccgt cccccgtgcc cgcagttttt attaaacata 3420
   gcgtgggatc tccacgcgaa tctcgggtac gtgttccgga catgggctct tctccggtag 3480
   cggcggagct tccacatccg agccctggtc ccatgcctcc agcggctcat ggtcgctcgg 3540
   cagctccttg ctcctaacag tggaggccag acttaggcac agcacaatgc ccaccaccac 3600
   cagtgtgccg cacaaggccg tggcggtagg gtatgtgtct gaaaatgagc tcggagattg 3660
   ggctcgcacc gtgacgcaga tggaagactt aaggcagcgg cagaagaaga tgcaggcagc 3720
   tgagttgttg tattctgata agagtcagag gtaactcccg ttgcggtgct gttaacggtg 3780
   gagggcagtg tagtctgagc agtactcgtt gctgccgcgc gcgccaccag acataatagc 3840
   tgacagacta acagactgtt cctttccatg ggtcttttct gcagtcaccg tccaagcttg 3900
   caatcatgga tgcaatgaag agagggctct gctgtgtgct gctgctgtgt ggagcagtct 3960
   tcgtttcggc tagcaatgat gataagttat atcgggcaga ttctagacct cctgatgaaa 4020
   taaagcagtc aggtggtctt atgccaagag gacagagtga gtactttgac cgaggtactc 4080
   aaatgaatat caacctttat gatcatgcaa gaggaactca gacgggattt gttaggcacg 4140
   atgatggata tgtttccacc tcaattagtt tgagaagtgc ccacttagtg ggtcaaacta 4200
   tattgtctgg tcattctact tattatatat atgttatagc cactgcaccc aacatgttta 4260
   acgttaatga tgtattaggg gcatacagtc ctcatccaga tgaacaagaa gtttctgctt 4320
   taggtgggat tccatactcc caaatatatg gatggtatcg agttcatttt ggggtgcttg 4380
   atgaacaatt acatcgtaat aggggctaca gagatagata ttacagtaac ttagatattg 4440
   ctccagcagc agatggttat ggattggcag gtttccctcc ggagcataga gcttggaggg 4500
   aagagccgtg gattcatcat gcaccgccgg gttgtgggaa tgctccaaga tcatcgatga 4560
   gtaatacttg cgatgaaaaa acccaaagtc taggtgtaaa attccttgac gaataccaat 4620
   ctaaagttaa aagacaaata ttttcaggct atcaatctga tattgataca cataatagaa 4680
   tttgaggatc ctcgcaatcc ctaggaggat taggcaaggg cttgagctca cgctcttgtg 4740
   agggacagaa atacaatcag gggcagtata tgaatactcc atggagaaac ccagatctac 4800
   gtatgatcag cctcgactgt gccttctagt tgccagccat ctgttgtttg cccctccccc 4860
   gtgccttcct tgaccctgga aggtgccact cccactgtcc tttcctaata aaatgaggaa 4920
   attgcatcgc attgtctgag taggtgtcat tctattctgg ggggtggggt ggggcaggac 4980
   agcaaggggg aggattggga agacaatagc aggcatgctg gggatgcggt gggctctatg 5040
   gcttctgagg cggaaagaac cagctggggc tcgacagctc gactctagaa ttcactggcc 5100
   gtcgttttac aacgtcgtga ctgggaaaac cctggcgtta cccaacttaa tcgccttgca 5160
   gcacatcccc ctttcgccag ctggcgtaat agcgaagagg cccgcaccga tcgcccttcc 5220
   caacagttgc gcagcctgaa tggcgaatgg cgcctgatgc ggtattttct ccttacgcat 5280
   ctgtgcggta tttcacaccg catatggtgc actctcagta caatctgctc tgatgccgca 5340
   tagttaagcc agccccgaca cccgccaaca cccgctgacg cgccctgacg ggcttgtctg 5400
   ctcccggcat ccgcttacag acaagctgtg accgtctccg ggagctgcat gtgtcagagg 5460
   ttttcaccgt catcaccgaa acgcgcga 5488
<210> 4
   <211> 5500
   <212> DNA
   <213> plasmid pPJV2004
<400> 4
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agcattgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagcta gtcgacataa atcaatattg gctattggcc attgcatacg 2280
   ttgtatctat atcataatat gtacatttat attggctcat gtccaatatg accgccatgt 2340
   tgacattgat tattgactag ttattaatag taatcaatta cggggtcatt agttcatagc 2400
   ccatatatgg agttccgcgt tacataactt acggtaaatg gcccgcctcg tgaccgccca 2460
   acgacccccg cccattgacg tcaataatga cgtatgttcc catagtaacg ccaataggga 2520
   ctttccattg acgtcaatgg gtggagtatt tacggtaaac tgcccacttg gcagtacatc 2580
   aagtgtatca tatgccaagt ccggccccct attgacgtca atgacggtaa atggcccgcc 2640
   tggcattatg cccagtacat gaccttacgg gactttccta cttggcagta catctacgta 2700
   ttagtcatcg ctattaccat ggtgatgcgg ttttggcagt acaccaatgg gcgtggatag 2760
   cggtttgact cacggggatt tccaagtctc caccccattg acgtcaatgg gagtttgttt 2820
   tggcaccaaa atcaacggga ctttccaaaa tgtcgtaata accccgcccc gttgacgcaa 2880
   atgggcggta ggcgtgtacg gtgggaggtc tatataagca gagctcgttt agtgaaccgt 2940
   cagatcgcct ggagacgcca tccacgctgt tttgacctcc atagaagaca ccgggaccga 3000
   tccagcctcc gcggccggga acggtgcatt ggaacgcgga ttccccgtgc caagagtgac 3060
   gtaagtaccg cctatagact ctataggcac acccctttgg ctcttatgca tgctatactg 3120
   tttttggctt ggggcctata cacccccgct ccttatgcta taggtgatgg tatagcttag 3180
   cctataggtg tgggttattg accattattg accactcccc tattggtgac gatactttcc 3240
   attactaatc cataacatgg ctctttgcca caactatctc tattggctat atgccaatac 3300
   tctgtccttc agagactgac acggactctg tatttttaca ggatggggtc ccatttatta 3360
   tttacaaatt cacatataca acaacgccgt cccccgtgcc cgcagttttt attaaacata 3420
   gcgtgggatc tccacgcgaa tctcgggtac gtgttccgga catgggctct tctccggtag 3480
   cggcggagct tccacatccg agccctggtc ccatgcctcc agcggctcat ggtcgctcgg 3540
   cagctccttg ctcctaacag tggaggccag acttaggcac agcacaatgc ccaccaccac 3600
   cagtgtgccg cacaaggccg tggcggtagg gtatgtgtct gaaaatgagc tcggagattg 3660
   ggctcgcacc gtgacgcaga tggaagactt aaggcagcgg cagaagaaga tgcaggcagc 3720
   tgagttgttg tattctgata agagtcagag gtaactcccg ttgcggtgct gttaacggtg 3780
   gagggcagtg tagtctgagc agtactcgtt gctgccgcgc gcgccaccag acataatagc 3840
   tgacagacta acagactgtt cctttccatg ggtcttttct gcagtcaccg tccaagcttg 3900
   caatcatgga tgcaatgaag agagggctct gctgtgtgct gctgctgtgt ggagcagtct 3960
   tcgtttcggc tagcaatggc gacaaattat accgtgctga ctctagaccc ccagatgaaa 4020
   taaaacgttc cggaggtctt atgcccagag ggcataatga gtacttcgat agaggaactc 4080
   aaatgaatat taatctttat gatcacgcga gaggaacaca aaccggcttt gtcagatatg 4140
   atgacggata tgtttccact tctcttagtt tgagaagtgc tcacttagca ggacagtcta 4200
   tattatcagg atattccact tactatatat atgttatagc gacagcacca aatatgttta 4260
   atgttaatga tgtattaggc gtatacagcc ctcacccata tgaacaggag gtttctgcgt 4320
   taggtggaat accatattct cagatatatg gatggtatcg tgttaatttt ggtgtgattg 4380
   atgaacgatt acatcgtaac agggaatata gagaccggta ttacagaaat ctgaatatag 4440
   ctccggcaga ggatggttac agattagcag gtttcccacc ggatcaccaa gcttggagag 4500
   aagaaccctg gattcatcat gcaccacaag gttgtggaaa ttcatcaaga acaattacag 4560
   gtgatacttg taatgaggag acccagaatc tgagcacaat atatctcagg aaatatcaat 4620
   caaaagttaa gaggcagata ttttcagact atcagtcaga ggttgacata tataacagaa 4680
   ttcgggatga attatgagga tcctcgcaat ccctaggagg attaggcaag ggcttgagct 4740
   cacgctcttg tgagggacag aaatacaatc aggggcagta tatgaatact ccatggagaa 4800
   acccagatct acgtatgatc agcctcgact gtgccttcta gttgccagcc atctgttgtt 4860
   tgcccctccc ccgtgccttc cttgaccctg gaaggtgcca ctcccactgt cctttcctaa 4920
   taaaatgagg aaattgcatc gcattgtctg agtaggtgtc attctattct ggggggtggg 4980
   gtggggcagg acagcaaggg ggaggattgg gaagacaata gcaggcatgc tggggatgcg 5040
   gtgggctcta tggcttctga ggcggaaaga accagctggg gctcgacagc tcgactctag 5100
   aattcactgg ccgtcgtttt acaacgtcgt gactgggaaa accctggcgt tacccaactt 5160
   aatcgccttg cagcacatcc ccctttcgcc agctggcgta atagcgaaga ggcccgcacc 5220
   gatcgccctt cccaacagtt gcgcagcctg aatggcgaat ggcgcctgat gcggtatttt 5280
   ctccttacgc atctgtgcgg tatttcacac cgcatatggt gcactctcag tacaatctgc 5340
   tctgatgccg catagttaag ccagccccga cacccgccaa cacccgctga cgcgccctga 5400
   cgggcttgtc tgctcccggc atccgcttac agacaagctg tgaccgtctc cgggagctgc 5460
   atgtgtcaga ggttttcacc gtcatcaccg aaacgcgcga                       5500
<210> 5
   <211> 5089
   <212> DNA
   <213> plasmid pPJV2005
<400> 5
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agcattgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagcta gtcgacataa atcaatattg gctattggcc attgcatacg 2280
   ttgtatctat atcataatat gtacatttat attggctcat gtccaatatg accgccatgt 2340
   tgacattgat tattgactag ttattaatag taatcaatta cggggtcatt agttcatagc 2400
   ccatatatgg agttccgcgt tacataactt acggtaaatg gcccgcctcg tgaccgccca 2460
   acgacccccg cccattgacg tcaataatga cgtatgttcc catagtaacg ccaataggga 2520
   ctttccattg acgtcaatgg gtggagtatt tacggtaaac tgcccacttg gcagtacatc 2580
   aagtgtatca tatgccaagt ccggccccct attgacgtca atgacggtaa atggcccgcc 2640
   tggcattatg cccagtacat gaccttacgg gactttccta cttggcagta catctacgta 2700
   ttagtcatcg ctattaccat ggtgatgcgg ttttggcagt acaccaatgg gcgtggatag 2760
   cggtttgact cacggggatt tccaagtctc caccccattg acgtcaatgg gagtttgttt 2820
   tggcaccaaa atcaacggga ctttccaaaa tgtcgtaata accccgcccc gttgacgcaa 2880
   atgggcggta ggcgtgtacg gtgggaggtc tatataagca gagctcgttt agtgaaccgt 2940
   cagatcgcct ggagacgcca tccacgctgt tttgacctcc atagaagaca ccgggaccga 3000
   tccagcctcc gcggccggga acggtgcatt ggaacgcgga ttccccgtgc caagagtgac 3060
   gtaagtaccg cctatagact ctataggcac acccctttgg ctcttatgca tgctatactg 3120
   tttttggctt ggggcctata cacccccgct ccttatgcta taggtgatgg tatagcttag 3180
   cctataggtg tgggttattg accattattg accactcccc tattggtgac gatactttcc 3240
   attactaatc cataacatgg ctctttgcca caactatctc tattggctat atgccaatac 3300
   tctgtccttc agagactgac acggactctg tatttttaca ggatggggtc ccatttatta 3360
   tttacaaatt cacatataca acaacgccgt cccccgtgcc cgcagttttt attaaacata 3420
   gcgtgggatc tccacgcgaa tctcgggtac gtgttccgga catgggctct tctccggtag 3480
   cggcggagct tccacatccg agccctggtc ccatgcctcc agcggctcat ggtcgctcgg 3540
   cagctccttg ctcctaacag tggaggccag acttaggcac agcacaatgc ccaccaccac 3600
   cagtgtgccg cacaaggccg tggcggtagg gtatgtgtct gaaaatgagc tcggagattg 3660
   ggctcgcacc gtgacgcaga tggaagactt aaggcagcgg cagaagaaga tgcaggcagc 3720
   tgagttgttg tattctgata agagtcagag gtaactcccg ttgcggtgct gttaacggtg 3780
   gagggcagtg tagtctgagc agtactcgtt gctgccgcgc gcgccaccag acataatagc 3840
   tgacagacta acagactgtt cctttccatg ggtcttttct gcagtcaccg tccaagcttg 3900
   caatcatgga tgcaatgaag agagggctct gctgtgtgct gctgctgtgt ggagcagtct 3960
   tcgtttcggc tagcgctccc cagtctatta cagaactatg ttcggaatat cgcaacacac 4020
   aaatatatac gataaatgac aagatactat catatacgga atcgatggca ggcaaaagag 4080
   aaatggttat cattacattt aagagcggcg caacatttca ggtcgaagtc ccgggcagtc 4140
   aacatataga ctcccaaaaa aaagccattg aaaggatgaa ggacacatta agaatcacat 4200
   atctgaccga gaccaaaatt gataaattat gtgtatggaa taataaaacc cccaattcaa 4260
   ttgcggcaat cagtatggaa aactagggat cctcgcaatc cctaggagga ttaggcaagg 4320
   gcttgagctc acgctcttgt gagggacaga aatacaatca ggggcagtat atgaatactc 4380
   catggagaaa cccagatcta cgtatgatca gcctcgactg tgccttctag ttgccagcca 4440
   tctgttgttt gcccctcccc cgtgccttcc ttgaccctgg aaggtgccac tcccactgtc 4500
   ctttcctaat aaaatgagga aattgcatcg cattgtctga gtaggtgtca ttctattctg 4560
   gggggtgggg tggggcagga cagcaagggg gaggattggg aagacaatag caggcatgct 4620
   ggggatgcgg tgggctctat ggcttctgag gcggaaagaa ccagctgggg ctcgacagct 4680
   cgactctaga attcactggc cgtcgtttta caacgtcgtg actgggaaaa ccctggcgtt 4740
   acccaactta atcgccttgc agcacatccc cctttcgcca gctggcgtaa tagcgaagag 4800
   gcccgcaccg atcgcccttc ccaacagttg cgcagcctga atggcgaatg gcgcctgatg 4860
   cggtattttc tccttacgca tctgtgcggt atttcacacc gcatatggtg cactctcagt 4920
   acaatctgct ctgatgccgc atagttaagc cagccccgac acccgccaac acccgctgac 4980
   gcgccctgac gggcttgtct gctcccggca tccgcttaca gacaagctgt gaccgtctcc 5040
   gggagctgca tgtgtcagag gttttcaccg tcatcaccga aacgcgcga             5089
<210> 6
   <211> 5488
   <212> DNA
   <213> plasmid pPJV2007
<400> 6
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agcattgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagcta gtcgacataa atcaatattg gctattggcc attgcatacg 2280
   ttgtatctat atcataatat gtacatttat attggctcat gtccaatatg accgccatgt 2340
   tgacattgat tattgactag ttattaatag taatcaatta cggggtcatt agttcatagc 2400
   ccatatatgg agttccgcgt tacataactt acggtaaatg gcccgcctcg tgaccgccca 2460
   acgacccccg cccattgacg tcaataatga cgtatgttcc catagtaacg ccaataggga 2520
   ctttccattg acgtcaatgg gtggagtatt tacggtaaac tgcccacttg gcagtacatc 2580
   aagtgtatca tatgccaagt ccggccccct attgacgtca atgacggtaa atggcccgcc 2640
   tggcattatg cccagtacat gaccttacgg gactttccta cttggcagta catctacgta 2700
   ttagtcatcg ctattaccat ggtgatgcgg ttttggcagt acaccaatgg gcgtggatag 2760
   cggtttgact cacggggatt tccaagtctc caccccattg acgtcaatgg gagtttgttt 2820
   tggcaccaaa atcaacggga ctttccaaaa tgtcgtaata accccgcccc gttgacgcaa 2880
   atgggcggta ggcgtgtacg gtgggaggtc tatataagca gagctcgttt agtgaaccgt 2940
   cagatcgcct ggagacgcca tccacgctgt tttgacctcc atagaagaca ccgggaccga 3000
   tccagcctcc gcggccggga acggtgcatt ggaacgcgga ttccccgtgc caagagtgac 3060
   gtaagtaccg cctatagact ctataggcac acccctttgg ctcttatgca tgctatactg 3120
   tttttggctt ggggcctata cacccccgct ccttatgcta taggtgatgg tatagcttag 3180
   cctataggtg tgggttattg accattattg accactcccc tattggtgac gatactttcc 3240
   attactaatc cataacatgg ctctttgcca caactatctc tattggctat atgccaatac 3300
   tctgtccttc agagactgac acggactctg tatttttaca ggatggggtc ccatttatta 3360
   tttacaaatt cacatataca acaacgccgt cccccgtgcc cgcagttttt attaaacata 3420
   gcgtgggatc tccacgcgaa tctcgggtac gtgttccgga catgggctct tctccggtag 3480
   cggcggagct tccacatccg agccctggtc ccatgcctcc agcggctcat ggtcgctcgg 3540
   cagctccttg ctcctaacag tggaggccag acttaggcac agcacaatgc ccaccaccac 3600
   cagtgtgccg cacaaggccg tggcggtagg gtatgtgtct gaaaatgagc tcggagattg 3660
   ggctcgcacc gtgacgcaga tggaagactt aaggcagcgg cagaagaaga tgcaggcagc 3720
   tgagttgttg tattctgata agagtcagag gtaactcccg ttgcggtgct gttaacggtg 3780
   gagggcagtg tagtctgagc agtactcgtt gctgccgcgc gcgccaccag acataatagc 3840
   tgacagacta acagactgtt cctttccatg ggtcttttct gcagtcaccg tccaagcttg 3900
   caatcatgga tgcaatgaag agagggctct gctgtgtgct gctgctgtgt ggagcagtct 3960
   tcgtttcggc tagcaatggc gacaaattat accgtgctga ctctagaccc ccagatgaaa 4020
   taaaacgttc cggaggtctt atgcccagat ggcataatga gtacttcgat agaggaactc 4080
   aaatgaatat taatctttat gatcacgcga gaggaacaca aaccggcttt gtcagatatg 4140
   atgacggata tgtttccact tctcttagtt tgagaagtgc tcacttagca ggacagtcta 4200
   tattatcagg atattccact tactatatat atgttatagc gacagcacca aatatgttta 4260
   atgttaatga tgtattaggc gtatacagcc ctcacccata tgaacaggag gtttctgcgt 4320
   taggtggaat accatattct cagatatatg gatggtatcg tgttaatttt ggtgtgattg 4380
   atgaacgatt acatcgtaac agggaatata gagaccggta ttacagaaat ctgaatatag 4440
   ctccggcaga ggatggttac agattagcag gtttcccacc ggatcaccaa gcttggagag 4500
   aagaaccctg gattcatcat gcaccacaag gttgtggaaa ttcatcaaga acaattacag 4560
   gtgatacttg taatgaggag acccagaatc tgagcacaat atatctcagg aaatatcaat 4620
   caaaagttaa gaggcagata ttttcagact atcagtcaga ggttgacata tataacagaa 4680
   tttgaggatc ctcgcaatcc ctaggaggat taggcaaggg cttgagctca cgctcttgtg 4740
   agggacagaa atacaatcag gggcagtata tgaatactcc atggagaaac ccagatctac 4800
   gtatgatcag cctcgactgt gccttctagt tgccagccat ctgttgtttg cccctccccc 4860
   gtgccttcct tgaccctgga aggtgccact cccactgtcc tttcctaata aaatgaggaa 4920
   attgcatcgc attgtctgag taggtgtcat tctattctgg ggggtggggt ggggcaggac 4980
   agcaaggggg aggattggga agacaatagc aggcatgctg gggatgcggt gggctctatg 5040
   gcttctgagg cggaaagaac cagctggggc tcgacagctc gactctagaa ttcactggcc 5100
   gtcgttttac aacgtcgtga ctgggaaaac cctggcgtta cccaacttaa tcgccttgca 5160
   gcacatcccc ctttcgccag ctggcgtaat agcgaagagg cccgcaccga tcgcccttcc 5220
   caacagttgc gcagcctgaa tggcgaatgg cgcctgatgc ggtattttct ccttacgcat 5280
   ctgtgcggta tttcacaccg catatggtgc actctcagta caatctgctc tgatgccgca 5340
   tagttaagcc agccccgaca cccgccaaca cccgctgacg cgccctgacg ggcttgtctg 5400
   ctcccggcat ccgcttacag acaagctgtg accgtctccg ggagctgcat gtgtcagagg 5460
   ttttcaccgt catcaccgaa acgcgcga                                    5488
<210> 7
   <211> 30
   <212> DNA
   <213> Artificial Sequence
<220>
   <223> Description of Artificial Sequence:synthetic construct
<400> 7
   ggagctagca atgatgataa gttatatcgg 30
<210> 8
   <211> 30
   <212> DNA
   <213> Artificial Sequence
<220>
   <223> Description of Artificial Sequence:synthetic construct
<400> 8
   cctggatcct cataattcat ccttaattct 30
   <210> 9
   <211> 30
   <212> DNA
   <213> Artificial Sequence
<220>
   <223> Description of Artificial Sequence:synthetic construct
<400> 9
   ggagctagca cacctcaaaa tattactgat 30
   <210> 10
   <211> 30
<212> DNA
   <213> Artificial Sequence
<220>
   <223> Description of Artificial Sequence:synthetic construct
<400> 10
   cctggatcct taatttgcca tactaattgc 30
   <210> 11
   <211> 30
   <212> DNA
   <213> Artificial Sequence
<220>
   <223> Description of Artificial Sequence:synthetic construct
<400> 11
   cctggatcct caaattctat tatgtgtatc 30
   <210> 12
   <211> 30
   <212> DNA
   <213> Artificial Sequence
<220>
   <223> Description of Artificial Sequence:synthetic construct
<400> 12
   ggagctagca atggcgacaa attataccgt 30
   <210> 13
   <211> 30.
   <212> DNA
   <213> Artificial Sequence
<220>
   <223> Description of Artificial Sequence:synthetic construct
<400> 13
   cctggatcct cataattcat cccgaattct 30
   <210> 14
   <211> 30
   <212> DNA
   <213> Artificial Sequence
<220>
   <223> Description of Artificial Sequence:synthetic construct
<400> 14
   ggagctagcg ctccccagtc tattacagaa 30
   <210> 15
   <211> 30
   <212> DNA
   <213> Artificial Sequence
<220>
   <223> Description of Artificial Sequence:synthetic construct
<400> 15
   cctggatccc tagttttcca tactgattgc 30
   <210> 16
   <211> 30
   <212> DNA
   <213> Artificial Sequence
<220>
   <223> Description of Artificial Sequence:synthetic construct
<400> 16
   cctggatcct caaattctgt tatatatgtc 30
<210> 17
   <211> 65
   <212> DNA
   <213> Artificial Sequence
<220>
   <223> Description of Artificial Sequence:synthetic construct
<400> 17
   cccaagcttc caccatgagc cttctaaccg aggtcgaaac acctatcaga aacgaatggg 60
   agtgc 65
<210> 18
   <211> 28
   <212> DNA
   <213> Artificial Sequence
<220>
   <223> Description of Artificial Sequence:synthetic construct
<400> 18
   cccggatcct tactccagct ctatgctg 28
   <210> 19
   <211> 17
   <212> PRT
   <213> Artificial Sequence
<220>
   <223> Description of Artificial Sequence:synthetic construct
<400> 19
   Ser Leu Leu Thr Glu Val Glu Thr Pro Ile Arg Asn Glu Trp Glu Cys
   1 5 10 15
   Arg
<210> 20
   <211> 12
   <212> PRT
   <213> Artificial Sequence
<220>
   <223> Description of Artificial Sequence:synthetic construct
<400> 20
   Ile Pro Gln Ser Leu Asp Ser Trp Trp Thr Ser Leu
   1 5 10
<210> 21
   <211> 15
   <212> PRT
   <213> Artificial Sequence
<220>
   <223> Description of Artificial Sequence:synthetic construct
<400> 21
   Arg Ile Gln Arg Gly Pro Gly Arg Ala Phe Val Ile Thr Gly Lys
     1               5                  10                  15
<210> 22
   <211> 10
   <212> PRT
   <213> Artificial Sequence
<220>
   <223> Description of Artificial Sequence:synthetic construct
<400> 22
   Arg Gly Pro Gly Arg Ala Phe Val Thr Ile
     1               5                  10

## Description

### Technical Field

The invention relates to the fields of molecular biology and immunology, and generally relates to nucleic acid immunization techniques. More specifically, the invention relates to polynucleotides encoding an adjuvant, and to immunization strategies employing such polynucleotides.

### Background

Techniques for the injection of DNA and mRNA into mammalian tissue for the purposes of immunization against an expression product have been described in the art. The techniques, termed "nucleic acid immunization" herein, have been shown to elicit both humoral and cell-mediated immune responses. For example, sera from mice immunized with a DNA construct encoding the envelope glycoprotein, gp160, were shown to react with recombinant gp160 in immunoassays, and lymphocytes from the injected mice were shown to proliferate in response to recombinant gp120. Wang et al. (1993) Proc. Natal. Acad. Sci. USA 90:4156-4160. Similarly, mice immunized with a human growth hormone (hGH) gene demonstrated an antibody-based immune response. Tang et al. (1992) Nature 356:152-154. Intramuscular injection of DNA encoding influenza nucleoprotein driven by a mammalian promoter has been shown to elicit a CD8+ CTL response that can protect mice against subsequent lethal challenge with virus. Ulmer et al. (1993) Science 259:1745-1749. Immunohistochemical studies of the injection site revealed that the DNA was taken up by myeloblasts, and cytoplasmic production of viral protein could be demonstrated for at least 6 months. US 5,980,898 is concerned with transcutaneous immunization.

### Summary of the Invention

It is a primary object of the invention to provide a polynucleotide adjuvant composition as defined in the appendant claims.

The first and second nucleic acid sequences may be present in the same or in different nucleic acid constructs. The truncated subunit coding regions may be obtained or derived from the same bacterial ADP-ribosylating exotoxin and, in certain preferred embodiments, the bacterial ADP-ribosylating exotoxin is a cholera toxin (CT) or an E. *coli* heat labile enterotoxin (LT). In addition, at least one of the truncated subunit coding regions may be genetically modified to detoxify the subunit peptide encoded thereby, for example wherein the truncated A subunit coding region has been genetically modified to disrupt or inactivate ADP-ribosyl transferase activity in the subunit peptide expression product.

It is also a primary object of the invention to provide a polynucleotide adjuvant composition containing first and second nucleic acid sequences, wherein the first nucleic acid sequence is a modified A subunit coding region obtained or derived from a bacterial ADP-ribosyliting exotoxin, and the second nucleic acid sequence is a B subunit coding region obtained or derived from a bacterial ADP-ribosylating exotoxin. The modified A subunit coding region and said B subunit coding region each encode a mature subunit peptide, and the modified A subunit coding region has been genetically modified so as to delete a C-terminal KDEL or RDEL motif in the subunit peptide encoded thereby.

As above, the first and second nucleic acid sequences may be present in the same or in different nucleic acid constructs. The truncated subunit coding regions may be obtained or derived from the same bacterial ADP-ribosylating exotoxin and, in certain preferred embodiments, the bacterial ADP-ribosylating exotoxin is a cholera toxin (CT) or an *E. coli* heat labile enterotoxin (LT). In addition, at least one of the truncated subunit coding regions may be genetically modified to detoxify the subunit peptide encoded thereby, for example wherein the truncated A subunit coding region has been genetically modified to disrupt or inactivate ADP-ribosyl transferase activity in the subunits peptide expression product.

In certain aspects of the invention, the above compositions can be provided in particulate form, for example wherein the compositions are particulates suitable for delivery from a particle delivery device. In this regard, the present compositions may be coated onto the same or a different core carrier particle and thus suitable for delivery using a particle-mediated transfection technique. Preferred core carrier particles will have an average diameter of about 0.1 to 10 µm, and may comprise a metal such as gold. Accordingly, it is a still further object of the invention to provide a particle delivery device loaded with (e.g., containing) a particulate composition as defined herein.

It is also a primary object of the invention to provide for the use of a composition containing a first and second nucleic acid sequence, where each sequence includes a coding region for a subunit from a bacterial ADP-ribosylating exotoxin in the manufacture of a medicament for enhancing an immune response in a vertebrate subject against an antigen of interest in the said subject. The antigen of interest and the composition are administered to the subject such that the toxin subunits encoded by the first and second nucleic acid sequences are expressed in an amount sufficient to elicit an enhanced immune response against the antigen. The first nucleic acid sequence contains a truncated A subunit coding region obtained or derived from a bacterial ADP-ribosylating exotoxin, and the second nucleic acid sequence contains a truncated B subunit coding region obtained or derived from a bacterial ADP-ribosylating exotoxin, however with the proviso that each of the truncated subunit coding regions has a 5' deletion and encodes a subunit peptide not having an amino terminal bacterial signal peptide.

It is a related primary object of the invention to provide a method for enhancing an immune response against an antigen of interest in a subject. The method generally entails: (a) administering the antigen of interest to the subject; (b) providing an adjuvant composition comprising first and second nucleic acid sequences, wherein the first nucleic acid sequence is a truncated A subunit coding region obtained or derived from a bacterial ADP-ribosylating exotoxin, and the second nucleic acid sequence is a truncated B subunit coding region obtained or derived from a bacterial ADP-ribosylating exotoxin; and (c) administering the adjuvant composition to the subject, whereby upon introduction to the subject, the first and second nucleic acid sequences are expressed to provide subunit peptides in an amount sufficient to elicit an enhanced immune response against the antigen of interest. The subunit coding regions are truncated in that each coding region has a 5' deletion and encodes a subunit peptide not having an amino terminal bacterial signal peptide.

It is yet a further primary object of the invention to provide for the use of a composition comprising a first and second nucleic acid sequence, where each sequence includes a coding region for a subunit from a bacterial ADP-ribosylating exotoxin in the manufacture of a medicament for enhancing an immune response in a vertebrate subject against an antigen of interest in the said subject. The antigen of interest and the composition are administered to the subject such that the toxin subunits encoded by the first and second nucleic acid sequences are expressed in an amount sufficient to elicit an enhanced immune response against the antigen. The first nucleic acid sequence contains a modified A subunit coding region obtained or derived from a bacterial ADP-ribosylating exotoxin, and the second nucleic acid sequence contains a B subunit coding region obtained or derived from a bacterial ADP-ribosylating exotoxin, however with the proviso that the modified A subunit coding region and B subunit coding region each encode a mature subunit peptide, and with the further proviso that the modified A subunit coding region has been genetically modified so as to delete a C-terminal KDEL or RDEL motif in the subunit peptide encoded thereby.

It is a related primary object of the invention to provide a method for enhancing an immune response against an antigen of interest in a subject, wherein the method entails: (a) administering the antigen of interest to the subject; (b) providing an adjuvant composition comprising first and second nucleic acid sequences, wherein the first nucleic acid sequence is a modified A subunit coding region obtained or derived from a bacterial ADP-ribosylating exotoxin, and the second nucleic acid sequence is a B subunit coding region obtained or derived from a bacterial ADP-ribosylating exotoxin; and (c) administering the adjuvant composition to the subject, whereby upon introduction to the subject, the first and second nucleic acid sequences are expressed to provide subunit peptides in an amount sufficient to elicit an enhanced immune response against the antigen of interest. The subunit coding regions are modified in that each encodes a mature subunit peptide, but the A subunit coding region has been genetically modified so as to delete a C-terminal KDEL or RDEL motif in the subunit peptide encoded thereby.

In the uses and methods of the invention, administering the adjuvant compositions entails transfecting cells of the subject with a polynucleotide adjuvant composition according to the present invention. Expression cassettes and/or vectors containing the nucleic acid molecules of the present invention can be used to transfect the cells, and transfection is carried out under conditions that permit expression of the subunit peptides within the subject. The method may further entail one or more steps of administering at least one secondary composition to the subject.

The transfection procedure carried out during the immunization can be conducted *either in vivo,* or *ex* vivo (e.g., to obtain transfected cells which are subsequently introduced into the subject prior to carrying out the secondary immunization step). When *in vivo* transfection is used, the nucleic acid molecules can be administered to the subject by way of intramuscular or intradermal injection of plasmid DNA or, preferably, administered to the subject using a particle-mediated delivery technique. Vaccine compositions (containing the antigen of interest) can be provided in the form of any suitable vaccine composition, for example, in the form of a peptide subunit composition, in the form of a nucleic acid vaccine composition, or in the form of a whole or split virus influenza vaccine composition.

In certain methods, the antigen of interest and the adjuvant composition are administered to the same site in the subject. For example, the adjuvant composition and the antigen of interest can be administered concurrently (e.g., provided in a single vaccine composition). In certain preferred embodiments, the adjuvant and, optionally the antigen of interest, is administered in particulate form, for example wherein the adjuvant composition has been coated onto a core carrier particle and delivered to the subject using a particle-mediated delivery technique.

In these methods, administration of the polynucleotide adjuvant compositions of the present invention preferably results in an augmented cellular immune response against the co-administered antigen of interest. Such an enhanced immune response may be generally characterized by increased titers of interferon-producing CD4⁺ and/or CD8⁺ T lymphocytes, increased antigen-specific cytotoxic T lymphocyte (CTL) activity, and a T helper 1-like immune response (Th1) against the antigen of interest (characterized by increased antigen-specific antibody titers of the subclasses typically associated with cellular immunity (e.g., IgG2a), usually with a concomitant reduction of antibody titers of the subclasses typically associated with humoral immunity (e.g., IgG1)) instead of a T helper 2-like immune response (Th2) such as that normally produced when immunizing a subject using a bacterial ADP-ribosylating exotoxin adjuvant such as CT or LT.

Advantages of the present invention include, but are not limited to the ability of the present adjuvant compositions to provide significant adjuvant effect and thereby enhance the immunogenicity of a co-administered antigen in an immunized subject, as well as the ability to favor a Th1-like immune responses against the co-administered antigen which is beneficial in a vaccine product.

These and other objects, aspects, embodiments and advantages of the present invention will readily occur to those of ordinary skill in the art in view of the disclosure herein.

### Brief Description of the Drawings

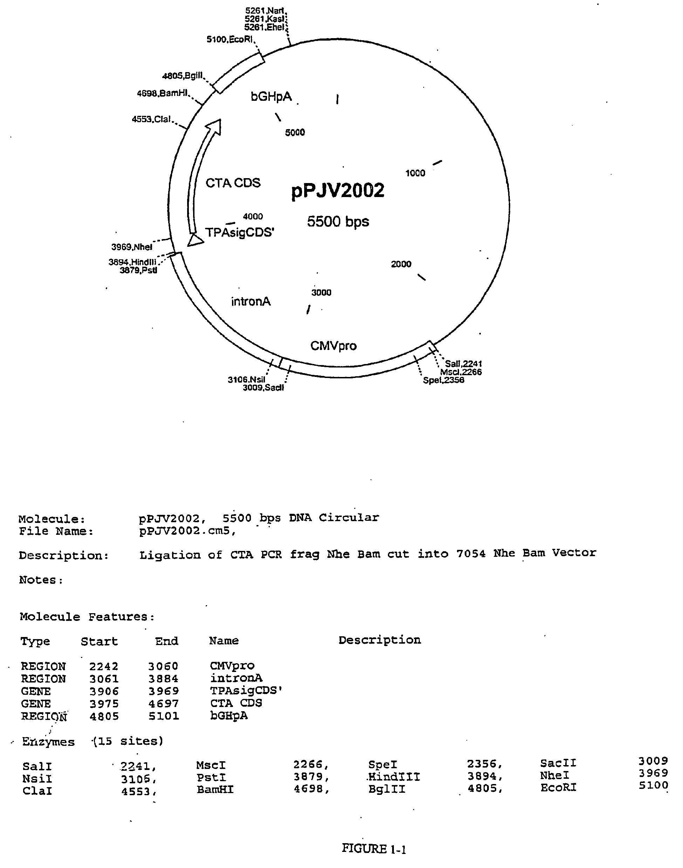

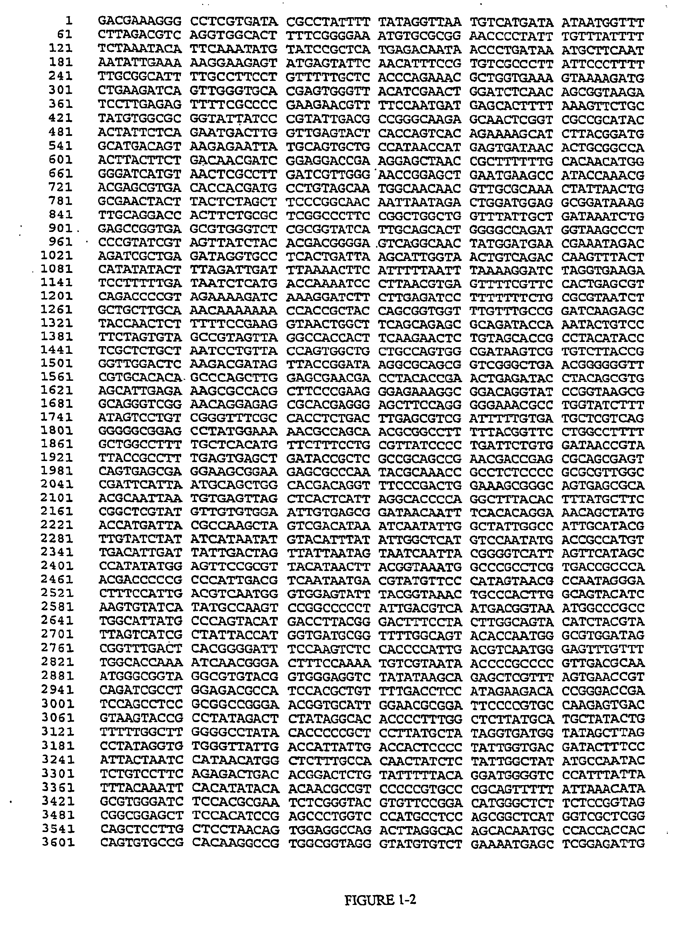

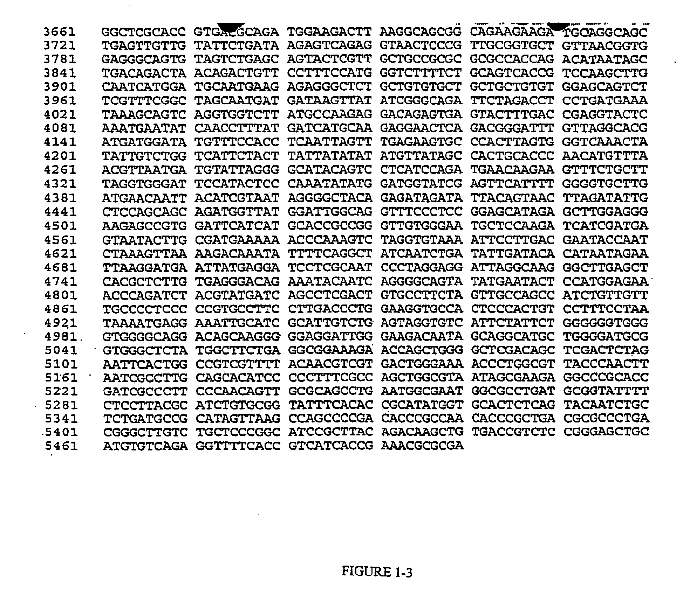

Figure 1 is a restriction map and functional map of plasmid pPJV2002 that contains a truncated coding sequence for a Cholera Toxin (CT) subunit A (CTA) peptide, wherein the plasmid further contains the human cytomegalovirus (hCMV) immediate early promoter and associated intron A sequence, and the coding sequence for the signal peptide of human tissue plasminogen activator to allow for secretion from mammalian cells of the truncated CTA expression product. The figure further contains the complete nucleic acid sequence (SEQ ID NO: 1) for the pPJV2002 plasmid.

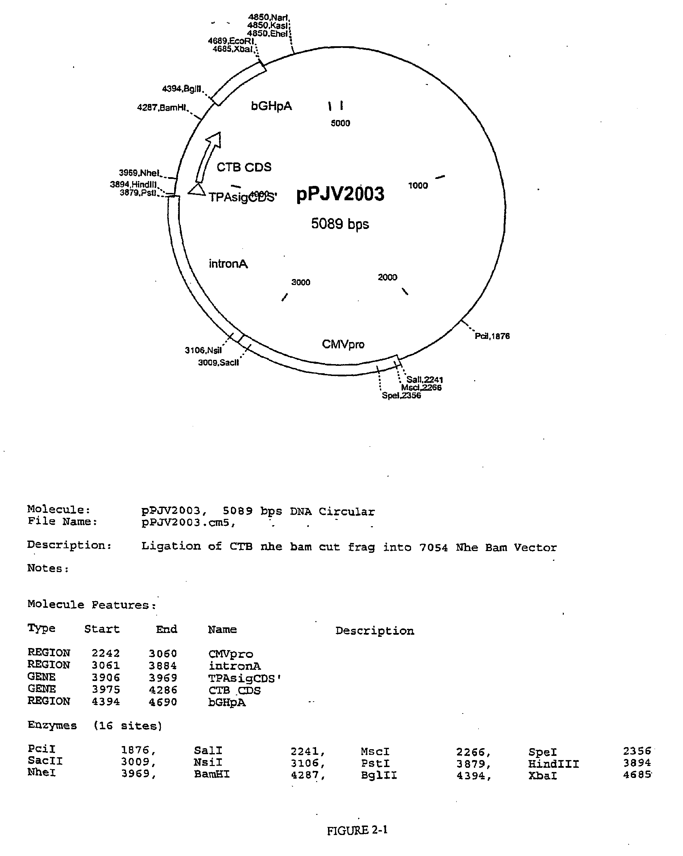

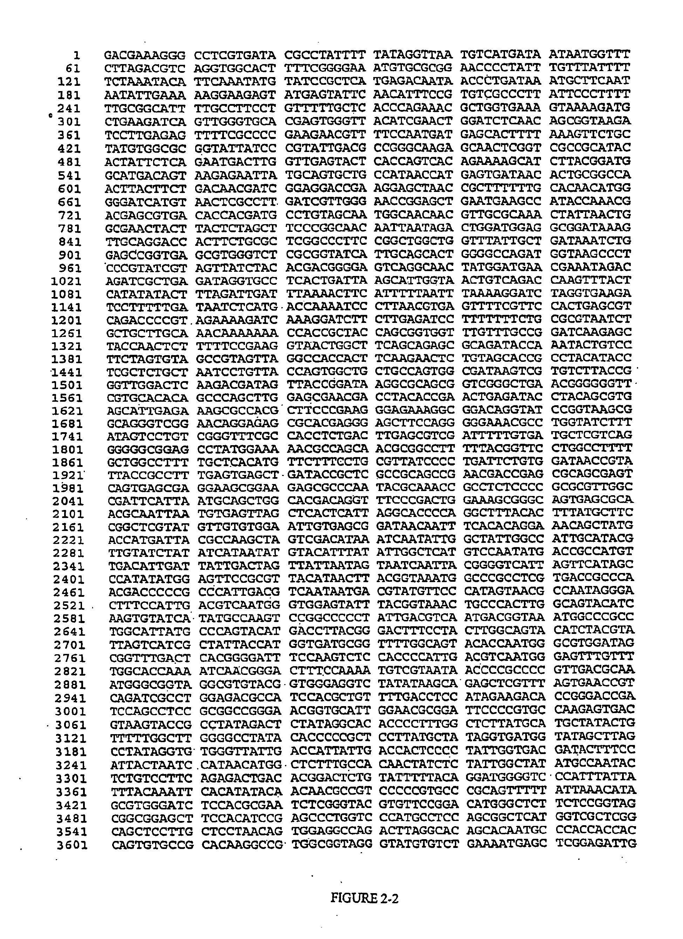

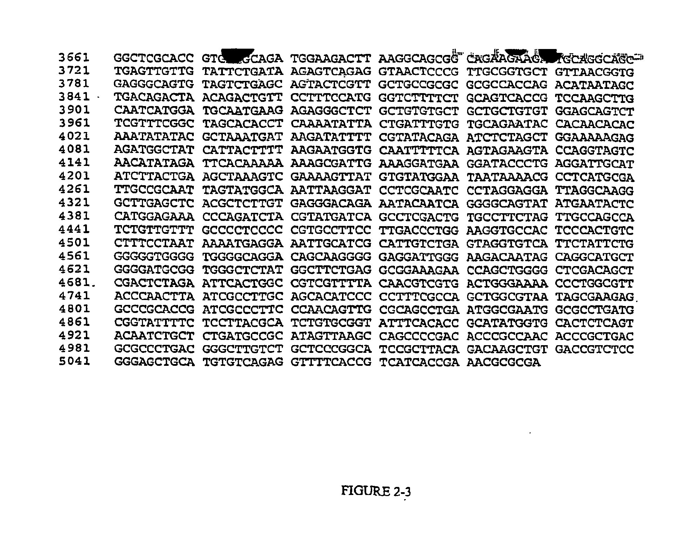

Figure 2 is a restriction map and functional map of plasmid pPJV2003 that contains a truncated coding sequence for a Cholera Toxin (CT) subunit B (CTB) peptide, wherein the plasmid further contains the hCMV immediate early promoter and associated intron A sequence, and the coding sequence for the signal peptide of human tissue plasminogen activator to allow for secretion from mammalian cells of the truncated CTB expression product. The figure further contains the complete nucleic acid sequence (SEQ ID NO: 2) for the pPJV2003 plasmid.

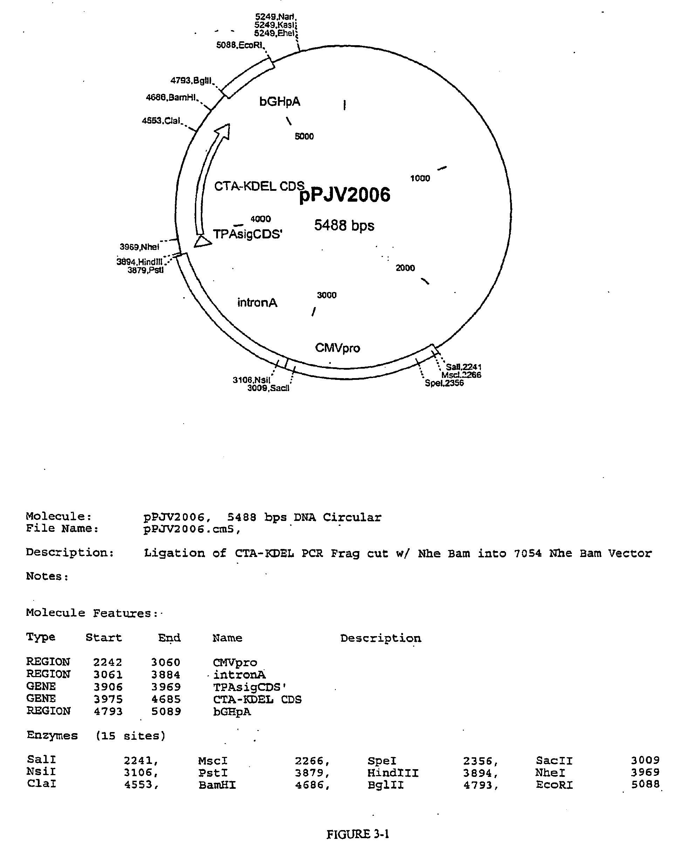

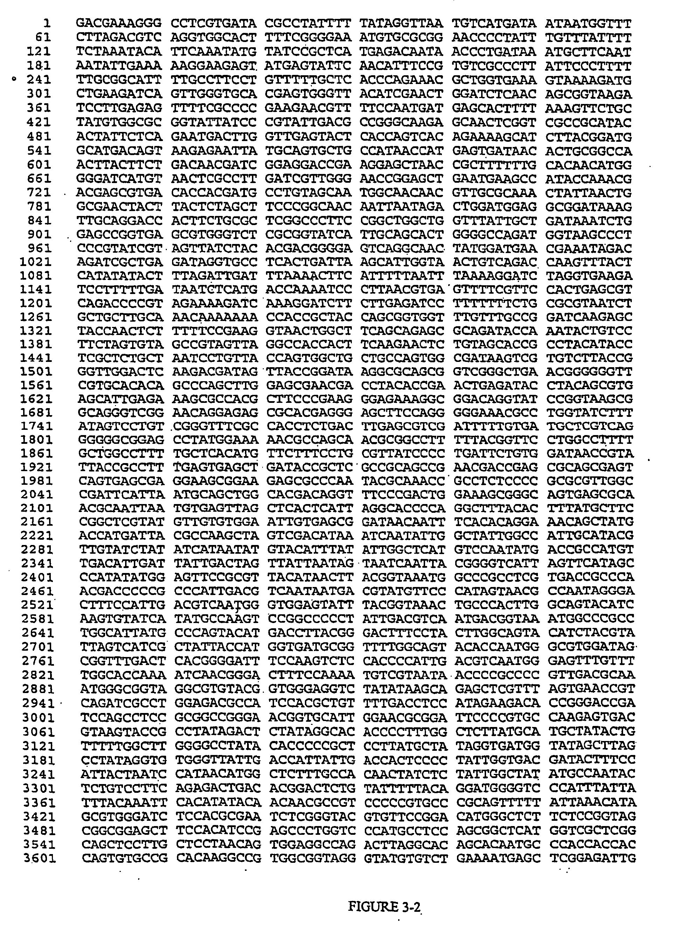

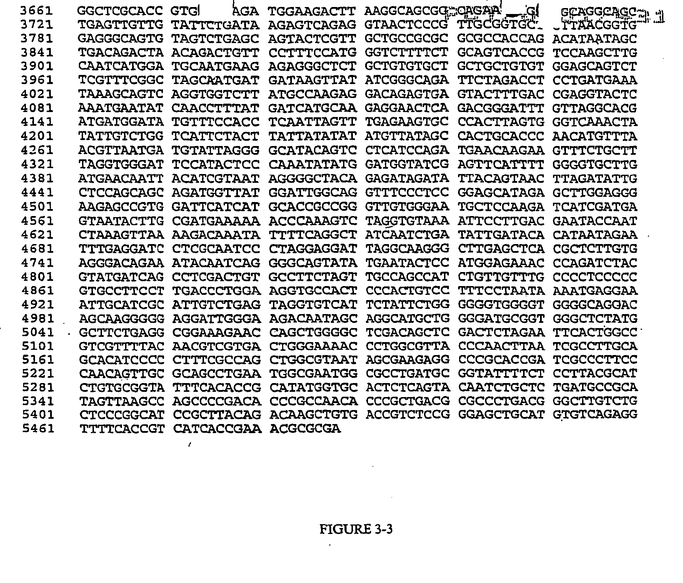

Figure 3 is a restriction map and functional map of plasmid pPJV2006 that contains a truncated coding sequence for a CTA peptide, wherein the truncated CTA coding sequence has been further modified to delete a C-terminal KDEL motif in the subunit peptide encoded thereby. The plasmid further contains the hCMV immediate early promoter and associated intron A sequence, and the coding sequence for the signal peptide of human tissue plasminogen activator to allow for secretion from mammalian cells of the truncated CTA expression product. The figure further contains the complete nucleic acid sequence (SEQ ID NO: 3) for the pPJV2006 plasmid.

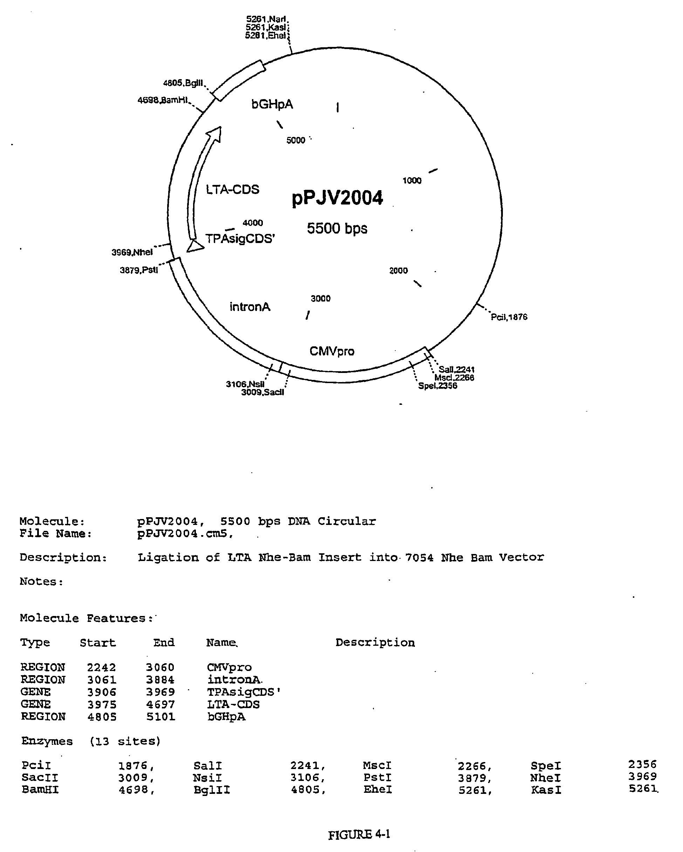

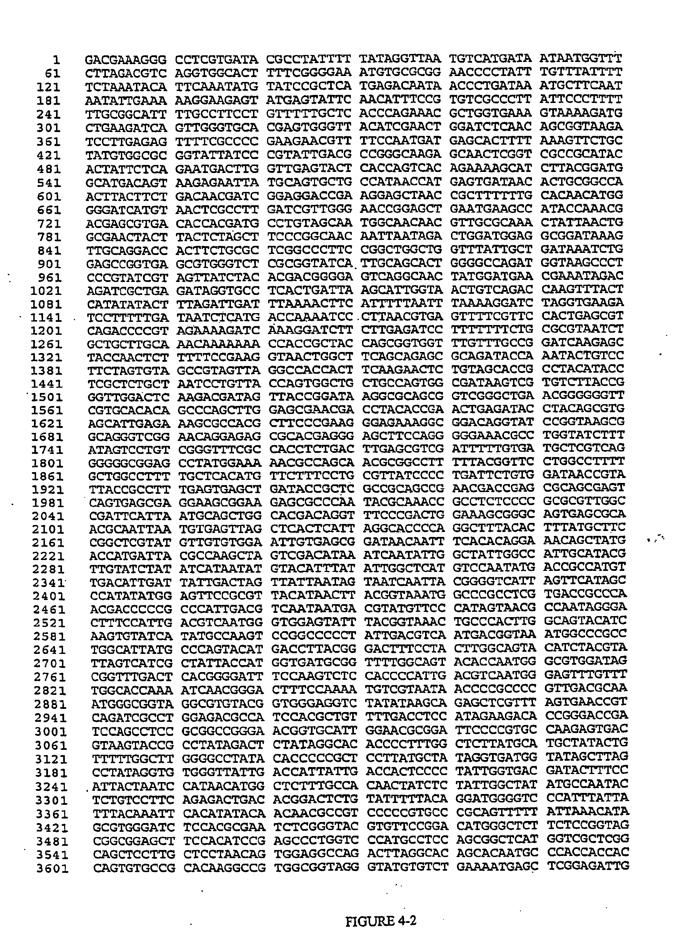

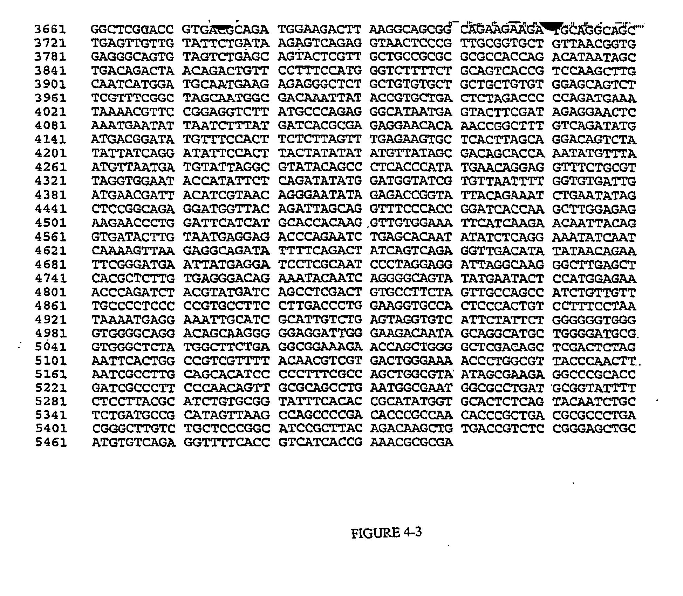

Figure 4 is a restriction map and functional map of plasmid pPJV2004 that contains a truncated coding sequence for an *E. coli* heat labile enterotoxin (LT) subunit A (LTA) peptide, wherein the plasmid further contains the hCMV immediate early promoter and associated intron A sequence, and the coding sequence for the signal peptide of human tissue plasminogen activator to allow for secretion from mammalian cells of the truncated LTA expression product. The figure further contains the complete nucleic acid sequence (SEQ ID NO: 4) for the pPJV2004 plasmid.

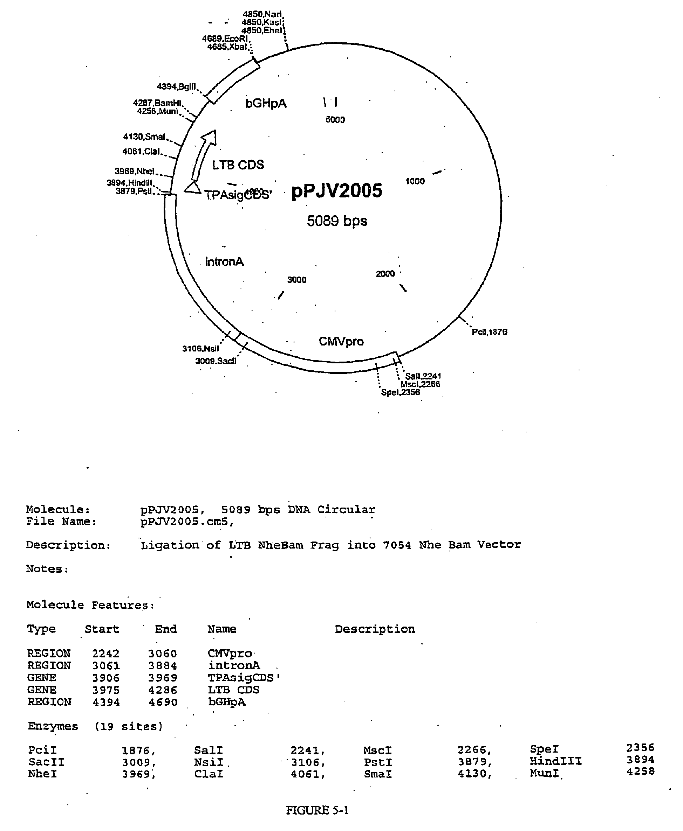

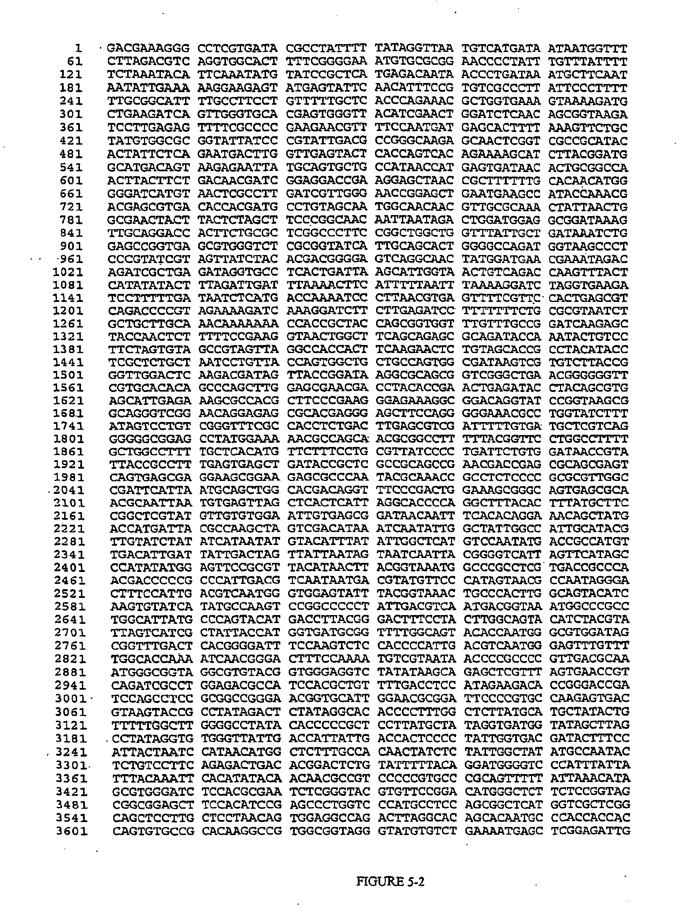

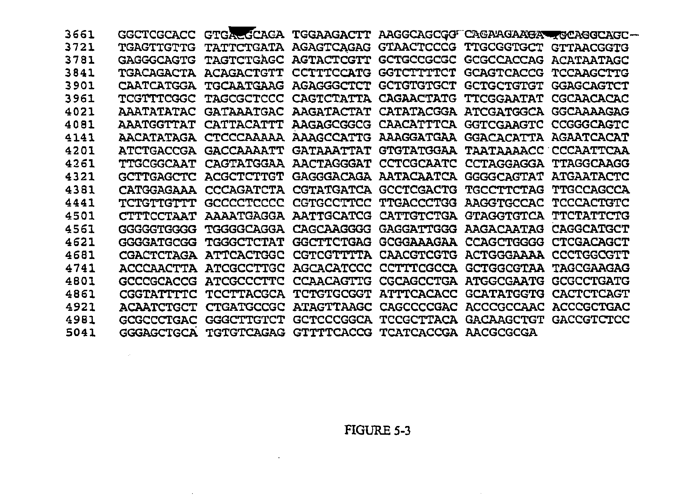

Figure 5 is a restriction map and functional map of plasmid pPJV2005 that contains a truncated coding sequence for an LT subunit B (LTB) peptide, wherein the plasmid further contains the hCMV immediate early promoter and associated intron A sequence, and the coding sequence for the signal peptide of human tissue plasminogen activator to allow for secretion from mammalian cells of the truncated LTB expression product. The figure further contains the complete nucleic acid sequence (SEQ ID NO: 5) for the pPJV2005 plasmid.

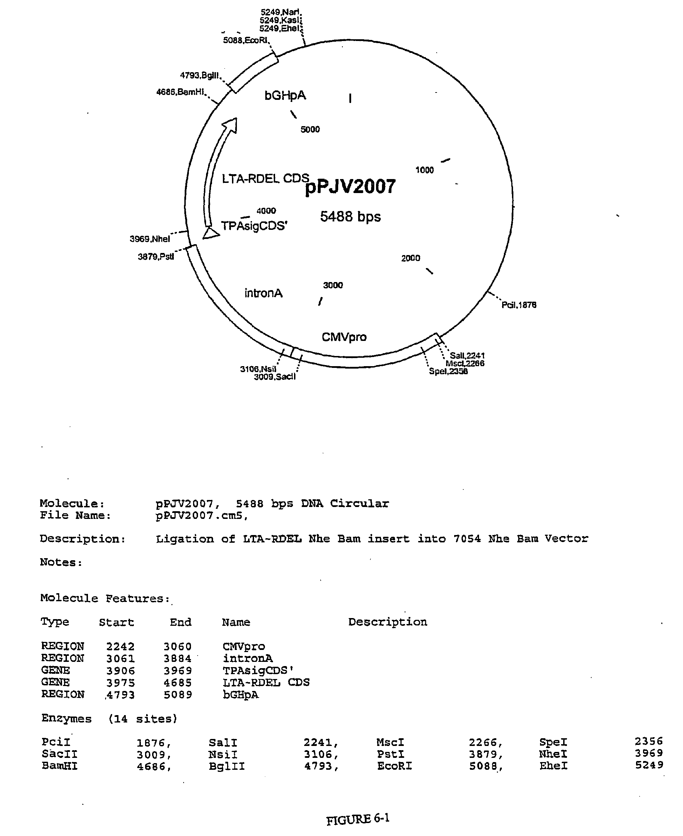

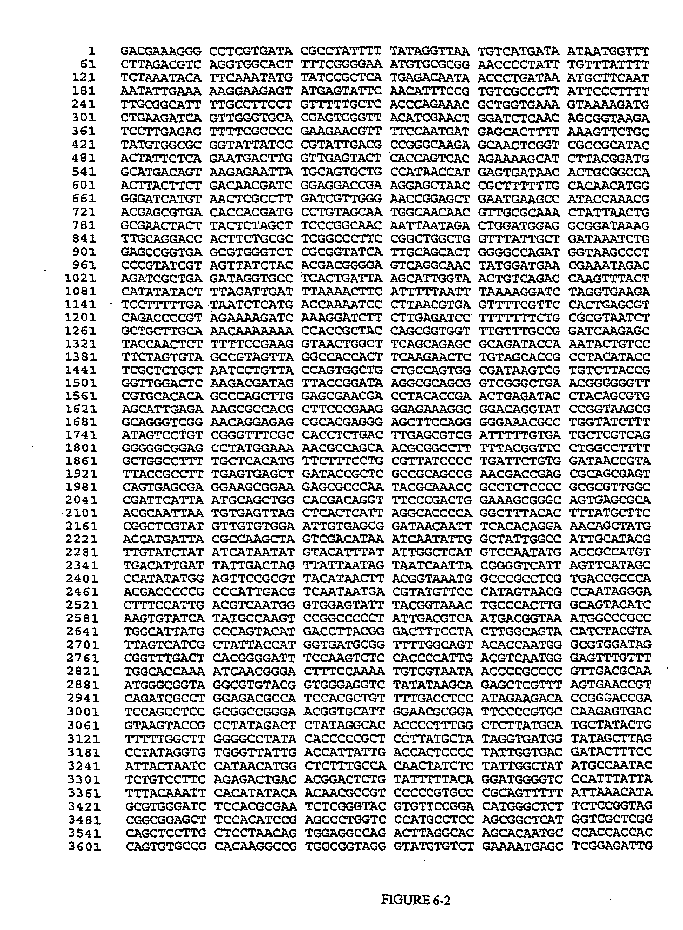

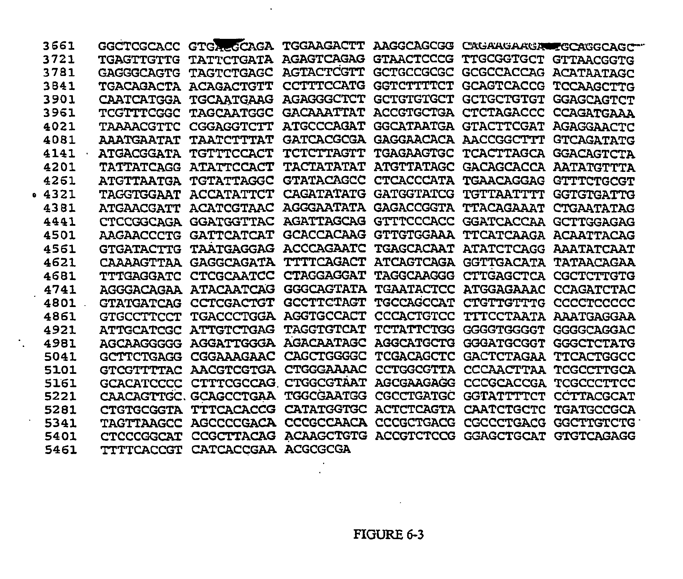

Figure 6 is a restriction map and functional map of plasmid pPJV2007 that contains a truncated coding sequence for an LTA peptide, wherein the truncated LTA coding sequence has been further modified to delete a C-terminal RDEL motif in the subunit peptide encoded thereby. The plasmid further contains the hCMV immediate early promoter and associated intron A sequence, and the coding sequence for the signal peptide of human tissue plasminogen activator to allow for secretion from mammalian cells of the truncated LTA expression product. The figure further contains the complete nucleic acid sequence (SEQ ID NO: 6) for the pPJV2007 plasmid.

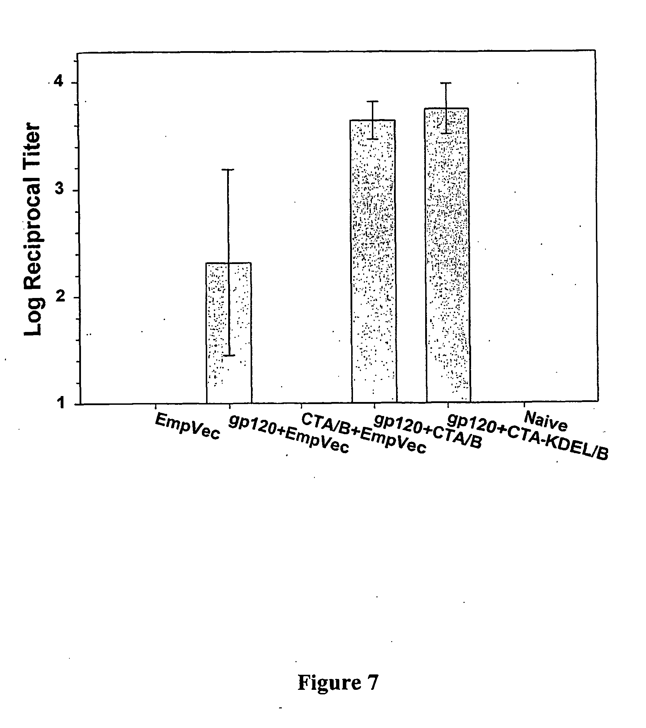

Figure 7 depicts the results from the ELISA carried out in Example 5. The histogram represents the log reciprocal titer of anti-gp120 antibody present in the animals receiving, from left to right in the figure, Formulation #1 containing the empty pWRG7054 vector ("EmpVec"); Formulation #2 containing the EmpVec (pWRG7054) and the pCIA-EnvT plasmid ("gp120"); Formulation #3 containing the EmpVec (pWRG7054) combined with the pPJV2002 and pPJV2003 adjuvant vectors ("CTA/B"); Formulation #4 containing the pCIA-EnvT plasmid ("gp120") combined with the pPJV2002 and pPJV2003 adjuvant vectors ("CTA/B"); Formulation #5 containing the pCIA-EnvT plasmid ("gp120") combined with the pPJV2006 and pPJV2003 adjuvant vectors ("CTA-KDEL/B"); or no vaccine and/or adjuvant composition ("naive").

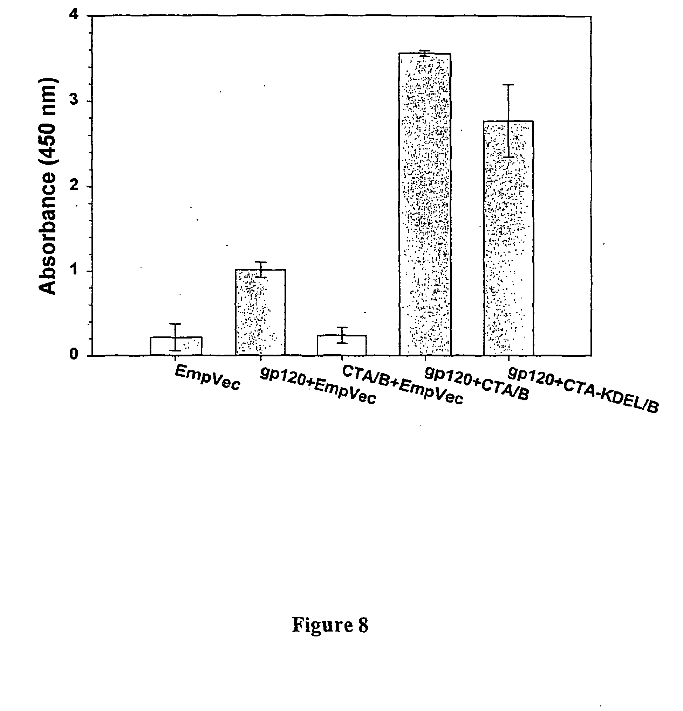

Figure 8 depicts the results from *the in situ* ELISA carried out in Example 5. The histogram represents the relative level of gp120-specific IFN-γ production in splenocytes obtained from animals receiving, from left to right in the figure, Formulation #1 containing the empty pWRG7054 vector ("EmpVec"); Formulation #2 containing the EmpVec (pWRG7054) and the pCIA-EnvT plasmid ("gp120"); Formulation #3 containing the EmpVec (pWRG7054) combined with the pPJV2002 and pPJV2003 adjuvant vectors ("CTA/B"); Formulation #4 containing the pCIA-EnvT plasmid ("gp 120") combined with the pPJV2002 and pPJV2003 adjuvant vectors ("CTA/B"); or Formulation #5 containing the pCIA-EnvT plasmid ("gp120") combined with the pPJV2006 and pPJV2003 adjuvant vectors ("CTA-KDEL/B").

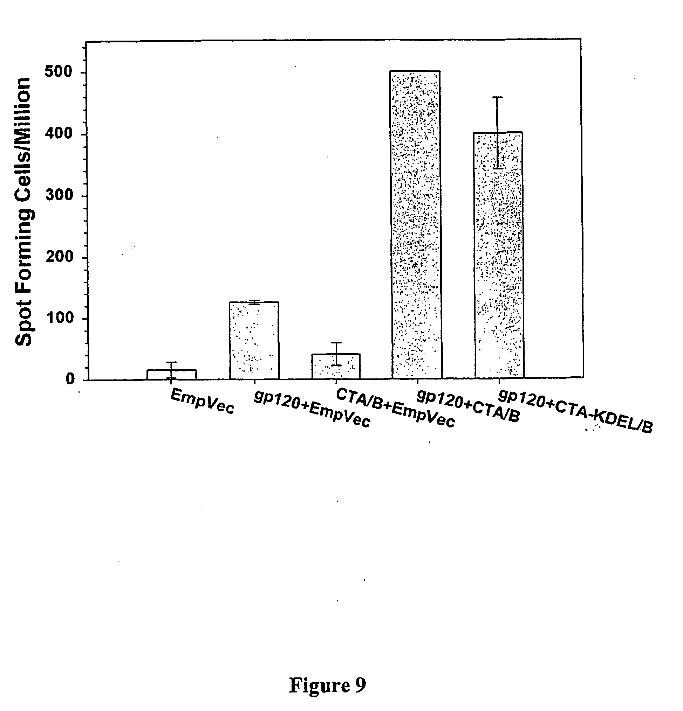

Figure 9 depicts the results from the ELISPOT assay carried out in Example 5. The histogram represents the relative levels of 1FN-γ-producing splenocytes obtained from animals receiving, from left to right in the figure, Formulation #1 containing the empty pWRG7054 vector ("EmpVec"); Formulation #2 containing the EmpVec (pWRG7054) and the pCIA-EnvT plasmid ("gp120"); Formulation #3 containing the EmpVec (pWRG7054) combined with the pPJV2002 and pPJV2003 adjuvant vectors ("CTA/B"); Formulation #4 containing the pCIA-EnvT plasmid ("gp 120") combined with the pPJV2002 and pPJV2003 adjuvant vectors ("CTAB"); or Formulation #5 containing the pCIA-EnvT plasmid ("gp120") combined with the pPJV2006 and pPJV2003 adjuvant vectors ("CTA-KDEL/B").

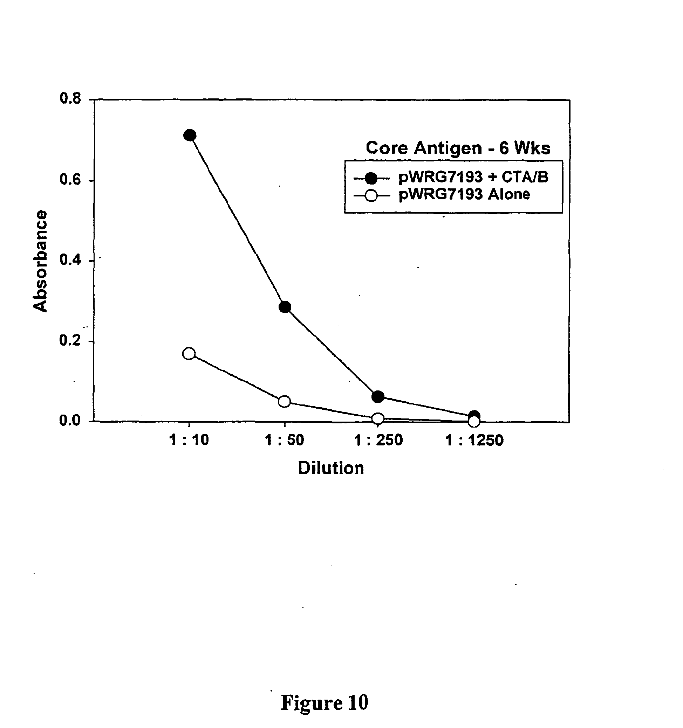

Figure 10 depicts the results from the ELISA carried out in Example 6. In this figure, the geometric mean absorbance values represent the titer of anti-HBcAg antibody present in serum samples (at four different dilutions) taken at the booster immunization (week 6 of the study) from animals receiving either Formulation #1 containing the HBcAg/HBsAg vector plasmid (pWRG7193); or Formulation #2 containing the HBcAg/HBsAg vector plasmid (pWRG7193) combined with the pPJV2002 and pPJV2003 adjuvant vectors ("CTA/B").

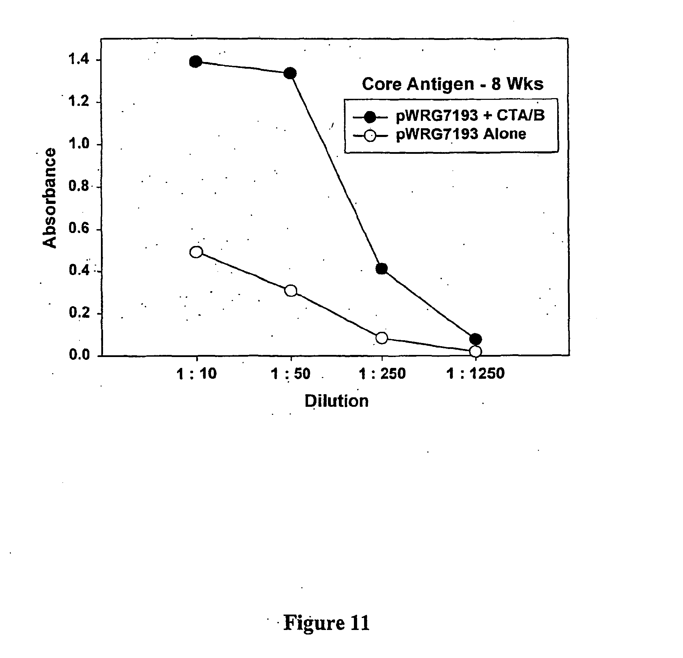

Figure 11 depicts the results from the ELISA carried out in Example 6. In this figure, the geometric mean absorbance values represent the titer of anti-HBcAg antibody present in serum samples (at four different dilutions) taken 2 weeks following the booster immunization (week 8 of the study) from animals receiving either Formulation #1 containing the HBcAg/HBsAg vector plasmid (pWRG7193); or Formulation #2 containing the HBcAg/HBsAg vector plasmid (pWRG7193) combined with the pPJV2002 and pPJV2003 adjuvant vectors ("CTA/B").

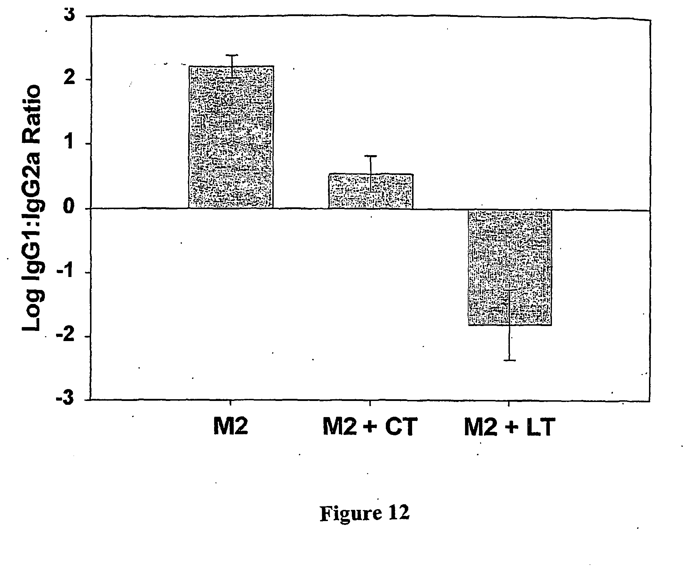

Figure 12 depicts the results from the ELISA carried out in Example 7. The histrogram represents the log IgGI ::IgG2a ratios from each immunization group receiving, from left to right in the figure, Formulation #1 containing the pM2-FL plasmid ("M2")combined with the empty vector plasmid control (pWRG7054); Formulation #2 containing the pM2-FL plasmid combined with the pPJV2002 and pPJV2003 CTA/B adjuvant vectors ("M2 + CT"); or Formulation #7 containing the pM2-FL plasmid combined with the pPJV2004 and pPJV2005 LTA/B adjuvant vectors ("M2 + LT").

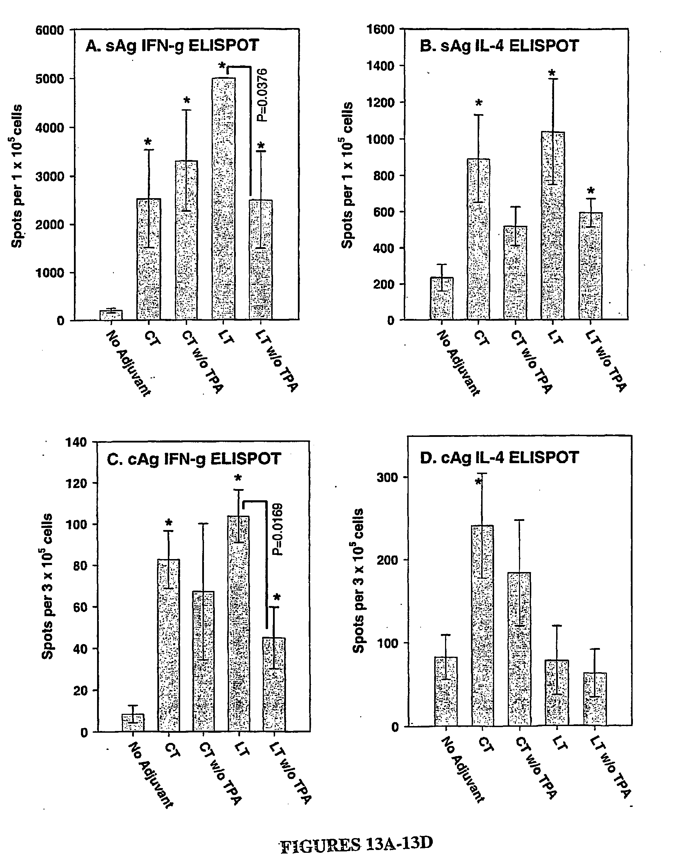

Figures 13A-13D depict the results from the IFN-γ and the IL4 ELISPOT assays used to assess the immune response to the Hepatitis B virus surface and core antigens in the first study of Example 8. The histograms represent the number of spots per 1X10⁵ spleen cells from the various experimental groups.

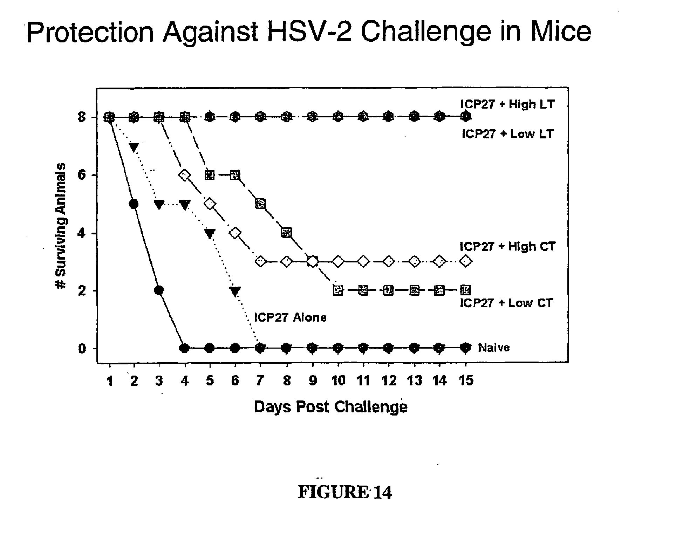

Figure 14 depicts the survival results from the HSV-2 virus challenge study carried out in Example 9.

### Detailed Description of the Invention

Before describing the present invention in detail, it is to be understood that this invention is not limited to particularly exemplified molecules or process parameters as such may, of course, vary. It is also to be understood that the terminology used herein is for the purpose of describing particular embodiments of the invention only, and is not intended to be limiting. In addition, the practice of the present invention will employ, unless otherwise indicated, conventional methods of virology, microbiology, molecular biology, recombinant DNA techniques and immunology all of which are within the ordinary skill of the art. Such techniques are explained fully in the literature. See, e.g., Sambrook, et al., Molecular Cloning: A Laboratory Manual (2nd Edition, 1989); DNA Cloning: A Practical Approach, vol. I & II (D. Glover, ed.); Oligonucleotide Synthesis (N. Gait, ed., 1984*);* A Practical Guide to Molecular Cloning (1984); and PCT/US01/43151 Fundamental Virology, 2nd Edition, vol. I & II (B.N. Fields and D.M. Knipe, eds.).

It must be noted that, as used in this specification and the appended claims, the singular forms "a," "an" and "the" include plural referents unless the content clearly dictates otherwise.

### Definitions

Unless defined otherwise, all technical and scientific terms used herein have the same meaning as commonly understood by one of ordinary skill in the art to which the invention pertains. Although a number of methods and materials similar or equivalent to those described herein can be used in the practice of the present invention, the preferred materials and methods are described herein.

In describing the present invention, the following terms will be employed, and are intended to be defined as indicated below.

The term "adjuvant" intends any material or composition capable of specifically or non-specifically altering, enhancing directing, redirecting, potentiating or initiating an antigen-specific immune response. Thus, coadministration of an adjuvant with an antigen may result in a lower dose or fewer doses of antigen being necessary to achieve a desired immune response in the subject to which the antigen is administered, or coadministration may result in a qualitatively and/or quantitatively different immune response in the subject. The effectiveness of an adjuvant can be determined by administering the adjuvant with a vaccine composition in parallel with a vaccine composition alone to animals and comparing antibody and/or cellular-mediated immunity in the two groups using standard assays such as radioimmunoassay, ELISAs, CTL assays, and the like, all well known in the art. Typically, in a vaccine composition, the adjuvant is a separate moiety from the antigen, although a single molecule can have both adjuvant and antigen properties.

An "adjuvant composition" intends any pharmaceutical composition containing an adjuvant. Adjuvant compositions can be delivered in the methods of the invention while in any suitable pharmaceutical form, for example, as a liquid, powder, cream, lotion, emulsion, gel or the like. However, preferred adjuvant compositions will be in particulate form. It is intended, although not always explicitly stated, that molecules having similar biological activity as wild-type or purified peptide or chemical adjuvants.

The term "peptide" is used in it broadest sense to refer to a compound of two or more subunit amino acids, amino acid analogs, or other peptidomimetics. The subunits may be linked by peptide bonds or by other bonds, for example ester, ether, etc. As used herein, the term "amino acid" refers to either natural and/or unnatural or synthetic amino acids, including glycine and both the D or L optical isomers, and amino acid analogs and peptidomimetics. A peptide of three or more amino acids is commonly called an "oligopeptide" if the peptide chain is short. If the peptide chain is long, the peptide is typically referred to as a "polypeptide" or a "protein".

An "antigen" refers to any agent, generally a macromolecule, which can elicit an immunological response in an individual. The term may be used to refer to an individual macromolecule or to a homogeneous or heterogeneous population of antigenic macromolecules. As used herein, "antigen" is generally used to refer to a peptide or carbohydrate molecule that contains one or more epitopes. For purposes of the present invention, antigens can be obtained or derived from any appropriate source. Furthermore, for purposes of the present invention, an "antigen" includes a peptide having modifications, such as deletions, additions and substitutions (generally conservative in nature) to the native sequence, so long as the peptide maintains sufficient immunogenicity. These modifications may be deliberate, for example through site-directed mutagenesis, or may be accidental, such as through mutations of hosts which produce the antigens.

An "immune response" against an antigen of interest is the development in an individual of a humoral and/or a cellular immune response to that antigen. For purposes of the present invention, a "humoral immune response" refers to an immune response mediated by antibody molecules, while a "cellular immune response" is one mediated by T-lymphocytes and/or other white blood cells.

The term "nucleic acid immunization" is used herein to refer to the introduction of a nucleic acid molecule encoding one or more selected antigens into a host cell for the *in vivo* expression of the antigen or antigens. The term also encompasses introduction of a nucleic acid molecule encoding one or more selected adjuvants into a host cell for the *in vivo* expression of the adjuvant or adjuvants. The nucleic acid molecule can be introduced directly into the recipient subject, such as by standard intramuscular or intradermal injection; transdermal particle delivery; inhalation; topically, or by oral, intranasal or mucosal modes of administration. The molecule alternatively can be introduced *ex vivo* into cells which have been removed from a subject. In this latter case, cells containing the nucleic acid molecule of interest are re-introduced into the subject such that an immune response can be mounted against the antigen encoded by the nucleic acid molecule, or such that the adjuvant encoded by the nucleic acid molecule can exert its adjuvant effect.

The terms "nucleic acid molecule" and "polynucleotide" are used interchangeably herein and refer to a polymeric form of nucleotides of any length, either deoxyribonucleotides or ribonucleotides, or analogs thereof. Polynucleotides may have any three-dimensional structure, and may perform any function, known or unknown. Non-limiting examples of polynucleotides include a gene, a gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers.

A polynucleotide is typically composed of a specific sequence of four nucleotide bases: adenine (A); cytosine (C); guanine (G); and thymine (T) (uracil (U) for thymine (T) when the polynucleotide is RNA). Thus, the term nucleic acid sequence is the alphabetical representation of a polynucleotide molecule. This alphabetical representation can be input into databases in a computer having a central processing unit and used for bioinformatics applications such as functional genomics and homology searching.

A "vector" is capable of transferring nucleic acid sequences to target cells (e.g., viral vectors, non-viral vectors, particulate carriers, and liposomes). Typically, "vector construct," "expression vector," and "gene transfer vector," mean any nucleic acid construct capable of directing the expression of a gene of interest and which can transfer gene sequences to target cells. Thus, the term includes cloning and expression vehicles, as well as viral vectors. A "plasmid" is a vector in the form of an extrachromosomal genetic element.

A nucleic acid sequence which "encodes" a selected adjuvant and/or antigen is a nucleic acid molecule which is transcribed (in the case of DNA) and translated (in the case of mRNA) into a polypeptide *in vivo* when placed under the control of appropriate regulatory sequences. The boundaries of the coding sequence are determined by a start codon at the 5' (amino) terminus and a translation stop codon at the 3' (carboxy) terminus. For the purposes of the invention, such nucleic acid sequences can include, but are not limited to, cDNA from viral, procaryotic or eucaryotic mRNA, genomic sequences from viral or procaryotic DNA or RNA, and even synthetic DNA sequences. A transcription termination sequence may be located 3' to the coding sequence.

A "promoter" is a nucleotide sequence which initiates and regulates transcription of a polypeptide-encoding polynucleotide. Promoters can include inducible promoters (where expression of a polynucleotide sequence operably linked to the promoter is induced by an analyte, cofactor, regulatory protein, etc.), repressible promoters (where expression of a polynucleotide sequence operably linked to the promoter is repressed by an analyte, cofactor, regulatory protein, etc.), and constitutive promoters. It is intended that the term "promoter" or "control element" includes full-length promoter regions and functional (e.g., controls transcription or translation) segments of these regions.

"Operably linked" refers to an arrangement of elements wherein the components so described are configured so as to perform their usual function. Thus, a given promoter operably linked to a nucleic acid sequence is capable of effecting the expression of that sequence when the proper enzymes are present. The promoter need not be contiguous with the sequence, so long as it functions to direct the expression thereof. Thus, for example, intervening untranslated yet transcribed sequences can be present between the promoter sequence and the nucleic acid sequence and the promoter sequence can still be considered "operably linked" to the coding sequence.

"Recombinant" is used herein to describe a nucleic acid molecule (polynucleotide) of genomic, cDNA, semisynthetic, or synthetic origin which, by virtue of its origin or manipulation is not associated with all or a portion of the polynucleotide with which it is associated in nature and/or is linked to a polynucleotide other than that to which it is linked in nature. Two nucleic acid sequences which are contained within a single recombinant nucleic acid molecule are "heterologous" relative to each other when they are not normally associated with each other in nature.

An "isolated polynucleotide" is a nucleic acid molecule separate and discrete from the whole organism with which the molecule is found in nature; or a nucleic acid molecule devoid, in whole or part, of sequences normally associated with it in nature; or a sequence, as it exists in nature, but having heterologous sequences (as defined below) in association therewith. A sequence is "derived or obtained from" a molecule if it has the same or substantially the same base pair sequence as a region of the source molecule, its cDNA, complements thereof, or if it displays sequence identity as described below.

Techniques for determining nucleic acid and amino acid "sequence identity" or "sequence homology" also are known in the art. Typically, such techniques include determining the nucleotide sequence of the mRNA for a gene and/or determining the amino acid sequence encoded thereby, and comparing these sequences to a second nucleotide or amino acid sequence. In general, "identity" refers to an exact nucleotide-to-nucleotide or amino acid-to-amino acid correspondence of two polynucleotides or polypeptide sequences, respectively. Two or more sequences (polynucleotide or amino acid) can be compared by determining their "percent identity." The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M.O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, WI) in the "BestFit" utility application. The default parameters for this method are described in the Wisconsin Sequence Analysis Package Program Manual, Version 8 (1995) (available from Genetics Computer Group, Madison, WI). A preferred method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects "sequence identity." Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.nobi.nlm.gov/cgi-bin/BLAST.

Alternatively, homology can be determined by hybridization of polynucleotides under conditions which form stable duplexes between homologous regions, followed by digestion with single-stranded-specific nuclease(s), and size determination of the digested fragments. Two DNA, or two polypeptide sequences are "substantially homologous" to each other when the sequences exhibit at least about 80%-85%, preferably at least about 90%, and most preferably at least about 95%-98% sequence identity over a defined length of the molecules, as determined using the methods above. As used herein, substantially homologous also refers to sequences showing complete identity to the specified DNA or polypeptide sequence. DNA sequences that are substantially homologous can be identified in a Southern hybridization experiment under, for example, stringent conditions, as defined for that particular system. For example, stringent hybridization conditions can include 50% formamide, 5x Denhardt's Solution, 5x SSC, 0.1% SDS and 100 µg/ml denatured salmon sperm DNA and the washing conditions can include 2x SSC, 0.1% SDS at 37°C followed by 1x SSC, 0.1% SDS at 68°C. Defining appropriate hybridization conditions is within the skill of the art. See, e.g., Sambrook et *al., supra; DNA Cloning, supra; Nucleic Acid Hybridization, supra.*

The term "transdermal" delivery intends intradermal (e.g., into the dermis or epidermis), transdermal (e.g., "percutaneous") and transmucosal administration, i.e., delivery by passage of an agent into or through skin or mucosal tissue. *See, e.g.,* Transdermal Drug Delivery: Developmental Issues and Research Initiatives, Hadgraft and Guy (eds.), Marcel Dekker, Inc., (1989); Controlled Drug Delivery: Fundamentals and Applications, Robinson and Lee (eds.), Marcel Dekker Inc., (1987); and Transdermal Delivery of Drugs, Vols. 1-3, Kydonieus and Berner (eds.), CRC Press, (1987). Thus, the term encompasses delivery of an agent using a particle delivery device (e.g., a needleless syringe) such as those described in U.S. Patent No. 5,630,796, as well as delivery using particle-mediated delivery devices such as those described in U.S. Patent No. 5,865,796.

By "core carrier" is meant a carrier on which a guest nucleic acid (e.g., DNA, RNA) is coated in order to impart a defined particle size, as well as a sufficiently high density to achieve the momentum required for cell membrane penetration, such that the guest molecule can be delivered using particle-mediated techniques (see, e.g., U.S. Patent No. 5,100,792). Core carriers typically include materials such as tungsten, gold, platinum, ferrite, polystyrene and latex. See e.g., Particle Bombardment Technology for Gene Transfer, (1994) Yang, N. ed., Oxford University Press, New York, NY pages 10-11.

By "particle delivery device" is meant an instrument which delivers a particulate composition transdermally without the aid of a conventional needle to pierce the skin. Particle delivery devices for use with the present invention are discussed throughout this document.

As used herein, the term "treatment" includes any of following: the prevention of infection or reinfection; the reduction or elimination of symptoms; and the reduction or complete elimination of a pathogen. Treatment may be effected prophylactically (prior to infection) or therapeutically (following infection).

The terms "individual" and "subject" are used interchangeably herein to refer to any member of the subphylum cordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The terms do not denote a particular age. Thus, both adult and newborn individuals are intended to be covered. The methods described herein are intended for use in any of the above vertebrate species, since the immune systems of all of these vertebrates operate similarly.

### General Overview

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

The present invention provides novel compositions containing nucleic acid sequences, wherein a first sequence in the composition is a coding sequence for an A subunits obtained or derived from an ADP-ribosylating bacterial toxin, and a second sequence in the composition is a coding sequence for a B subunit obtained or derived from an ADP-ribosylating bacterial toxin. The first and second sequences are useful in immunization methods wherein they are delivered to a subject in order to provide for an adjuvant effect (against a co-administered antigen of interest) in the immunized subject. ADP-ribosylating bacterial toxins are a family of related bacterial exotoxins and include diphtheria toxin (DT), pertussis toxin (PT), cholera toxin (CT), the *E. coli* heat-labile toxins (LT1 and LT2), *Pseudomonas* endotoxin A, *Pseudomonas* exotoxin S, *B. cereus* exoenzyme, *B. sphaericus* toxin, C. *botulinum* C2 and C3 toxins, C. *limosum* exoenzyme, as well as toxins from *C. perfringens, C. spiriforma* and C. *difficile, Staphylococcus aureus* EDIN, and ADP-ribosylating bacterial toxin mutants such as GRM_{197,} a non-toxic diphtheria toxin mutant (see, e.g., Bixler et al. (1989) Adv. Exp. Med. Biol. 251:175; and Constantino et al. (1992) Vaccine*).* Most ADP-ribosylating bacterial toxins are organized as an A:B multimer, wherein the A subunit contains the ADP-ribosyltransferase activity, and the B subunit acts as the binding moiety. Preferred ADP-ribosylating bacterial toxins for use in the compositions of the present invention include cholera toxin and the *E*. *coli* heat-labile toxins.

Cholera toxin (CT) and the related E. *coli* heat labile enterotoxins (LT) are secretion products of their respective enterotoxic bacterial strains that are potent immunogens and exhibit strong toxicity when administered systemically, orally, or mucosally. Both CT and LT are known to provide adjuvant effects for antigen when administered via the intramuscular or oral routes. These adjuvant effects have been observed at doses below that required for toxicity. The two toxins are extremely similar molecules, and are at least about 70-80% homologous at the amino acid level.

The CT and LT toxins are hexamers, composed of a single molecule of an A subunit surrounded by a doughnut-shaped ring composed of 5 molecules of the B subunit. The A subunit possesses an ADP-ribosylase activity resulting in G protein modifications that lead to cAMP and protein kinase A upregulation following internalization of the A subunit in a mammalian cell. The A subunit can be nicked by exogenous proteases yielding the A1 and A2 fragments linked via a single disulfide bridge. The A subunit of CT contains a C-terminal KDEL peptide motif (RDEL for the A subunit of LT) that is associated with retrieval of proteins from the trans-Golgi network into the ER. This is likely important for delivery of the A fragment to the correct cellular compartment for toxicity following internalization, and retention of the toxin within that cellular compartment. The A1 subunit enters the cell cytoplasm and triggers Cl-efflux by catalysing ADP-ribosylation of a G protein, which activates adenylate cyclase leading to elevated levels of cAMP. Elevated cAMP causes protein kinase A to phosphorylate and open the cystic fibrosis transmembrane conductance regulator chloride channel.

The A2 fragment's main role is in interacting with the B subunit. Toxin internalization is mediated by the five copies of the B subunit which possess binding activity for the GM1 ganglioside, a glycosphingolipid found ubiquitously on the surface of mammalian cells.

The relative importance of the A and B subunits for adjuvanticity is controversial. There is speculation in the field that the toxic activity of the A subunit can modulate the adjuvant effects associated with the B subunit. Some studies demonstrate that mutations in the A subunit abrogate adjuvant activity while others show that A subunit mutations that block ADP-ribosylase activity have no effect on adjuvanticity. Other reports show varying levels of adjuvant effects for purified B subunits or recombinant B subunit preparations. It is likely that the A and B subunits have separate functions that independently contribute to adjuvanticity. These functions are ADP-ribosylase activity and receptor triggering activity, respectively. Regarding the B subunit of LT in particular, binding to the GM1 receptor on B cells results in polyclonal activation, occurs in the absence of significant proliferation, and involves the upregulation of a number of important molecules such as MHC class II, B7, CD40, ICAM-1 and IL2-Rα. For T cells, addition of CT or LT holotoxins or recombinant B subunits to concanavilin A-stimulated T cells results in a reduction in thymidine incorporation. Affects of these molecules on macrophages and dendritic cells have not been reported. In PBMC cultures, EtxB stimulates high level TNF-α and IL-10 production but not IL-12. This is consistent with the observations of predominately Th2-like responses associated with the use of CT and LT forms as adjuvants.

It is therefore a surprising feature of the present invention that administration of the present polynucleotides adjuvant compositions preferentially produces an augmented Th1-like immune response against the co-administered antigen of interest, rather than the Th2-like response that would be expected from the use of an ADP-ribosylating exotoxin adjuvant composition.

### Coding Sequences for ADP-ribosylating Exotoxin Subunits

In one embodiment, a composition is provided which includes one or more recombinant nucleic acid molecules, said one or more molecules containing first and second nucleic acid sequences wherein (a) the first nucleic acid sequence is a coding region for a truncated A subunit peptide obtained or derived from an ADP-ribosylating exotoxin, and (b) the second nucleic acid sequence is a coding region for a truncated B subunit peptide obtained or derived from an ADP-ribosylating exotoxin. The coding regions provide for "truncated" subunit peptides in that each said subunit coding region has been genetically altered so as to create a 5' deletion, whereby the encoded subunit peptide does not have an amino terminal bacterial signal peptide.

In another embodiment, a composition is provided which includes one or more recombinant nucleic acid molecules, said one or more molecules containing first and second nucleic acid sequences wherein (a) the first nucleic acid sequence is a coding region for a modified, mature A subunit peptide obtained or derived from an ADP-ribosylating exotoxin, and (b) the second nucleic acid sequence is a coding region for a mature B subunit peptide obtained or derived from an ADP-ribosylating exotoxin. The first nucleic acid sequence contains a coding region that provides for a "modified" A subunit peptide in that the said coding region has been genetically altered so as to delete a four amino acid residue C-terminal KDEL or RDEL motif normally found in the encoded subunit peptide.

In still a further embodiment, a composition is provided which includes one or more recombinant nucleic acid molecules, said one or more molecules containing first and second nucleic acid sequences wherein (a) the first nucleic acid sequence is a coding region for a truncated and modified A subunit peptide obtained or derived from an ADP-ribosylating exotoxin, and (b) the second nucleic acid sequence is a coding region for a truncated B subunit peptide obtained, or derived from an ADP-ribosylating exotoxin.

In particularly preferred embodiments, the ADP-ribosylating exotoxin peptide subunit coding sequences are obtained or derived from a cholera toxin (CT). In other particularly preferred embodiments, the ADP-ribosylating exotoxin subunit peptide coding sequences are obtained or derived from an E. *coli* heat labile enterotoxin (LT), for example LT1 or LT2.

Portions of the genomes of various enterotoxic bacterial species, particularly those portions containing the coding sequences for ADP-ribosylating. exotoxins, are generally known and the sequences therefor are publically available, for example on the World Wide Web, and/or are deposited with repositories such as the GenBank database. For example, a GenBank entry for the complete sequences of the CT subunit A and B genes can be found at Locus VIBCTXABB (Accession No. D30053), while a GenBank entry for the complete sequences of the LT subunit A and B genes can be found at Locus AB0116677 (Accession No. AB011677). Active variants or fragments of these toxin sequences may also be used in the compositions and methods of the present invention. For a general discussion of ADP-ribosylating exotoxins, see e.g., Krueger et al. (1995) Clin. Microbiol. Rev. 8:34-47. Furthermore, in certain aspects of the invention, one or both of the toxin subunit peptide coding regions can be further genetically modified to detoxify the subunit peptide(s) encoded thereby. For example, genetically altered toxin mutants which have disrupted ADP-ribosylating activity, trypsin cleavage site mutations, or disrupted binding activity are known in the art. See, e.g., Bumette et al. (1994) "Recombinant microbial ADP-ribosylating toxins of Bordetella pertusis, Vibrio cholerae, and enterotoxigenic Escherichia coli: structure, function and toxoid vaccine development," in Bioprocess Technology, Bumette et al. eds., pp. 185-203; Rappuoli et al. (1995) Int. Archiv. Allergy Immunol. 108:327-333; and Rappuoli et al. (1996) Ad. Exp. Med. Biol. 397:55-60. Sequences encoding the selected toxin subunits are typically inserted into an appropriate vector (e.g., a plasmid backbone) using known techniques and as described below in the Examples.

The sequence or sequences encoding the ADP-ribosylating exotoxin subunit peptides of interest can be obtained and/or prepared using known methods. For example, substantially pure preparations can be obtained using standard molecular biological tools. That is, polynucleotide sequences coding for the above-described toxin subunuits can be obtained using recombinant methods, such as by screening cDNA libraries from cells expressing the toxin subunits, or by deriving the coding sequence for the toxin subunits from a vector known to include the same. The toxin subunit coding sequences from various bacterial strains are on deposit with the American Type Culture Collection ATCC, and yet others are available from national and international health organizations such as the Centers of Disease Control (Atlanta, GA). See, e.g., Sambrook et al., *supra,* for a description of techniques used to obtain and isolate nucleic acid molecules. Polynucleotide sequences can also be produced synthetically, rather than cloned.

Yet another convenient method for isolating specific nucleic acid molecules is by the polymerase chain reaction (PCR). Mullis et al. (1987) Methods Enzymol. 155:335-350. This technique uses DNA polymerase, usually a thermostable DNA polymerase, to replicate a desired region of DNA. The region of DNA to be replicated is identified by oligonucleotides of specified sequence complementary to opposite ends and opposite strands of the desired DNA to prime the replication reaction. The product of the first round of replication is itself a template for subsequent replication, thus repeated successive cycles of replication result in geometric amplification of the DNA fragment delimited by the primer pair used.

Once the relevant sequences for the ADP-ribosylating exotoxin subunits of interest have been obtained, they can be linked together to provide one or more contiguous nucleic acid molecules using standard cloning or molecular biology techniques. More particularly, after sequence information for the selected toxin subunit combination has been obtained, the coding sequences can be combined with each other or with other sequences to form a hybrid sequence, or handled separately. In hybrid sequences, the subunit peptide coding sequences can be positioned in any manner relative to each other, and be included in a single molecule in any number ways, for example, as a single copy, randomly repeated in the molecule as multiple copies, or included in the molecule as multiple tandem repeats or otherwise ordered repeat motifs.

Although any number of routine molecular biology techniques can be used to construct such recombinant nucleic acid molecules, one convenient method entails using one or more unique restriction sites in a shuttle or cloning vector (or inserting one or more unique restriction sites into a suitable vector sequence) and standard cloning techniques to direct the subunit peptide coding sequence or sequences into particular target locations within a vector.

Alternatively, hybrid molecules can be produced synthetically rather than cloned. The nucleotide sequence can be designed with the appropriate codons for the particular amino acid sequence desired. In general, one will select preferred codons for the intended host in which the sequence will be expressed. The complete sequence can then be assembled from overlapping oligonucleotides prepared by standard methods and assembled into a complete coding sequence. See, e.g., Edge (1981) Nature 292:756; Nambair et al. (1984) Science (1984) 223:1299; Jay et al. (1984) J. Biol. Chem. 259:6311.

Once the relevant ADP-ribosylating exotoxin peptide coding sequences have been obtained or constructed, they can be inserted into one or more suitable vectors which include control sequences operably linked to the inserted sequence or sequences, thus providing expression cassettes that allow for expression of the toxin subunit peptides *in vivo* in a targeted subject species.

Typical promoters for mammalian cell expression include the SV40 early promoter, a CMV promoter such as the CMV immediate early promoter, the mouse mammary tumor virus LTR promoter, the adenovirus major late promoter (Ad MLP), and other suitably efficient promoter systems. Nonviral promoters, such as a promoter derived from the murine metallothionein gene, may also be used for mammalian expression. Inducible, repressible or otherwise controllable promoters may also be used. Typically, transcription termination and polyadenylation sequences will also be present, located 3' to each translation stop codon. Preferably, a sequence for optimization of initiation of translation, located 5' to each coding sequence, is also present. Examples of transcription terminator/polyadenylation signals include those derived from SV40, as described in Sambrook et al., *supra,* as well as a bovine growth hormone terminator sequence. Introns, containing splice donor and acceptor sites, may also be designed into the expression cassette.

In addition, enhancer elements may be included within the expression cassettes in order to increase expression levels. Examples of suitable enhancers include the SV40 early gene enhancer (Dijkema et al. (1985) EMBO J. 4:761), the enhancer/promoter derived from the long terminal repeat (LTR) of the Rous Sarcoma Virus (Gorman et al. (1982) Proc. Natl. Acad. Sci. USA 79:6777), and elements derived from human or murine CMV (Boshart et al. (1985) Cell 41:521), for example, elements included in the CMV intron A sequence.

A further ancillary sequence is included which provides for secretion of the attached ADP-ribosylating exotoxin subunit peptide from a mammalian cell. Such secretion leader sequences are known to those skilled in the art, and include, for example, the tissue plasminogen activator (tpa) leader signal sequence.

After one or more suitable expression cassettes (or nucleic acid constructs such as plasmid vectors) have been produced that contain the ADP-ribosylating exotoxin subunit peptide coding sequences of interest, the expression cassettes can be provided in a suitable transfection vector such as a DNA plasmid vector or a viral vector for subsequent administration to a subject. In this regard, the polynucleotide compositions of the invention can be used as standalone adjuvant compositions, or co-administered with an antigen of interest, e.g., as part of a multi-component vaccine composition. For example, in a multi-component vaccine composition, the present nucleic acid molecules (containing the coding sequences for the ADP-ribosylating exotoxin subunit peptides of interest) can be combined with additional nucleic acid molecules encoding one or more antigens known to be important for providing protection against a pathogen, for example, molecules containing sequences that encode influenza HA or NA antigens, or molecules containing sequences that encode HIV antigens. Alternatively, the multi-component vaccine composition may contain, in addition to the polynucleotides encoding the toxin subunits, antigen in the form of conventional whole virus, split virus, polysaccahride, or purified subunit vaccine preparations as described herein below.

### Antigens

The compositions and methods described herein are useful in adjuvanting an immune response against a wide variety of co-administered antigens, for example antigens obtained or derived from diseased cells or tissues, of from human or animal pathogens. For the purposes of the present invention, an adjuvant is used to either enhance the immune response to a specific antigen, e.g., when an adjuvant is co-administered with a vaccine composition, the immune response is greater than the immune response elicited by an equivalent amount of the vaccine composition administered without the adjuvant, or the adjuvant is used to direct a particular type or class of immune response against a co-administered antigen. Co-administration of an "effective amount" of the adjuvant compositions of the present invention will be that amount which enhances an immunological response to the co-administered antigen such that, for example, lower or fewer doses of the antigen are required to generate an efficient immune response.

As used herein, the term "co-administered," such as when an ADP-ribosylating exotoxin subunit encoding adjuvant composition according to the present invention is "co-administered" with an antigen of interest (e.g., a vaccine composition), intends either the simultaneous or concurrent administration of the adjuvant composition and the antigen, e.g., when the two are present in the same composition or administered in separate compositions at nearly the same time but at different sites, as well as the delivery of the adjuvant composition and antigen in separate compositions at different times, including delivery to different sites. For example, the adjuvant composition may be delivered prior or subsequent to delivery of the antigen at the same or a different site. The timing between adjuvant and antigen deliveries can range from about several minutes apart, to several hours apart, to several days apart.

For the purposes of the instant invention, the term "pathogen" is used in a broad sense to refer to a specific causative agent of a disease or condition, and includes any agent that provides a source of a molecule that elicits an immune response. Thus, pathogens include, but are not limited to, viruses; bacteria, fungi, protozoa, parasites, cancer cells and the like. Typically, the immune response is elicited by one or more peptide or carbohydrate antigens produced by the pathogen. Methods for identifying suitable antigens, obtaining and preparing such molecules, and then determining suitable dosages, assaying for suitable immunogenicity and treating with such antigens are well known in the art. See e.g., Plotkin et al. (1994) Vaccines, 2nd Edition, W.B. Saunders, Philadelphia, PA. Non-limiting examples of sources for antigens that can be used to vaccinate vertebrate subjects, particularly, humans and non-human mammals, thus include viruses, bacteria, fungi, and other pathogenic organisms.

Suitable viral antigens include, but are not limited to, those obtained or derived from the hepatitis family of viruses, including hepatitis A virus (HAV), hepatitis B virus (HBV), hepatitis C virus (HCV), the delta hepatitis virus (HDV), hepatitis E virus (HEV) and hepatitis G virus (HGV). See, e.g., International Publication Nos. WO 89/04669; WO 90/11089; and WO 90/14436. The HCV genome encodes several viral proteins, including E1 and E2. See, e.g., Houghton et al. (1991) Hepatology 14:381-388. Genomic fragments containing sequences encoding these proteins, as well as antigenic fragments thereof, will find use in the present methods. Similarly, the coding sequence for the δ-antigen from HDV is known (see, e.g., U.S. Patent No. 5,378,814).

In like manner, a wide variety of proteins from the herpesvirus family can be used as antigens in the present invention, including proteins derived from herpes simplex virus (HSV) types 1 and 2, such as HSV-1 and HSV-2 glycoproteins gB, gD and gH; antigens from varicella zoster virus (VZV), Epstein-Barr virus (EBV) and cytomegalovirus (CMV) including CMV gB and gH; and antigens from other human herpesviruses such as HHV6 and HHV7. (See, e.g. Chee et al. (1990) Cytomegaloviruses (J.K. McDougall, ed., Springer-Verlag, pp. 125-169; McGeoch et al. (1988) J. Gen. Virol. 69:1531-1574; U.S. Patent No. 5,171,568; Baer et al. (1984) Nature 310:207-211; and Davison et al. (1986) J. Gen. Virol. 67:1759-1816.)

Human immunodeficiency virus (HIV) antigens, such as gp120 molecules for a multitude of HIV-1 and HIV-2 isolates, including members of the various genetic subtypes of HIV, are known and reported (see, e.g., Myers et al., Los Alamos Database, Los Alamos National Laboratory, Los Alamos, New Mexico (1992); and Modrow et al. (1987) J. Virol. 61:570-578) and antigen-containing genomic fragments derived or obtained from any of these isolates will find use in the present invention. Furthermore, other immunogenic proteins derived or obtained from any of the various HIV isolates will find use herein, including fragments containing one or more of the various envelope proteins such as gp160 and gp41, gag antigens such as p24gag and p55gag, as well as proteins derived from the pol, env, tat, vif, rev, nef, vpr, vpu and LTR regions of HIV.

Antigens derived or obtained from other viruses will also find use herein, such as without limitation, antigens from members of the families Picomaviridae (e.g., polioviruses, rhinoviruses, etc.); Caliciviridae; Togaviridae (e.g., rubella virus, dengue virus, etc.); Flaviviridae; Coronaviridae; Reoviridae (e.g., rotavirus, etc.); Bimaviridae; Rhabodoviridae (e.g., rabies virus, etc.); Orthomyxoviridae (e.g., influenza virus types A, B and C, etc.); Filoviridae; Paramyxoviridae (e.g., mumps virus, measles virus, respiratory syncytial virus, parainfluenza virus, etc.); Bunyaviridae; Arenaviridae; Retroviradae (e.g., HTLV-I; HTLV-II; HIV-1 (also known as HTLV-III, LAV, ARV, hTLR, etc.)), including but not limited to antigens from the isolates HIV_{IIIb}, HIV_{SF2}, HIV_{LAV,} HIV_{LAI,} HIV_{MN}); HIV-1_{CM235}, HIV-1_{US4;} HIV-2, among others; simian immunodeficiency virus (SIV); Papillomavirus, the tick-bourne encephalitis viruses; and the like. See, e.g. Virology, 3rd Edition (W.K. Joklik ed. 1988); Fundamental Virology, 2nd Edition (B.N. Fields and D.M. Knipe, eds. 1991), for a description of these and other viruses.

In some contexts, it may be preferable that the selected viral antigens are obtained or derived from a viral pathogen that typically enters the body via a mucosal surface and is known to cause or is associated with human disease, such as, but not limited to, HIV (AIDS), influenza viruses (Flu), herpes simplex viruses (genital infection, cold sores, STDs), rotaviruses (diarrhea), parainfluenza viruses (respiratory infections), poliovirus (poliomyelitis), respiratory syncytial virus (respiratory infections), measles and mumps viruses (measles, mumps), rubella virus (rubella), and rhinoviruses (common cold).

Genomic fragments containing bacterial and parasitic antigens can be obtained or derived from known causative agents responsible for diseases including, but not limited to, Diptheria, Pertussis, Tetanus, Tuberculosis, Bacterial or Fungal Pneumonia, Otitis Media, Gonnorhea, Cholera, Typhoid, Meningitis, Mononucleosis, Plague, Shigellosis or Salmonellosis, Legionaire's Disease, Lyme Disease, Leprosy, Malaria, Hookworm, Onchocerciasis, Schistosomiasis, Trypamasomialsis, Lesmaniasis, Giardia, Amoebiasis, Filariasis, Borelia, and Trichinosis. Still further antigens can be obtained or derived from unconventional viruses such as the causative agents of kuru, Creutzfeldt-Jakob disease (CJD), scrapie, transmissible mink encephalopathy, and chronic wasting diseases, or from proteinaceous infectious particles such as prions that are associated with mad cow disease.

Specific pathogens can include *M. tuberculosis, Chlamydia, N. gonorrhoeae,Shigella, Salmonella, Vibrio Cholera, Treponema pallidua, Pseudomonas, Bordetella pertussis, Brucella, Franciscella tulorensis, Helicobacter pylori, Leptospria interrogaus, Legionella pneumophila, Yersinia pestis,* Streptococcus (types A and B), *Pneumococcus, Meningococcus, Hemophilus influenza* (type b), *Toxoplasma gondic, Complylobacteriosis, Moraxella catarrhalis, Donovanosis,* and *Actinomycosis;* fungal pathogens including Candidiasis and Aspergillosis; parasitic pathogens including Taenia, Flukes, Roundworms, Amebiasis, Giardiasis, Cryptosporidium, Schistosoma, *Pneumocystis carinii,* Tricliomoniasis and Trichinosis. Thus, the present invention can also be used to provide a suitable immune response against numerous veterinary diseases, such as Foot and Mouth diseases, Coronavirus, *Pasteurella multocida, Helicobacter, Strongylus vulgaris, Actinobacillus pleuropneumonia,* Bovine viral diarrhea virus (BVDV), *Klebsiella pneumoniae, E. coli, Bordetella pertussis, Bordetella parapertussis* and *brochiseptica.*

In some embodiments, the antigen of interest can be an allergen. An "allergen" is an antigen which can initiate a state of hypersensitivity, or which can provoke an immediate hypersensitivity reaction in an individual already sensitized with the allergen. Allergens are commonly proteins or chemicals bound to proteins which have the property of being allergenic; however, allergens can also include organic or inorganic materials derived from a variety of man-made or natural sources such as plant materials, metals, ingredients in cosmetics or detergents, latexes, or the like. Classes of suitable allergens for use in the methods of the invention can include, but are not limited to, pollens, animal dander, grasses, molds, dusts, antibiotics, stinging insect venoms, and a variety of environmental (including chemicals and metals), drug and food allergens. Common tree allergens include pollens from cottonwood, popular, ash, birch, maple, oak, elm, hickory, and pecan trees; common plant allergens include those from rye, ragweed, English plantain, sorrel-dock and pigweed; plant contact allergens include those from poison oak, poison ivy and nettles; common grass allergens include Timothy, Johnson, Bermuda, fescue and bluegrass allergens; common allergens can also be obtained from molds or fungi such as Alternaria, Fusarium, Hormodendrum, Aspergillus, Micropolyspora, Mucor and thermophilic actinomycetes; penicillin and tetracycline are common antibiotic allergens; epidermal allergens can be obtained from house or organic dusts (typically fungal in origin), from insects such as house mites (dermatphagoides pterosinyssis), or from animal sources such as feathers, and cat and dog dander; common food allergens include milk and cheese (diary), egg, wheat, nut (e.g., peanut), seafood (e.g., shellfish), pea, bean and gluten allergens; common environmental allergens include metals (nickel and gold), chemicals (formaldehyde, trinitrophenol and turpentine), Latex, rubber, fiber (cotton or wool), burlap, hair dye, cosmetic, detergent and perfume allergens; common drug allergens include local anesthetic and salicylate allergens; antibiotic allergens include penicillin and sulfonamide allergens; and common insect allergens include bee, wasp and ant venom, and cockroach calyx allergens. Particularly well characterized allergens include, but are not limited to, the major and cryptic epitopes of the Der pI allergen (Hoyne et al. (1994) Immunology 83190-195), bee venom phospholipase A2 (PLA) (Akdis et al. (1996) J. Clin. Invest. 98:1676-1683), birch pollen allergen Bet v 1 (Bauer et al. (1997) Clin. Exp. Immunol. 107:536-541), and the multi-epitopic recombinant grass allergen rKBG8.3 (Cao et al. (1997) Immunology 90:46-51). These and other suitable allergens are commercially available and/or can be readily prepared as extracts following known techniques.

Incertain other embodiments, the antigen of interest can be a tumor-specific antigen. For the purposes of the present invention, tumor-specific antigens include, but are not limited to, any of the various MAGEs (melanoma associated antigen E), including MAGE 1, MAGE 2, MAGE 3 (HLA-A1 peptide), MAGE 4, etc.; any of the various tyrosinases (HLA-A2 peptide); mutant ras; mutant p53; and p97 melanoma antigen. Other tumor-specific antigens include the Ras peptide and p53 peptide associated with advanced cancers, the HPV 16/18 and E6/E7 antigens associated with cervical cancers, MUC1-KLH antigen associated with breast carcinoma, CEA (carcinoembryonic antigen) associated with colorectal cancer, gp100 or MART1 antigens associated with melanoma, and the PSA antigen associated with prostate cancer. *The p53* gene sequence is known (see e.g., Harris et al. (1986) Mol. Cell. Biol. 6:4650-4656) and is deposited with GenBank under Accession No. M14694. Thus, the adjuvant compositions of the present invention can be used to carry out immunotherapeutic methods for treating cervical, breast, colorectal, prostate, lung cancers, and melanomas.

Antigens for use with the present invention can be obtained or produced using a variety of methods known to those of skill in the art. In particular, the antigens can be isolated directly from native sources, using standard purification techniques. Alternatively, the antigens can be produced recombinantly using known techniques. See, e.g., Sambrook, Fritsch & Maniatis, Molecular Cloning: A Laboratory Manual, Vols. I, II and III, Second Edition (1989); DNA Cloning, Vols. I and II (D.N. Glover ed. 1985). Antigens for use herein may also be synthesized, based on described amino acid sequences, via chemical polymer syntheses such as solid phase peptide synthesis. Such methods are known to those of skill in the art. See, e.g., J. M. Stewart and J. D. Young, Solid Phase Peptide Synthesis, 2nd Ed., Pierce Chemical Co., Rockford, IL (1984) and G. Barany and R. B. Merrifield, The Peptides: Analysis, Synthesis, Biology, editors E. Gross and J. Meienhofer, Vol. 2, Academic Press, New York, (1980), pp. 3-254, for solid phase peptide synthesis techniques; and M. Bodansky, Principles of Peptide Synthesis, Springer-Verlag, Berlin (1984) and E. Gross and J. Meienhofer, Eds., The Peptides: Analysis, Synthesis, Biology, supra, Vol. 1, for classical solution synthesis.

If desired, polynucleotide sequences coding for the above-described antigens, can be obtained using recombinant methods, such as by screening cDNA and genomic libraries from cells expressing the gene, or by deriving the gene from a vector known to include the same. Furthermore, the desired gene can be isolated directly from cells and tissues containing the same, using standard techniques, such as phenol extraction and PCR of cDNA or genomic DNA. See, e.g., Sambrook et al., *supra,* for a description of techniques used to obtain and isolate DNA. Polynucleotide sequences can also be produced synthetically, rather than cloned.

In those compositions wherein the antigen component will be provided by way of a nucleic acid sequence encoding an antigen of interest, the coding sequence for the selected antigen can be combined with one or both of the coding sequences for the ADP-ribosylating exotoxin subunit peptides to provide a single construct carrying all three coding sequences, combined with just one of the coding sequences for a toxin subunit to provide, e.g., a two plasmid composition, or provided in a separate construct from either a single construct, or multiple constructs carrying the toxin subunit coding sequences. In each of the above cases, the antigen can be operably linked to and under the transcriptional control of the same or different control elements, e.g., promoters and enhancers. In those constructs where a single control element is used to direct transcription of two or more coding sequences, an internal ribosome entry sequence (IRES) can be used to facilitate transcription of the multiple sequences.

### Administration of Polynucleotides

The polynucleotides (nucleic acid molecules containing coding sequences for the selected ADP-ribosylating exotoxin subunit peptides) described herein may be administered by any suitable method. In a preferred embodiment, described below, the polynucleotides are administered by coating one or more suitable constructs (e.g., one or more DNA plasmid constructs) containing the coding sequences for the ADP-ribosylating exotoxin subunit peptides of interest (and, in certain embodiments, a coding sequence for an antigen of interest on the same or a different construct) onto core carrier particles and then administering the coated particles to the subject or cells. However, the polynucleotides of the present invention may also be delivered using a viral vector or using non-viral systems, e.g., naked nucleic acid delivery.

### Viral Vectors

A number of viral based systems have been used for gene delivery. For example, retroviral systems are known and generally employ packaging lines which have an integrated defective provirus (the "helper") that expresses all of the genes of the virus but cannot package its own genome due to a deletion of the packaging signal, known as the psi sequence. Thus, the cell line produces empty viral shells. Producer lines can be derived from the packaging lines which, in addition to the helper, contain a viral vector which includes sequences required in *cis* for replication and packaging of the virus, known as the long terminal repeats (LTRs). The polynucleotide molecule(s) of interest can be inserted into the vector and packaged in the viral shells synthesized by the retroviral helper. The recombinant virus can then be isolated and delivered to a subject (See, e.g., U.S. Patent No. 5,219,740.) Representative retroviral vectors include but are not limited to vectors such as the LHI, N2, INSAL, LSHL and LHL2 vectors described in e.g., U.S. Patent No. 5,219,740, as well as derivatives of these vectors, such as the modified N2 vector described herein. Retroviral vectors can be constructed using techniques well known in the art. See, e.g., U.S. Patent No 5,219,740; Mann et aL (1983) Cell 33:153-159.

Adenovirus based systems have been developed for gene delivery and are suitable for delivering the nucleic acid molecules described herein. Human adenoviruses are double-stranded DNA viruses, which enter cells by receptor-mediated endocytosis. These viruses are particularly well suited for genetic transfer because they are easy to grow and manipulate and they exhibit a broad host range *in vivo* and *in vitro.* For example, adenoviruses can infect human cells of hematopoietic, lymphoid and mycloid origin. Furthermore, adenoviruses infect quiescent as well as replicating target cells. Unlike retroviruses which integrate into the host genome, adenoviruses persist extrachromosomally thus minimizing the risks associated with insertional mutagenesis. The virus is easily produced at high titers and is stable so that it can be purified and stored. Even in the replication-competent form, adenoviruses cause only low level morbidity and are not associated with human malignancies. Accordingly, adenovirus vectors have been developed which make use of these advantages. For a description of adenovirus vectors and their uses *see, e.g.,* Haj-Ahmad and Graham (1986) J. Virol. 57:267-274; Bett et al. (1993) J. Virol. 67:5911-5921; Mittereder et al. (1994) Human Gene Therapy 5:717-729; Seth et al. (1994) J. Virol. 68:933-940; Barr et al. (1994) Gene Therapy 1:51-58*;* Berkner, K.L. (1988) BioTecleniques 6:616-629;rich et al. (1993) Human Gene Therapy 4:461-476.

Adeno-associated viral vectors (AAV) can also be used to administer the polynucleotide molecules described herein. In this regard, AAV vectors can be derived from any AAV serotype, including without limitation, AAV-1, AAV-2, AAV-3, AAV-4, AAV-5, AAVX7, etc. AAV vectors can have one or more of the AAV wild-type genes deleted in whole or in part, preferably the *rep* and/or *cap* genes, but retain one or more functional flanking inverted terminal repeat (ITR) sequences. A functional ITR sequence is generally deemed necessary for the rescue, replication and packaging of the AAV virion. Thus, an AAV vector includes at least those sequences required in *cis* for replication and packaging (e.g., a functional ITR) of the virus. The ITR need not be the wild-type nucleotide sequence, and may be altered, e.g., by the insertion, deletion or substitution of nucleotides, so long as the sequence provides for functional rescue, replication and packaging.

AAV expression vectors are constructed using known techniques to at least provide as operatively linked components in the direction of transcription, control elements including a transcriptional initiation region, the DNA of interest and a transcriptional termination region. The control elements are selected to be functional in a mammalian cell. The resulting construct which contains the operatively linked components is bounded (5' and 3') with functional AAV ITR sequences. Suitable AAV constructs can be designed using techniques well known in the art. *See, e.g.,* U.S. Patent Nos. 5,173,414 and 5,139,941; International Publication Nos. WO 92/01070 (published 23 January 1992) and WO 93/03769 (published 4 March 1993); Lebkowski et al. (1988) Molec. Cell. Biol. 8:3988-3996; Vincent et al. (1990) Vaccines 90 (Cold Spring Harbor Laboratory Press); Carter, B.J. (1992) Current Opinion in Biotechnology 3:533-539; Muzyczka, N. (1992) Current Topics in Microbiol. and Immunol. 158:97-129; Kotin, R.M. (1994) Human Gene Therapy 5 :793-801*;* Shelling and Smith (1994) Gene Therapy 1:165-169; and Zhou et al. (1994) J. Exp. Med. 179:1867-1875.

### Conventional Pharmaceutical Preparations

Formulation of a preparation comprising the polynucleotide molecules of the present invention, with or without addition of an antigen composition, can be carried out using standard pharmaceutical formulation chemistries and methodologies all of which are readily available to the ordinarily skilled artisan. For example, compositions containing one or more suitable vectors can be combined with one or more pharmaceutically acceptable excipients or vehicles to provide a liquid preparation.

Auxiliary substances, such as wetting or emulsifying agents, pH buffering substances and the like, may be present in the excipient or vehicle. These excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can also be included, therein, for example, mineral acid salts such as hydrochlorides, hydrobromides, phosphates, sulfates, and the like; and the salts of organic acids such as acetates, propionates, malonates, benzoates, and the like. It is also preferred, although not required, that the preparation will contain a pharmaceutically acceptable excipient that serves as a stabilizer, particularly for peptide, protein or other like antigen molecules if they are to be included in the vaccine composition. Examples of suitable carriers that also act as stabilizers for peptides include, without limitation, pharmaceutical grades of dextrose, sucrose, lactose, trehalose, mannitol, sorbitol, inositol, dextran, and the like. Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, vehicles and auxiliary substances is available in REMINGTON'S PHARMACEUTICAL SCIENCES (Mack Pub. Co., N.J. 1991), incorporated herein by reference.

Certain facilitators of nucleic acid uptake and/or expression ("transfection facilitating agents") can also be included in the compositions, for example, facilitators such as bupivacaine, cardiotoxin and sucrose, and transfection facilitating vehicles such as liposomal or lipid preparations that are routinely used to deliver nucleic acid molecules. Anionic and neutral liposomes are widely available and well known for delivering nucleic acid molecules (see, e.g., Liposomes: A Practical Approach, (1990) RPC New Ed., IRL Press). Cationic lipid preparations are also well known vehicles for use in delivery of nucleic acid molecules. Suitable lipid preparations include DOTMA (N-[1-(2,3-dioleyloxy)propyl]-N,N,N-trimethylammonium chloride), available under the tradename Lipofectin^{™}, and DOTAP (1,2-bis(oleyloxy)-3-(trimethylammonio)propane), see, e.g., Felgner et al. (1987) Proc. Natl. Acad. Sci. USA 84:7413-7416; Malone et al. (1989) Proc. Natl. Acad Sci. USA 86:6077-6081; US Patent Nos 5,283,185 and 5,527,928, and International Publication Nos WO 90/11092, WO 91/15501 and WO 95/26356. These cationic lipids may preferably be used in association with a neutral lipid, for example DOPE (dioleyl phosphatidylethanolamine). Still further transfection-facilitating compositions that can be added to the above lipid or liposome preparations include spermine derivatives (see, e.g., International Publication No. WO 93/18759) and membrane-permeabilizing compounds such as GALA, Granlicidine S and cationic bile salts (see, e.g., International Publication No. WO 93/19768).

Alternatively, the nucleic acid molecules of the present invention may be encapsulated, adsorbed to, or associated with, particulate carriers. Suitable particulate carriers include those derived from polymethyl methacrylate polymers, as well as PLG microparticles derived from poly(lactides) and poly(lactide-co-glycolides). See, e.g., Jeffery et al. (1993) Pharm. Res. 10:362-368. Other particulate systems and polymers can also be used, for example, polymers such as polylysine, polyarginine, polyornithine, spermine, spermidine, as well as conjugates of these molecules.

The formulated compositions will thus typically include one or more polynucleotide molecule (e.g., plasmid vector) containing the coding sequences for the selected ADP-ribosylating exotoxin subunit peptides in an amount sufficient to adjuvant an immunological response against a co-administered antigen. An appropriate effective amount can be readily determined by one of skill in the art. Such an amount will fall in a relatively broad range that can be determined through routine trials. For example, suitable adjuvant effect may be obtained using as little as 0.1µg of DNA, while in other administrations, up to 2mg of DNA may be used. It is generally expected that an effective dose of polynucleotides containing the ADP-ribosylating exotoxin subunit peptide coding sequences of interest will fall within a range of about 1µg to 1000µg, however, doses above and below this range may also be found effective. The compositions may thus contain from about 0.1% to about 99.9% of the polynucleotide molecules.

### Administration of Conventional Pharmaceutical Preparations

Administration of the above-described pharmaceutical preparations can be effected in one dose, continuously or intermittently throughout the course of treatment. That is, once suitably formulated, the compositions of the present invention can be administered to a subject *in vivo* using a variety of known routes and techniques. For example, the liquid preparations can be provided as an injectable solution, suspension or emulsion and administered via parenteral, subcutaneous, intradermal, intramuscular, intravenous injection using a conventional needle and syringe, or using a liquid jet injection system. Liquid preparations can also be administered topically to skin or mucosal tissue, or provided as a finely divided spray suitable for respiratory or pulmonary administration. Other modes of administration include oral administration, suppositories, and active or passive transdermal delivery techniques.

Alternatively, the compositions can be administered *ex vivo*, for example delivery and reimplantation of transformed cells into a subject are known (e.g., dextran-mediated transfection, calcium phosphate precipitation, electroporation, and direct microinjection of into nuclei). However, delivery will most typically be via conventional needle and syringe for the liquid compositions and for liquid suspensions containing particulate compositions. Methods of determining the most effective means and dosages of administration are well known to those of skill in the art and will vary with the delivery vehicle, the composition of the therapy, the target cells, and the subject being treated. Single and multiple administrations can be carried out with the dose level and pattern being selected by the attending physician. It should be understood that more than one subunit coding region and/or antigen coding region can be carried by a single polynucleotide vector construct. Alternatively, separate vectors (e.g., viral vectors, plasmids, or any combination thereof), each expressing one or more toxin subunit peptide and/or antigen derived from any pathogen can also be delivered to a subject as described herein.

Furthermore, it is also intended that the polynucleotides delivered by the methods of the present invention be combined with other suitable compositions and therapies. For instance, in order to further augment an immune response in a subject, the compositions and methods described herein can be combined with delivery of ancillary substances (e.g., other adjuvants), such as pharmacological agents, cytokines, or the like. Ancillary substances may be administered, for example, as proteins or other macromolecules at the same time, prior to, or subsequent to, administration of the polynucleotiode molecules described herein. The nucleic acid molecule compositions may also be administered directly to the subject or, alternatively, delivered *ex vivo,* to cells derived from the subject, using methods known to those skilled in the art.

### Coated Particles

In one embodiment, polynucleotide constructs containing the coding sequences for the selected ADP-ribosylating exotoxin subunit peptides, and other ancillary components (such as coding sequences for one or more antigens) are delivered using carrier particles. Particle-mediated delivery methods for administering such nucleic acid preparations are known in the art. Thus, once prepared and suitably purified, the above-described plasmid vector constructs can be coated onto carrier particles (e.g., core carriers) using a variety of techniques known in the art. Carrier particles are selected from materials which have a suitable density in the range of particle sizes typically used for intracellular delivery from an appropriate particle delivery device. The optimum carrier particle size will, of course, depend upon the diameter of the target cells.

For the purposes of the present invention, tungsten, gold, platinum and iridium core carrier particles can be used. Tungsten and gold particles are preferred. Tungsten particles are readily available in average sizes of 0.5 to 2.0 µm in diameter. Although such particles have optimal density for use in particle delivery methods, and allow highly efficient coating with DNA, tungsten may potentially be toxic to certain cell types. Accordingly, gold particles or microcrystalline gold (e.g., gold powder A1570, available from Engelhard Corp., East Newark, NJ) will also find use with the present methods. Gold particles provide uniformity in size (available from Alpha Chemicals in particle sizes of 1- - 3 µm, or available from Degussa, South Plainfield, NJ in a range of particle sizes including 0.95 µm) and reduced toxicity.

A number of methods are known and have been described for coating or precipitating DNA or RNA onto gold or tungsten particles. Most such methods generally combine a predetermined amount of gold or tungsten with plasmid DNA, CaCl₂ and spermidine. The resulting solution is vortexed continually during the coating procedure to ensure uniformity of the reaction mixture. After precipitation of the nucleic acid, the coated particles can be transferred to suitable membranes and allowed to dry prior to use, coated onto surfaces of a sample module or cassette, or loaded into a delivery cassette for use in a suitable particle delivery device.

Peptide antigens can also be coated onto the same or similar core carrier particles. For example, peptides can be attached to a carrier particle by simply mixing the two components in an empirically determined ratio, by ammonium sulfate precipitation or other solvent precipitation methods familiar to those skilled in the art, or by chemical coupling of the peptide to the carrier particle. The coupling of L-cysteine residues to gold has been previously described (Brown et al., Chemical Society Reviews 9:271-311 (1980)). Other methods would include, for example, dissolving the peptide in absolute ethanol, water, or an alcohol/water mixture, adding the solution to a quantity of carrier particles, and then drying the mixture under a stream of air or nitrogen gas while vortexing. Alternatively, the antigen can be dried onto carrier particles by centrifugation under vacuum. Once dried, the coated particles can be resuspended in a suitable solvent (e.g., ethyl acetate or acetone), and triturated (e.g., by sonication) to provide a substantially uniform suspension. The core carrier particles coated with the antigen can then be combined with core carrier particles carrying the ADP-ribosylating exotoxin subunit peptide construct(s) and administered in a single particle injection step, or administered separately from the toxin subunit compositions.

### Administration of Coated Particles

Following their formation, core carrier particles coated with the nucleic acid preparations of the present invention, alone or in combination with e.g., antigen preparations, are delivered to a subject using particle-mediated delivery techniques.

Various particle delivery devices suitable for particle-mediated delivery techniques are known in the art, and are all suited for use in the practice of the invention. Current device designs employ an explosive, electric or gaseous discharge to propel the coated core carrier particles toward target cells. The coated particles can themselves be releasably attached to a movable carrier sheet, or removably attached to a surface along which a gas stream passes, lifting the particles from the surface and accelerating them toward the target. An example of a gaseous discharge device is described in U.S. Patent No. 5,204,253. An explosive-type device is described in U.S. Patent No. 4,945,050. One example of an electric discharge-type particle acceleration apparatus is described in U.S. Patent No. 5,120,657. Another electric discharge apparatus suitable for use herein is described in U.S. Patent No. 5,149,655. The disclosure of all of these patents is incorporated herein by reference in their entireties.

The coated particles are administered to the subject to be treated in a manner compatible with the dosage formulation, and in an amount that will be effective to bring about the desired adjuvant effect/immune response. The amount of the composition to be delivered which, in the case of nucleic acid molecules is generally in the range of from 0.001 to 1000 µg, more typically 0.01 to 10.0 µg of nucleic acid molecule per dose, and in the case of peptide or protein molecules is 1 µg to 5 mg, more typically 1 to 50 µg of peptide, depends on the subject to be treated. The exact amount necessary will vary depending on the age and general condition of the individual being immunized and the particular nucleotide sequence or peptide selected, as well as other factors. An appropriate effective amount can be readily determined by one of skill in the art upon reading the instant specification.

### Particulate Compositions

Alternatively, polynucleotides carrying the selected ADP-ribosylating exotoxin subunit peptide coding sequences, as well as one or more selected antigen moiety, can be formulated as a particulate composition. More particularly, formulation of particles comprising one or more polynucleotide molecules can be carried out using standard pharmaceutical formulation chemistries and methodologies all of which are readily available to the reasonably skilled artisan. For example, one or more vector construct and/or antigen component can be combined with one or more pharmaceutically acceptable excipients or vehicles to provide a vaccine composition. Auxiliary substances, such as wetting or emulsifying agents, pH buffering substances, and the like, may be present in the excipient or vehicle. These excipients, vehicles and auxiliary substances are generally pharmaceutical agents that do not themselves induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can be included therein, for example, mineral acid salts such as hydrochlorides, hydrobromides, phosphates, sulfates, and the like; and the salts of organic acids suitable carriers that also act as stabilizers for peptides include, without limitation, pharmaceutical grades of dextrose, sucrose, lactose, trehalose, mannitol, sorbitol, inositol, dextran, and the like. Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, carriers, stabilizers and other auxiliary substances is available in REMINGTON'S PHARMACEUTICAL SCIENCES (Mack Pub. Co., N.J. 1991).

The formulated compositions will include an amount of the toxin subunit peptide-encoding polynucleotides which is sufficient to provide the desired adjuvant effect against a co-administered antigen, as defined above. An appropriate effective amount can be readily determined by one of skill in the art. Such an amount will fall in a relatively broad range, generally within the range of about 0.001 µg to 25 mg or more of the nucleic acid construct of interest, and specific suitable amounts can be determined through routine trials. The compositions may contain from about 0.1 % to about 99.9% of the nucleic acid molecule(s). If an antigen component is included in the composition, or the methods are used to provide a particulate antigen composition, the antigen will be present in a suitable amount as described above. The compositions are then prepared as particles using standard techniques, such as by simple evaporation (air drying), vacuum drying, spray drying, freeze drying (lyophilization), spray-freeze drying, spray coating, precipitation, supercritical fluid particle formation, and the like. If desired, the resultant particles can be densified using the techniques described in commonly owned International Publication No. WO 97/48485.

Single unit dosages or multidose containers, in which the particles may be packaged prior to use, can comprise a hermetically sealed container enclosing a suitable amount of the particles comprising a suitable nucleic acid construct and/or a selected antigen (e.g., to provide a multicomponent vaccine composition). The particulate compositions can be packaged as a sterile formulation, and the hermetically sealed container can thus be designed to preserve sterility of the formulation until use in the methods of the invention. If desired, the containers can be adapted for direct use in a particle delivery device. Such containers can take the form of capsules, foil pouches, sachets, cassettes, and the like. Appropriate particle delivery devices (e.g., needleless syringes) are described herein.

The container in which the particles are packaged can further be labelled to identify the composition and provide relevant dosage information. In addition, the container can be labelled with a notice in the form prescribed by a governmental agency, for example the Food and Drug Administration, wherein the notice indicates approval by the agency under Federal law of the manufacture, use or sale of the adjuvant, antigen (or vaccine composition) contained therein for human administration.

The particulate compositions can then be administered using a transdermal delivery technique. Preferably, the particulate compositions will be delivered via a powder injection method, e.g., delivered from a needleless syringe system such as those described in commonly owned International Publication Nos. WO 94/24263, WO 96/04947, WO 96/12513, and WO 96/20022.

Delivery of particles from such needleless syringe systems is typically practised with particles having an approximate size generally ranging from 0.1 to 250 µm, preferably ranging from about 10-70 µm. Particles larger than about 250 µm can also be delivered from the devices, with the upper limitation being the point at which the size of the particles would cause untoward damage to the skin cells. The actual distance which the delivered particles will penetrate a target surface depends upon particle size (e.g., the nominal particle diameter assuming a roughly spherical particle geometry), particle density, the initial velocity at which the particle impacts the surface, and the density and kinematic viscosity of the targeted skin tissue. In this regard, optimal particle densities for use in needleless injection generally range between about 0.1 and 25 g/cm³, preferably between about 0.9 and 1.5 g/cm³, and injection velocities generally range between about 100 and 3,000 m/sec, or greater. With appropriate gas pressure, particles having an average diameter of 10-70 µm can be accelerated through the nozzle at velocities approaching the supersonic speeds of a driving gas flow.

Compositions containing a therapeutically effective amount of the powdered molecules described herein can be delivered to any suitable target tissue via the above-described particle delivery devices. For example, the compositions can be delivered to muscle, skin, brain, lung, liver, spleen, bone marrow, thymus, heart, lymph, blood, bone cartilage, pancreas, kidney, gall bladder, stomach, intestine, testis, ovary, uterus, rectum, nervous system, eye, gland and connective tissues. For nucleic acid molecules, delivery is preferably to, and the molecules expressed in, terminally differentiated cells; however, the molecules can also be delivered to non-differentiated, or partially differentiated cells such as stem cells of blood and skin fibroblasts.

If desired, these needleless syringe systems can be provided in a preloaded condition containing a suitable dosage of the particles comprising the ADP-ribosylating exotoxin subunit peptide coding sequences of interest, The loaded syringe can be packaged in a hermetically sealed container, which may further be labelled as described above.

Thus, the method can be used to obtain nucleic acid particles having a size ranging from about 10 to about 250 µm, preferably about 10 to about 150 µm, and most preferably about 20 to about 60 µm; and a particle density ranging from about 0.1 to about 25 g/cm³, and a bulk density of about 0.5 to about 3.0 g/cm³, or greater.

Similarly, particles of selected antigens having a size ranging from about 0.1 to about 250 µm, preferably about 0.1 to about 150 µm, and most preferably about 20 to about 60 µm; a particle density ranging from about 0.1 to about 25 g/cm³, and a bulk density of preferably about 0.5 to about 3.0 g/cm³, and most preferably about 0.8 to about 1.5 g/cm³ can be obtained.

### Enhancing Immune Responses

In another embodiment of the invention, a method for enhancing an immune response against a co-administered antigen of interest is provided. In essence, the method entails (a) administering an antigen of interest to a subject, (b) providing an adjuvant composition according to the present invention, wherein the said composition contains one or more nucleic acid molecules containing selected coding sequences for ADP-ribosylating exotoxin subunit peptides, and (c) co-administering the adjuvant composition to the subject, whereby the toxin subunit peptides are expressed from their respective coding sequences in an amount sufficient to elicit an enhanced immune response against the co-administered antigen. As described in detail herein above, the coding sequences are operably linked to the same or different regulatory sequences to provide one or more expression cassettes. These expression cassettes are then provided in a suitable vector, for example a plasmid vector construct or a viral vector.

In one aspect, the method entails administering the polynucleotide composition to a subject using standard gene delivery techniques that are known in the art. See, e.g., U.S. Patent Nos. 5,399,346, 5,580,859, 5,589,466. Typically, the polynucleotide vaccine composition is combined with a pharmaceutically acceptable excipient or vehicle to provide a liquid preparation (as described herein above) and then used as an injectable solution, suspension or emulsion for administration via parenteral, subcutaneous, intradermal, intramuscular, intravenous injection using a conventional needle and syringe, or using a liquid jet injection system. It is preferred that the composition be administered to skin or mucosal tissue of the subject. Liquid preparations can also be administered topically to skin or mucosal tissue, or provided as a finely divided spray suitable for respiratory or pulmonary administration. Other modes of administration include oral administration, suppositories, and active or passive transdermal delivery techniques. The polynucleotide compositions can alternatively be delivered *ex vivo* to cells derived from the subject, whereafter the cells are reimplanted in the subject. Upon introduction into the subject, the nucleic acid sequence is expressed to provide the ADP-ribosylating exotoxin subunit peptides *in situ* in an amount sufficient to enhance an immune response against the co-administered antigen in the vaccinated subject. This enhanced immune response can be characterized as an enhanced humoral (antibody) response, an enhanced cellular (CTL) response, or be characterized as enhancing both humoral and cellular responses against the co-administered antigen.

In certain preferred embodiments, the enhanced immune response is characterized as an augmented Th1-like (cellular) immune response against the co-administered antigen, rather than a Th2-like response. In this regard, the augmented Th1-like immune response can be qualified by one or more of the following: increased titers of interferon-γ producing CD4⁺ helper T lymphocytes and/or CD8⁺ CTLs; increased antigen-specific CTL activity; of increased titers of antigen-specific antibodies of the subclass typically associated with cellular immunity (e.g., IgG2a).

It is preferred that the polynucleotide adjuvant compositions of the present invention be delivered in particulate form. For example, the compositions can be administered using a particle delivery device as described in detail herein above. In certain embodiments, the polynucleotide compositions can be coated onto core carrier particles using a variety of techniques known in the art and delivered using a particle-mediated delivery method. Carrier particles are selected from materials which have a suitable density in the range of particle sizes typically used for intracellular delivery from a particle delivery device. The optimum carrier particle size will, of course, depend on the diameter of the target cells.

These methods can alternatively be modified by co-administration of additional or ancillary components to the subject. For example, a secondary vaccine composition can be administered, wherein the secondary composition can comprise a nucleic acid vaccine, or the secondary vaccine composition can comprise a conventional vaccine such as a whole virus, split virus, or subunit vaccine. The secondary vaccine composition can be combined with the polynucleotide ADP-ribosylating exotoxin subunit peptide compositions to form a single composition, or the secondary vaccine composition can be administered separately to the same or to a different site, either concurrently, sequentially, or separated by a significant passage of time such as in a boosting step some days after the initial composition has been administered.

As above, the secondary vaccine composition and/or other ancillary component can be administered by injection using either a conventional syringe, or using a particle-mediated delivery system as also described above. Administration will typically be either subcutaneously, epidermally, intradermally, intramucosally (e.g., nasally, rectally and/or vaginally), intraperitoneally, intravenously, orally or intramuscularly. Other modes of administration include topical, oral and pulmonary administration, suppositories, and transdermal applications. Dosage treatment may be a single dose schedule or a multiple dose schedule.

### Experimental

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Example 1

### Development of Expression Vectors Encoding the A and B Subunits of CT.

Genomic DNA from *Vibrio cholerae* was used as a template for polymerase chain reactions (PCR) to generate DNA fragments containing the coding sequences for both the A and B subunits of cholera toxin (CT). For PCR generation of the A subunit-encoding fragment (CTA), the following two oligodeoxyribonucleotide primers were used:
Primer 1:5'- GGA GCT AGC AAT GAT GAT AAG TTA TAT CGG -3' (SEQ ID NO: 7); and
Primer 2: 5'- CCT GGA TCC TCA TAA TTC ATC CTT AAT TCT -3'(SEQ ID NO: 8).

In order to facilitate insertion into an expression vector, Primers 1 and 2 contain extra sequences at their 5' ends (outside the region of homology to the CTA coding sequence) which include recognition sites for *Nhe*I and *Bam*HI, respectively. Primers 1 and 2 were designed to lead to PCR generation of a fragment of the A subunit coding sequence starting at nucleotide position 164 and ending at nucleotide position 886 (GenBank Accession #D30053). This region encompasses the entire coding sequence for the mature subunit A peptide but does not include the sequence encoding the bacterial signal peptide found at the amino terminus of the pre-subunit A peptide.

For PCR generation of the B subunit-encoding fragment (CTB), the following two oligodeoxyribonucleotide primers were used:
Primer 3:5'- GGA GCT AGC ACA CCT CAA AAT ATT ACT GAT -3' (SEQ ID NO: 9); and
Primer 4: 5'- CCT GGA TCC TTA ATT TGC CAT ACT AAT TGC -3'(SEQ ID NO: 10).

In order to facilitate insertion into an expression vector, Primers 3 and 4 contain extra sequences at their 5' ends (outside the region of homology to the CTB coding sequence) which include recognition sites for *Nhe*I and *Bam*HI, respectively. Primers 3 and 4 were designed to lead to PCR generation of a fragment of the B subunit coding sequence starting at nucleotide position 946 and ending at nucleotide position 1257 (GenBank Accession #D30053). This region encompasses the entire coding sequence for the mature subunit B peptide but does not include the sequence encoding the bacterial signal peptide found at the amino terminus of the pre-subunit B peptide.

In addition to the two PCR reactions described above, a third PCR reaction was performed to generate a coding sequence for a modified form of subunit A of CT in which the C-terminal four amino acid KDEL motif was eliminated. This reaction involved the use of Primer 1 (SEQ ID NO: 7) and the following primer:
Primer 5: 5'- CCT GGA TCC TCA AAT TCT ATT ATG TGT ATC -3'(SEQ ID NO: 11).

All PCR reactions were performed using Pfu Turbo DNA polymerase (obtained from Strategene, La Jolla, CA) along with the PCR reaction buffer supplied by the manufacturer. PCR conditions were as follows: 95°C for 2 min., 30 cycles of (95°C for 1 min., 55°C for 2 min. 15 sec., 72°C for 1 min.), 72°C for 5 min., and 4°C hold.

Following completion of the PCR reactions, the newly synthesized fragments were digested with *Nhe*I and *Bam*HI enzymes to generate cohesive ends, and the individual fragments were inserted into the *Nhe*I- and *Bam*HI-cleaved pWRG7054 expression vector resulting in the following clones: pPJV2002, pPJV2003, and pPJV2006 encoding CTA, CTB, and modified CTA (minus KDEL), respectively. The parental cloning vector pWRG7054 contains the human cytomegalovirus immediate early promoter with the associated intron A sequence. In addition, the coding sequence for the signal peptide of human tissue plasminogen activator is included in pWRG7054 to allow for the secretion from mammalian cells of any protein whose coding sequence is inserted at the *Nhe*I site in the appropriate reading frame. (See, e.g., Chapman et al. (1991) Nuc. Acids Res. 19:3979-3986, and Burke et al. (1986) J. Biol. Chem. 261:12574-12578.)

The restriction maps and full sequences of plasmids pPJV2002, pPJV2003, and pPJV2006 are depicted in Figures 1, 2, and 3, respectively.

### Example 2

### Development of Expression Vectors Encoding the A and B Subunits of E. coli Heat Labile Enterotoxin.

Genomic DNA from *E. coli* strain E078:H11 (American Type Culture Collection #35401) was used as a template for polymerase chain reactions to generate DNA fragments containing the coding sequences for both the A and B subunits of the heat labile enterotoxin (LT). For PCR generation of the A subunit-encoding fragment, the following two oligodeoxyribonucleotide primers were used:
Primer 6: 5'- GGA GCT AGC AAT GGC GAC AAA TTA TAC CGT -3' (SEQ ID NO: 12); and
Primer 7: 5'- CCT GGA TCC TCA TAA TTC ATC CCG AAT TCT -3'(SEQ ID NO: 13).

In order to facilitate insertion into an expression vector, Primers 6 and 7 contain extra sequences at their 5' ends (outside the region of homology to the LTA coding sequence) which include recognition sites for *Nhe*I and *Bam*HI, respectively. Primers 6 and 7 were designed to lead to PCR generation of a fragment of the A subunit coding sequence starting at nucleotide position 145 and ending at nucleotide position 867 of the sequence found in the GenBank database (Accession #AB011677). This region encompasses the entire coding sequence for the mature subunit A peptide but does not include the sequence encoding the bacterial signal peptide found at the amino terminus of the pre-subunit A peptide.

For PCR generation of the B subunit-encoding fragment, the following two oligodeoxyribonucleotide primers were used:
Primer 8:5'- GGA GCT AGC GCT CCC CAG TCT ATT ACA GAA -3' (SEQ ID NO: 14); and
Primer 9: 5'- CCT GGA TCC CTA GTT TTC CAT ACT GAT TGC -3'(SEQ ID NO: 15).

In order to facilitate insertion into an expression vector, Primers 8 and 9 contain extra sequences at their 5' ends (outside the region of homology to the LTB coding sequence) which include recognition sites for *Nhe*I and *Bam*HI, respectively. Primers 8 and 9 were designed to lead to PCR generation of a fragment of the B subunit coding sequence starting at nucleotide position 927 and ending at nucleotide position 1238 of the sequence found in the GenBank database (Accession #AB011677). This region encompasses the entire coding sequence for the mature subunit B peptide but does not include the sequence encoding the bacterial signal peptide found at the amino terminus of the pre-subunit B peptide.

In addition to the two PCR reactions described above, a third PCR reaction was performed to generate a coding sequence for a modified form of subunit A of LT in which the C-terminal four amino acid RDEL motif was eliminated. This reaction involved the use of Primer 6 (SEQ ID NO: 12) and the following primer:
Primer 10: 5'- CCT GGA TCC TCA AAT TCT GTT ATA TAT GTC -3' (SEQ ID NO: 16).

All PCR reactions were performed using Pfu Turbo DNA polymerase (obtained from Strategene, La Jolla, CA) along with the PCR reaction buffer supplied by the manufacturer. PCR conditions were as follows: 95°C for 2 min., 30 cycles of (95°C for 1 min., 55°C for 2 min. 15 sec., 72°C for 1 min.), 72°C for 5 min., 4°C hold.

Following completion of the PCR reactions, the newly synthesized fragments were digested with *Nhe*I and *Bam*HI to generate cohesive ends and the individual fragments were inserted into the *Nhe*I- and *Bam*HI-cleaved pWRG7054 expression vector resulting in clones pPJV2004, pPJV2005, and pPJV2007 encoding LTA, LTB, and modified LTA (minus RDEL), respectively.

The restriction maps and complete sequences of plasmids pPJV2004, pPJV2005, and pPJV2007 are shown in Figures 4, 5 and 6, respectively.

### Example 3

### Enhancement of Antigen-Specific Antibody Response to a DNA Vaccine Using Plasmid Vectors Encoding CTA and/or CTB.

A DNA vaccine vector encoding the M2 protein of influenza A virus was employed to test the adjuvant effects of the pPJV2002, pPJV2003, and pPJV2006 adjuvant vectors in the context of particle-mediated DNA vaccination. Particle-mediated DNA vaccination was performed by precipitating the M2 DNA vaccine vector, with our without various combinations of the pPJV2002, pPJV2003 and pPJV2006 adjuvant vectors, onto microscopic gold particles and accelerating the coated gold particles into the epidermis of mice using a PowderJect® XR-1 particle delivery device (PowderJect Vaccines, Inc. Madison, WI).

More particularly, the sequence from the RNA segment #7 (that encodes the M2 protein) of influenza virus strain A/Kagoshima/10/95 (H3N2) was used as a model to design PCR primers to facilitate cloning of the mature M2 coding sequence from A/Sydney/5/97 (H3N2). The A/Kagoshima sequence was used for primer design since the sequence of RNA segment 7 of A/Sydney has not yet been determined. The high degree of conservation among M2 sequences was expected to facilitate the use of primers designed from a different viral strain.

Since M2 is translated from a spliced RNA, it was deemed necessary that nucleotide positions 27 to 714 in the coding region of segment 7 RNA were spliced out. Accordingly, a set of PCR primers was generated and designed to generate the complete M2 coding sequence but ensure that the intron was cleanly eliminated from resulting M2 coding sequence clone. The PCR primers used to generate the full-length M2 coding sequence clone were as follows:
Primer 11: 5'-CCC AAG CTT CCA CCA TGA GCC TTC TAA CCG AGG TCG AAA CAC CTA TCA GAA ACG AAT GGG AGT GC-3'(SEQ ID NO: 17); and
Primer 12: 5'-CCC GGA TCC TTA CTC CAG CTC TAT GCT G-3'(SEQ ID NO: 18).

Primer 11 (SEQ ID NO: 17) contains additional sequences at its 5' end that include a recognition site for *Hind*III and a Kozak consensus sequence to facilitate mRNA translation initiation. Also, Primer 12 (SEQ ID NO: ) contains additional sequences at its 5' end that includes a recognition sequence for *Bam*HI.

Viral RNA was isolated from a sample of A/Sydney/5/97 (H3N2) that was grown in embryonated chicken eggs. The viral RNA isolation process used standard techniques known to those skilled in the art. RNA from this virus was used in a reverse transcriptase / polymerase chain reaction (RT-PCR) using an RT-PCR kit obtained from Stratagene (La Jolla, CA). The RT reaction step was completed by adding 5.9µl of RNase-free water to a reaction tube. To this tube was added 1.0µl 10X MMLV-RT buffer and 1.0µl dNTP mix from the kit. Also, 1µl of A/Sydney/5/97 RNA and 0.6µl (0.6µg) of Primer 11 (SEQ ID NO: 17) was added. The reaction was heated to 65°C for 5 minutes to denature the RNA, after which 0.5µl of reverse transcriptase from the kit was added. The reaction was incubated at 37°C for 15 minutes to complete the reverse transcription step.

The PCR reaction step was completed by addition of the following components to a new reaction tube: 40µl water; 5µl 10X ultra HF buffer from the kit; 1.0µl dNTP mix from the kit; 1.0µl Primer 11 (SEQ ID NO: 17) (1.0µg); 1.0µl Primer 12 (SEQ ID NO: 18) (1.0µg); 1µl of the reverse transcriptase reaction mix from above; and 1µl Turbo PFU polymerase from the kit. The PCR reaction was carried out using the following incubation scheme: 1 minute @ 95°C; followed by 30 cycles of (30 sec @ 95°C, 30 sec @ 46°C, 3 min @ 68°C), followed by 10 minutes @ 68°C. PCR products were electrophoresed on a 2% agarose gel revealing a single DNA band of the expected size of approximately 300 bp.

The approximately 300 bp band was isolated from the gel and digested with *Hind*III and *Bam*HI in order to generate the necessary sticky ends for insertion into the pWRG7077 DNA vaccine expression vector (Schmaljohn et al. (1997) J. Virol. 71:9563-9569). The pWRG7077 DNA was digested partially with *Hind*III and completely with *Bam*HI to facilitate insertion of the M2 coding insert. The requirement for a partial *Hind*III digestion of the vector was due to the presence of a second *Hind*III site in the Kanamycin resistance marker of this plasmid. The resulting M2 DNA vaccine vector was termed pM2-FL. The pM2-FL vector contains the immediate early promoter from human cytomegalovirus (hCMV) and its associated intron A sequence to drive transcription from the M2 coding sequence. This vector also includes a polyadenylation sequence from the bovine growth hormone gene.

The pM2-FL plasmid was then precipitated onto 2 micron gold particles as single vector, or mixed vector plus adjuvant vector samples (i.e., the M2 plasmid vector was combined with pPJV2002, pPJV2003, and/or pPJV2006 adjuvant vectors). Specifically, plasmid DNA (single M2 vector or M2 vector plus one or more adjuvant vectors) was mixed with 2 micron gold particles (Degussa, Lot 65-0) in a small centrifuge tube containing 400 µl of 50 mM spermidine. The DNA-to-gold ratio varied from 2.5 µg DNA per mg of gold to 4.0 µg of DNA per mg of gold, and a single batch contained 26 mg of gold. DNA was precipitated onto the gold particles by addition of a 1/10 volume of 10% CaCl₂ during continuous agitation of the tube on a rotary mixer. DNA-gold complexes were washed three times with absolute ethanol, and then injected into a TEFZEL® tube (McMaster-Carr) housed in a tube turner coating machine (PowderJect Vaccines, Inc., Madison WI) which coats the inside of the tube with the gold/DNA complex. This tube turner machine is described in US Patent No. 5,733,600. See also PCT patent application PCT/US95/00780 and US Patent Nos. 5,780,100; 5,865,796 and 5,584,807 After the coating procedure was completed, the tubes were cut into 0.5 inch "cartridges" suitable for loading into a particle delivery device.

The following DNA-gold formulations were generated for a mouse DNA vaccine adjuvant trial.
Formulation #1: pM2-FL DNA vector alone, 2.5 µg DNA per mg gold, 0.5 mg gold per cartridge;
Formulation #2: pM2-FL DNA vector precipitated onto one batch of gold (2 µg pM2-FL DNA per mg gold), pPJV2002 and pPJV2003 DNA vectors coprecipitated onto a second batch of gold (1.75 µg of each DNA adjuvant vector per mg gold), the gold batches were mixed equally, 0.5 mg gold per cartridge;
Formulation #3: pM2-FL DNA vector, pPJV2002 and pPJV2003 DNA vectors all coprecipitated onto single batch of gold (2 µg pM2-FL DNA per mg gold, and 1 µg each of pPJV2002 and pPJV2003 per mg gold), 0.5 mg gold per cartridge;
Formulation #4: pM2-FL DNA vector, pPJV2006 and pPJV2003 DNA vectors all coprecipitated onto single batch of gold (2 µg pM2-FL DNA per mg gold, and 1µg each of pPJV2006 and pPJV2003 DNA per mg gold), 0.5 mg gold per cartridge;
Formulation #5: pM2-FL DNA vector and pPJV2002 coprecipitated onto single batch of gold (2 µg pM2-FL DNA per mg gold, and 1 µg of pPJV2002 DNA per mg gold), 0.5 mg gold per cartridge; and
Formulation #6: pM2-FL DNA vector and pPJV2003 coprecipitated onto single batch of gold (2 µg pM2-FL DNA per mg gold, and 1 µg of pPJV2003 DNA per mg gold), 0.5 mg gold per cartridge.

These DNA vaccine formulations were then administered to six groups of mice as follows. Each experimental group contained 7 animals and each animal received two immunizations with the respective formulation with a 4 week resting period between immunizations. Each immunization consisted of two tandem deliveries to the abdominal epidermis (one cartridge per delivery) using a PowderJect® XR-1 particle delivery device (PowderJect Vaccines Inc., Madison, WI) at a helium pressure of 400 p.s.i.. Serum samples were collected 4 weeks following the primary immunization (just before the boost) and two weeks following the second or booster immunization.

Individual serum samples were assayed for M2-specific antibody responses using an ELISA assay in which 96-well plates were pre-coated with an M2 synthetic peptide consisting of the following sequence:
SLLTEVETPIRNEWECR (SEQ ID NO: 19). ELISA plates were coated with the M2 peptide overnight at 4°C using the peptide in phosphate buffered saline (PBS) at a concentration of 1 µg/ml. On the next day, the plates were blocked with 5% nonfat dry milk in PBS for 1 hour at room temperature. Plates were then washed three times with wash buffer (10 mM Tris Buffered Saline, 0.1% Brij-35). Diluted serum samples were then added to the wells and the plates were incubated for 2 hours at room temperature. The plates were washed three times with wash buffer and 100 µl of a secondary antibody was added and plates were incubated for 1 hour at room temperature. The secondary antibody consisted of a goat anti-mouse IgG (H+L) biotin-labeled antibody (Southern Biotechnology) that was diluted 1:8000 in 1% BSA/PBS/0.1% Tween-20. Plates were then washed three times, and a streptavidin-horse radish peroxidase conjugate (Southern Biotechnology) diluted to 1:8000 in PBS/0.1% Tween-20 was added and the plate incubated for 1 hour at room temperature. Following three additional washes, 100 µl of TMB substrate (Bio Rad, Hercules, CA) was added and color development was allowed to proceed for 30 minutes at room temperature. Color development was stopped by the addition of 1N H₂SO₄ and the plates were read of 450 nm. Endpoint dilution titers were determined by identifying the highest dilution of serum that still yielded an absorbance value that was two times the background absorbance value obtained using a non immune control sample.

Endpoint antibody titers for individual animals in each group and geometric mean titers for each experimental group are shown in Table 1 below.

**TABLE 1**

| **Formulation #** | **Individual Titers** | **Geometric Mean Titer** |
|---|---|---|
| 1 | 24,300 | 99,781 |
| | 24,300 | |
| | 72,900 | |
| | 218,700 | |
| | 218,700 | |
| | 218,700 | |
| | 218,700 | |
| 2 | 72,900 | 186,934 |
| | 72,900 | |
| | 218,700 | |
| | 218,700 | |
| | 218,700 | |
| | 656,100 | |
| 3 | 24,300 | 186,934 |
| | 72,900 | |
| | 72,900 | |
| | 218,700 | |
| | 656,100 | |
| | 656,100 | |
| | 656,100 | |
| 4 | 24,300 | 350,211 |
| | 218,700 | |
| | 656,100 | |
| | 656,100 | |
| | 656,100 | |
| | 656,100 | |
| | 656,100 | |
| | 656,100 | |
| 5 | ----* | 262,645 |
| | 72,900 | |
| | 218,700 | |
| | 218,700 | |
| | 218,700 | |
| | 656,100 | |
| | 656,100 | |
| 6 | 24,300 | 409,722 |
| | 72,900 | |
| | 218,700 | |
| | 218,700 | |
| | 1,968,300 | |
| | 1,968,300 | |
| | 5,904,900 | |

| | | |
|---|---|---|
| * (deceased) | | |

As can be seen, all experimental groups immunized with a formulation containing one or more of the CT-encoding adjuvant vectors (Formulations #2-6) exhibited an increased geometric mean titer following the booster immunization relative to control animals immunized with the M2 vector (Formulation #1) alone.

### Example 4

### Enhancement of Antigen-Specific Cellular Responses to a DNA Vaccine Encoding the Hepatitis B Surface Antigen (HBsAg) Using Adjuvant Plasmid Vectors Encoding the CT-A and CT-B Subunit Peptides.

A Hepatitis B surface antigen (HBsAg) vector plasmid was constructed as follows. To generate the HbsAg coding region, the pAM6 construct (obtained from the American Type Culture Collection "ATCC") was cut with *Nco*I and treated with mung bean nuclease to remove the start codon of the X-antigen. The resultant DNA was then cut with *Bam*HI and treated with T4 DNA polymerase to blunt-end the DNA and create an HBsAg expression cassette. The HBsAg expression cassette is present in the 1.2 kB fragment. The plasmid construct pPJV7077 (Schmaljohn et al. (1997) J. Virol. 71:9563-9569) which contains the full-length human CMV (Towne strain) immediate early promoter (with enhancer) was cut with *Hind*III and *Bgl*II, and then treated with T4 DNA polymerase and calf-alkaline phosphatase to create blunt-ended DNA, and the HBsAg expression cassette was ligated into the plasmid to yield the pWRG7128 construct.

The pWRG7128 plasmid was precipitated onto gold particles following the procedure described in Example 3 above, again using 2 µg DNA per mg gold. Adjuvant plasmid vectors expressing the CTA and CTB subunit genes (pPJV2002 and pPJV2003, respectively) were mixed together and precipitated onto gold particles with each plasmid being present at 1 µg DNA per mg gold such that the total amount was also 2 µg DNA per mg gold. The gold particles coated with pWRG7128 and those coated with the pPJV2002 and pPJV2003 were mixed 1:1 and then loaded into TEFZEL® tubing as above. For immunization, 0.5 inch lengths of tubing representing 1 µg DNA (0.5 µg pWRG7128 plasmid and 0.25 µg of each of pPJV2002 and pPJV2003 plasmid) were delivered into the epidermis of Balb/c mice using the PowderJect® XR-1 particle delivery device using the same delivery conditions as described above in Example 3. For controls, mice were immunized with gold particles coated only with pWRG7128 (1 µg DNA/0.5 mg gold per delivery). Mice (4 per experimental group) were immunized and boosted at 4 weeks, then sacrificed at 2 weeks post-boost. Immune responses were evaluated for serum antibody levels by ELISA. In addition, cellular immune responses were measured using an ELISPOT assay to quantify CD8-specific IFN-γ secretion.

Serum samples of individual mice were tested for antibodies specific for HBsAg using an ELISA assay. For the ELISA, Falcon Pro Bind microtiter plates were coated overnight at 4°C with purified HBsAg (BioDesign) at 0.1 µg per well in PBS (phosphate buffered saline, BioWhittaker). The plates were blocked for 1 hour at room temperature (RT) with 5% dry milk/PBS then washed 3 times with wash buffer (10 mM Tris Buffered saline, 0.1% Brij-35), and serum samples diluted in dilution buffer (2% dry milk/PBS/0.05 % Tween 20) were added to the plate and incubated for 2 hours at RT. The plates were washed 3 times and a biotinylated goat anti-mouse antibody (Southern Biotechnology) diluted 1:8000 in dilution buffer was added to the plate and incubated for 1 hr at RT. Following the incubation, plates were washed 3 times, after which a Streptavidin-Horseradish peroxidase conjugate (Southern Biotechnology) diluted 1:8000 in PBS was added and the plate incubated a further 1 hr at RT. After an additional three washes, Plates were washed 3 times, then a TMB substrate solution (BioRad) was added and the reaction was stopped with 1N H₂SO₄ after 30 minutes. Optical density was read at 450 nm. Endpoint titers were calculated by comparison of the samples with a standard of known titer.

For the cellular immune assays, single cell suspensions of splenocytes from the spleens of the immunized animals were cultured *in vitro* in the presence of a peptide corresponding to a known CD8 epitope in Balb/c mice. The peptide was dissolved in DMSO (10 mg/ml) and diluted to 10 ug/ml in culture. The sequence of the peptide was IPQSLDSWWTSL (SEQ ID NO: 20).

For IFN-γ ELISPOT assays, Millipore Multiscreen membrane filtration plates were coated with 50 µl of 15 µg/ml anti-IFN-γ antiserum (Pharmingen) in sterile 0.1 M carbonate buffer (pH 9.6) overnight at 4°C. Plates were washed 6 times with sterile PBS and then blocked with tissue culture medium containing 10% fetal bovine serum (FBS) for 1-2 hr at RT. The medium was removed and spleen cells dispensed into the wells with a total of 1x10⁶ cells per well. For wells in which less than 1x10⁶ cells from immunized animals was added, cells from naïve animals were used to bring the total to 1x10⁶. Cells were incubated overnight in a tissue culture incubator in the presence of the peptide as described above. The plates were then washed 2 time with PBS and 1 time with distilled water. This was followed by 3 washes with PBS. A biotinylated anti IFN-γ monoclonal antibody (Pharmingen) was added to the plate (50 µl of a 1 µg/ml solution in PBS) and incubated for 2 hr at RT. The plates were washed 6 times with PBS after which 50 µl of a Streptavidin Alkaline phosphatase conjugate (1:1000 in PBS, Pharmingen) was added and incubated for 2 hr at RT. The plates were washed 6 times with PBS and an alkaline phosphatase color substrate (BioRad) was added and the reaction was allowed to proceed until dark spots appeared. The reaction was stopped by washing with water 3 times. Plates were air dried and spots counted under a microscope.

For the IFN-γ ELISA assays, the cells were cultured overnight in round bottom 96 well tissue culture plates in the presence of the peptide. Samples of the supernatant were taken and used for the determination of IFN-γ levels. High binding plates (Costar) were coated with 100 µl of 0.5 ug/ml of anti-mouse IFN-γ antibody (Pharmingen) in bicarbonate buffer pH 9.6. Plates were blocked for 1 hr at RT with tissue culture medium containing 10% FBS then washed 3 times with a TBS wash buffer. Supernatant samples obtained from cultured cells were diluted in tissue culture medium and loaded onto the plate and incubated for 2 hr at RT. Plates were washed 3 times with wash buffer and a secondary antibody (0.5 µg/ml of biotinylated rat anti-mouse INF-γ in PBS, Pharmingen) was added to the plates and incubated for 1 hr at RT. Plates were washed 3 times, and a Streptavidin-horseradish peroxidase conjugate (1:2000 in PBS, Southern Biotechnology) was added for 1 hr at RT. Plates were washed 3 times, then a TMB substrate solution was added (BioRad) and the reaction was stopped with 1N H₂SO₄. Optical density was read at 450 nm.

The results of the ELISA are reported below in Table 2.

**TABLE 2**

| **Formulation** | **Individual Titers** | **Average Titers** |
|---|---|---|
| pWRG7128 | 40,000 | 55,000 |
| | 84,000 | |
| | 41,000 | |
| | 54,000 | |
| pWRG7128, | 28,000 | 23,000 |
| pPJV2002 and | 14,000 | |
| pPJV2003 | 27,000 | |
| | 23,000 | |

As can be seen, antibody levels in control mice immunized with pWRG7128 alone were found to be higher than in those immunized with pWRG7128 in combination with the CT adjuvant vectors (pPJV2002 and pPJV2003). The average endpoint titer for the control animals was 55,000, while the average titer in the animals immunized with the adjuvanted formulation was 23,000. However, the group receiving the adjuvanted formulation actually received ½ the amount of pWRG7128 as the controls, which may account for the reduction in antibody titers.

The cellular immune responses measured in the two groups of mice indicate a significant enhancement of cellular responses by the CT adjuvant vectors. More particularly, the results of the CD8-specific IFN-γ ELISA assay are depicted below in Table 3.

**TABLE 3**

| **IFN-γ ELISA** | | | |
|---|---|---|---|
| **Formulation** | **Number of Cells/Well** | | |
| pWRG7128 | **1.0x10⁶** | **0.5x10⁶** | **0.1 x 10⁶** |
| | 0.77 | 0.213 | 0.001 |
| | 0.828 | 0.121 | 0.027 |
| | 1.35 | 0.312 | 0.006 |
| | 1.25 | 0.323 | 0.007 |
| **(Average)** | **1.05** | **0.242** | **0.01** |
| pWRG7128, | 1.96 | 1.19 | 0.079 |
| pPJV2002 and | 2.30 | 1.70 | 0.263 |
| pPJV2003 | 2.20 | 1.83 | 0.377 |
| | 2.44 | 2.33 | 0.898 |
| **(Average)** | **2.23** | **1.76** | **0.404** |

In this IFN-γ ELISA assay, the number of cells per well represents the number of cells recovered from immunized animals plated per well. Total number of cells per well is constant (e.g., 1x10⁶) and is supplemented with cells from naive animals. Values are the OD readings measured at 450 nm. The higher OD values in the ELISA found for the cells from mice treated with the CT adjuvanted formulation is indicative of a greater amount of IFN-γ secreted by these cells in response to antigen. Cells from naïve mice did not yield a measurable OD value in the IFN-γ ELISA.

The results of the CD8-specific IFN-γ ELISPOT assay are depicted below in Table 4.

**TABLE 4**

| **IFN-γ ELISPOTs** | |
|---|---|
| **Formulation** | **Number of Positive Cells/1 x 10⁶ Cells** |
| pWRG7128 | 510 |
| | 410 |
| | 530 |
| | 590 |
| **(Average)** | **510** |
| pWRG7128, | 1,480 |
| pPJV2002 and | 2,300 |
| pPJV2003 | 2,500 |
| | 3,500 |
| **(Average)** | **2,445** |

In this IFN-γ ELISPOT assay, the numbers are the average of duplicate wells and correspond to number of positive cells per 1x10⁶ cells. Cells from naive mice did not yield any spots in this ELISPOT assay. In addition, the greater number of ELISPOTs found in the animals treated with the CT adjuvanted formulation indicates a superior response as compared with the animals receiving the antigen (pWRG7128) alone.

The above data demonstrate that the novel adjuvant compositions of the present invention have a potent ability to enhance the cellular immune responses to a coadministered antigen, in this case a HBsAg expressed from a DNA vaccine.

### Example 5

### Enhancement of Humoral and Cellular Immune Responses to HIV-1 gp120 Antigen Using Simultaneous Delivery of a gp120 Antigen Vector and CTA/CTB Adjuvant Vectors.

A plasmid vector endoding HIV-1 gp120 was constructed as follows. The vector was constructed starting with a Bluescript (Stratagene, La Jolla, CA) plasmid backbone, the human cytomegalovirus (hCMV) immediate early promoter (Fuller et al. (1994) Aids Res. Hum Retroviruses 10:1433) and the SV40 virus late polyadenylation site. The hCMV promoter is contained within a 619 base pair (bp) *Acc*II fragment extending 522 bp upstream and 96 bp downstream from the immediate early transcription initiation site. The SV40 virus late polyadenylation sequence is contained within an approximately 800 bp *Bam*HI*-Bgl*II fragment derived from pSV2dhfr (formerly available from Bethesda Research Laboratories, catalogue #5369 SS). Initially, a plasmid encoding HIV-1 gp160, termed "pC-Env"was constructed. This plasmid contains a 2565 bp *Kpn*I-*Xho*I fragment from LAV-1_{BRU} (ATCC Accession No. 53069, GenBank Accession No. K02013), which begins at the sequence encoding amino acid position #4 of the mature gp160 amino terminus. The *env* coding sequence fragment was placed immediately downstream of, and fused in frame with a 160 bp synthetic fragment encoding the herpes simplex virus glycoprotein D (gD) signal peptide and none amino acids of the mature gD amino terminus as previously described (Fuller et al. (1994) Aids Res. Hum Retroviruses 10: 1433).

The plasmid encoding HIV-1 gp120, termed "pCIA-Env/T" herein, was then constructed as follows. The pCIA-Env/T plasmid encodes a truncated form of HIV-1 gp160, and is identical to the pC-Env construct except that the *env* coding sequences are truncated at the *Hind*III site at nucleotide position 8188. This results in a truncated gp160 translation product with the truncation point lying 128 amino acid residues downstream of the gp120/gp41 processing site.

A second plasmid vector encoding HIV-1 rev, termed "pC-rev" herein, was constructed as follows. This vector contains three discontinuous regions of the LAV-1_{BRU} provirus (nucleotide positions 678-1085, 5821-6379, and 8188-8944) placed directly between the hCMV promoter and the SV40 virus late polyadenylation sequence as described above. The three discontinuous regions contain the major 5' splicing donor, the first exon of the rev gene, and the second exon of the *rev* gene, respectively.

A third plasmid vector construct termed "pWRG7054" was used as an empty vector control in the study. The pWRG7054 construct contains an SIV *nef* coding region, the hCMV promoter immediate early with the Intron A region, a TPA leader sequence and the bovine growth hormone polyadenylation sequence. Construction of the pWRG7054 plasmid is described herein below in Example 6.

The following DNA-gold formulations were generated for a mouse DNA vaccine adjuvant trial.
Formulation #1: Empty vector control (pWRG7054 without the gp120 insert), 2.5 µg DNA per mg gold, 0.5 mg gold per cartridge;
Formulation #2: Empty vector control (pWRG7054), the gp120 (pCIA-EnvT) DNA vector, and the rev (pC-rev) DNA vector (to allow for expression of the HIV-1 gp120 molecule), all coprecipitated onto a single batch of gold (1.25 µg of each of pWRG7054 DNA and pCIA-EnvT DNA per mg gold, and 0.125 µg of pC-rev per mg gold), 0.5 mg gold per cartridge;
Formulation #3: Empty vector control (pWRG7054), CTA (pPJV2002) and CTB (pPJV2003) DNA vectors all coprecipitated onto a single batch of gold (1.25 µg pWRG7054 DNA per mg gold, and 1 µg each of pPJV2002 and pPJV2003 DNA per mg gold), 0.5 mg gold per cartridge;
Formulation #4: gp120 (pCIA-EnvT) DNA vector, HIV-1 rev (pC-rev) DNA vector, CTA (pPJV2002) and CTB (pPJV2003) DNA vectors all coprecipitated onto a single batch of gold (1.25 µg pCIA-EnvT DNA per mg gold, 0.125 µg pC-rev DNA per mg gold, and 1 µg each of pPJV2002 and pPJV2003 DNA per mg gold), 0.5 mg gold per cartridge; and
Formulation #5: gp120 (pCIA-EnvT) DNA vector, HIV-1 rev (pC-rev) DNA vector, CTA -KDEL (pPJV2006) and CTB (pPJV2003) DNA vectors all coprecipitated onto a single batch of gold (1.25 µg pCIA-EnvT DNA per mg gold, 0.125 µg pC-rev DNA per mg gold, and 1 µg each of pPJV2006 and pPJV2003 DNA per mg gold), 0.5 mg gold per cartridge.

The pWRG7054, pCIA-EnvT, pC-rev, pPJV2002, pPJV2003 and pPJV2006 plasmid vectors were precipitated onto gold particles following the procedures described in Example 3 above, again using 2 µg DNA per mg gold. The coated gold particles were loaded into TEFZEL® tubing, again using the procedures described in Example 3 above. For immunization, two 0.5 inch lengths of tubing were used to deliver a total payload of 1.0 mg gold into the epidermis of 5-6 week old female Balb/c mice using a particle delivery device operated under the same delivery conditions as described above in Example 3 (400 p.s.i. helium).

Each of the five experimental groups (one per each DNA vaccine formulation) consisted of 4 animals, and each animal received primary and booster immunizations with their respective formulations, timed at 0 and 5 weeks. Each immunization consisted of two tandem deliveries to the abdominal epidermis (one cartridge per delivery) using a PowderJect® XR-1 particle delivery device (PowderJect Vaccines Inc., Madison, WI).

Serum antibody responses to the HIV gp120 antigen were tested using an ELISA assay on specimens collected at week 5 and week 6.5 (post-prime and post-boost, respectively). For the ELISA, Costar high binding EIA plates were coated with 0.3 µg/well of recombinant HIV gp120 (Intracel) in 50 µl PBS by incubation overnight at 4°C. Plates were washed three times and blocked with 2% BSA in PBS for 2 hours at room temperature. Serial dilutions of serum were added to the coated plates, and incubated at 37°C for one hour. After washing, the plates were incubated with a 1:1500 dilution of alkaline phosphatase conjugated goat anti-mouse IgG (H+L) (BioRad), followed by color development with p-nitrophenylphosphate (PNPP) (BioRad) and OD reading @ 405nm.

The results of the ELISA are depicted in Figure 7. As can be seen, there was an approximate 20-fold augmentation of gp120-specific immune responses in the groups that received adjuvant vectors (Formulation #4 containing the pPJV2002 and pPJV2003 vectors, or Formulation #5 containing the pPJV2006 and pPJV2003 vectors) in combination with the gp120 vector as compared with the group that received the gp120 vector without adjuvant (Formulation #2, pCIA-EnvT).

Following collection of the post-boost serum samples, animals were sacrificed and spleens were collected from each mouse. Splenocytes were isolated by crushing the spleens, passing the cells through a 70 µm cell strainer, and lysing the red blood cells with ACK lysis buffer (BioWhittaker). Splenocytes were washed 3 times with RPMI-5% FCS and resuspended to a concentration of 1x10⁷ cells/ml in RPMI-10% FCS supplemented with antibiotics, sodium pyruvate, and non-essential amino acids.

The amount of antigen-specific IFN-γ secreted by the splenocytes was determined using an *in situ* ELISA. Costar high binding plates were coated with 10 µg/ml of anti-mouse IFN-gamma capture monoclonal antibody (mAb) (Pharmingen, San Diego, CA) in 50 µl 0.1M bicarbonate buffer (pH 9.6). After overnight incubation at 4°C, the wells were washed 5 times with PBS-0.05% Tween-20 and blocked with 200 µl of complete R10 medium at room temperature for 2 hours. 1 x10⁶ splenocytes were added to each well and were stimulated in medium alone (negative control), or in medium with 1 µg/ml of a HIV gp120 peptide having the following sequence: RIQRGPGRAFVITGK (SEQ ID NO: 21). Following a 24 hour incubation at 37°C in 5% CO₂, the plates were washed 2 times with deionized (DI) water to lyse the cells, then washed 3 times with PBS-0.05% Tween 20, and incubated for 1 hour at room temperature with 50 µl per well of 1 µg/ml biotinylated anti-mouse IFN-γ detecting mAb (Pharmingen). The plates were then washed 5 times and incubated for 1 hour with 50 µl per well of a 1:8000 dilution of strepavidin-horseradish peroxidase (HRP) solution (Southern Biotechnology). The plates were again washed 5 times and colorimetric development was accomplished by the addition of TMB substrate (BioRad, Hercules, CA) for 30 minutes at room temperature. The reaction was stopped by the addition of 1N sulfuric acid. Absorbance at 450nm was read with an optical plate reader.

The results of this study are depicted in Figure 8. This figure shows the relative level of antigen-specific IFN-γ production in the 5 different vaccine test groups receiving the above-described formulations (Formulations #1-5). Importantly, the two immunization groups that received the gp120 vector (pCIA-EnvT) in combination with the CT adjuvant vectors (i.e., Formulation #4 containing the pPJV2002 and pPJV2003 vectors, and Formulation #5 containing the pPJV2006 and pPJV2003 vectors) displayed significantly higher IFN-γ production levels than did the gp120 without adjuvant group (Formulation #2 containing the empty vector control and pCIA-EnvT), (P <0.000001 and P = 0.0068, respectively). These data demonstrate the ability of the present CT vector adjuvant combinations to markedly augment antigen-specific cellular immunity to an HIV antigen in an animal model.

In addition to an increase in the level of IFN-γ production, the number of IFN-γ secreting HIV-gp120 peptide-specific splenocytes was also increased markedly, as determined by an ELISPOT method. In the ELISPOT assay, nitrocellulose plates (Millipore) were coated with an IFN-γ capture antibody, washed, and blocked as described above for the T helper cell IFN-γ *in situ* ELISA. Splenocytes were added to pre-coated wells at an input cell number of 1 x 10⁶ cells/well, and stimulated in medium alone (negative control) or in medium containing 1 µg/ml of a peptide containing the immunodominant HIV-gp120 CTL epitope and having the following sequence: RGPGRAFVTI (SEQ ID NO: 22). The plates were incubated for 24 hours at 37°C in 5% CO₂, washed 2 times with DI water, 3 times with PBS, and incubated for 1 hour at room temperature with 50 µl per well of 1 µg/ml biotinylated anti-mouse IFN-γ detecting mAb (Pharmingen). The plates were then washed 5 times and incubated for 1 hour with 50 µl per well of a 1:1000 dilution of strepavidin-alkaline phosphatase (ALP) solution (Mabtech). The plates were again washed 5 times and colorimetric development was accomplished by the addition of ALP membrane substrate (BioRad, Hercules, CA) until spots formed (2-30 minutes, room temperature). The reaction was stopped by washing with DI water, and the plates were air-dried overnight. Spots were enumerated under a 40X microscope. Only large spots with fuzzy borders were scored as spot forming cells (SFC).

The results from this ELISPOT assay are depicted in Figure 9 which shows the relative levels of IFN-γ-producing splenocytes in the 5 different vaccine test groups (receiving Formulations #1-5, respectively). Importantly, the two immunization groups that received the gp120 vector in combination with the CT adjuvant vectors (i.e., Formulation #4 containing the pPJV2002 and pPJV2003 vectors, and Formulation #5 containing the pPJV2006 and pPJV2003 vectors) displayed significantly higher numbers of IFN-γ-producing cells than did the gp120 without adjuvant group (Formulation #2 containing the empty vector control and pCIA-EnvT), (P <0.000001 and P = 0.0032, respectively). These data demonstrate the ability of the present CT adjuvant combinations to markedly augment antigen-specific cellular immunity against a coadministered HIV gp 120 antigen in an animal model. In addition, the enhanced IFN-γ production seen in these studies indicates that use of the CT adjuvant vector combinations of the present invention provides a robust Th1-like immune response in the immunized animals.

### Example 6

### Enhancement of Antibody Responses to Hepatitis B Core and Surface Antigens Using Simultaneous Delivery of a Vector Encoding HBcAg and HBsAg with CTA/CTB Adjuvant Vectors.

A vector plasmid containing coding sequences for both the Hepatitis B core antigen (HBcAg) and Hepatitis B surface antigen (HBsAg) was constructed as follows. The HBcAg and HBsAg coding sequences were both obtained from the HBV clone pAM6 (ATCC Accession No. 45020). To generate the HBsAg coding region, the pAM6 construct was cut with *Nco*I and treated with mung bean nuclease to remove the start codon of the X-antigen. The resultant DNA was then cut with *Bam*HI and treated with T4 DNA polymerase to blunt-end the DNA and create an HBsAg expression cassette. The HBsAg expression cassette is present in the 1.2 kB fragment. The plasmid construct pPJV7077 (Schmaljohn et al. (1997) J. Virol. 71:9563-9569) which contains the full-length human CMV (Towne strain) immediate early promoter (with enhancer) was cut with *Hind*III and *Bgl*II, and then treated with T4 DNA polymerase and calf-alkaline phosphatase to create blunt-ended DNA, and the HBsAg expression cassette was ligated into the plasmid to yield the pWRG7128 construct.

To generate the HBcAg coding region, the pAM6 construct was cut to create an HBcAg expression cassette, after which the HBcAg sequence was truncated by site directed mutagenesis to remove the C-terminal arginine-rich region from the core antigen particle (which deletion does not interfere with particle formation). The truncated HBcAg sequence was then cloned into a plasmid construct containing the human elongation factor promoter ("hELF", Mizushima et al. (1990) Nucl. Acids Res. 18:5322) to provide a HBcAg vector construct.

Expression cassettes containing: (a) the CMV promoter/enhancer, the Intron A- 5' untranslated region, and the human tissue plasminogen activator (hTPA) signal peptide ("CMV-IA-TPA"); or (b) the bovine growth hormone polyA sequence (bGHpA) were each obtained from the JW4303 vector construct (gift of Dr. Harriet Robinson, University of Massachusetts) and inserted into a plasmid backbone. The resultant construct was cut with *Nhe*I, filled with polymerase and then cut with *Bam*HI to generate a vector fragment containing the pUC 19 origin of replication, the ampicillin resistance gene and the bGHpA sequence. The plasmid backbone was cut a second time with *Sal*I, filled with polymerase, and cut with *Bam*HI to liberate a vector fragment containing the CMV-IA-TPA vector fragment. The two vector fragments were ligated together to yield a construct termed pWRG7054.

The pWRG7054 construct was cut with *Nhe*I, filled with polymerase, and cut with *Bam*HI to produce a vector fragment. The HBcAg vector construct was cut with *Nco*I, filled with polymerase, and cut with *Bam*HI to produce an insert fragment. The two fragments were then ligated together to yield a construct termed pWRG7063.

PEL-Bos was cut with *Eco*RI and dephosphorylated with calf intestinal phosphatase to produce a vector fragment. The pWRG7063 plasmid was cut with *Hind*III, filled with polymerase, and cut with *Eco*RI to produce an insert fragment containing the hTPA signal peptide, the HBcAg antigen sequence and the bGHpA region. These two fragments were ligated together to provide a construct termed pWRG7145.

The pWRG7128 construct was cut with *Eco*RI and dephosphorylated with calf intestinal phosphatase to produce a vector fragment containing the HBsAg coding region under transcriptional control of the hCMV promoter. The pWRG7145 construct was cut with *Mfe*I and *Eco*RI to produce an insert fragment comprised of the hELF promoter/intron, the hTPA signal peptide sequence, the HBcAG antigen sequence and the bGHpA region. These fragments were then ligated together to provide the pPJV7193 plasmid construct containing the HBcAg and HBsAg coding sequences.

The following DNA-gold formulations were then generated for a pig DNA vaccine adjuvant trial.
Formulation #1: Control, the HBcAg/HBsAg vector (pWRG7193) alone, 2 µg DNA per mg gold, 0.5 mg gold per cartridge; and
Formulation #2: The HBcAg/HBsAg vector (pWRG7193), CTA -KDEL (pPJV2006) and CTB (pPJV2003) DNA vectors all coprecipitated onto single batch of gold (1.0 µg pWRG7193 DNA per mg gold, and 0.5 µg each of pPJV2006 and pPJV2003 DNA per mg gold), 0.5 mg gold per cartridge.

The pWRG7193 plasmid either alone or with the pPJV2006 and pPJV2003 adjuvant vectors was precipitated onto gold particles following the procedures described in Example 3 above, again at a final concentration of 2 µg DNA per mg gold. The coated gold particles were loaded into TEFZEL® tubing using the procedures described in Example 3 above. Two experimental groups (of 5 domestic pigs each) received two immunizations with Formulation #1 and Formulation #2, respectively, wherein the two immunizations were spaced 6 weeks apart. Each immunization consisted of two tandem 500 p.s.i. shots to the groin area using a (PowderJect® XR-1 particle delivery device in which each shot utilized a single cartridge. Thus, in the control group (Formulation #1), each immunization consisted of the delivery of a total of 1 mg gold and 2 µg of the DNA vaccine vector pWRG7193. Similarly, in the adjuvant test group (Formulation #2), each immunization consisted of a total delivery of 1 mg of gold, 2 µg of the DNA vaccine vector pWRG7193, and 1 µg each of the adjuvant vectors pPJV2006 and pPJV2003. Blood samples were collected from each animal at the time of the booster immunization (week 6) and 2 weeks following the booster immunization (week 8).

Detection of antibody responses specific for the core antigen was performed as follows: ELISA plates were coated with the hepatitis B core antigen (Biodesign) at 100 ng/ml in PBS. After coating overnight at 4°C, plates were blocked with 5% nonfat dry milk in PBS for 1 hour at room temperature. Plates were then washed 3 times with PBS containing 0.05% Tween-20. Swine serum samples were serially diluted in 2% nonfat dry milk/PBS/0.01% Tween-20 and added to the ELISA plates. After incubation at room temperature for 2 hours, plates were washed 3 times with PBS/0.05% Tween-20. The secondary antibody consisted of a goat anti-swine IgG conjugated to horse radish peroxidase (Kirkegaard and Perry) that was diluted 1:2000 in 2% nonfat dry milk/PBS/0.01% Tween-20, and added to the plates for a 1 hour incubation at room temperature. Plates were then washed 5 times with PBS/0.05% Tween-20 and 100 µl of TMB substrate as added. Color development proceeded for 15 minutes and was stopped by the addition of 100 µl of 1N H₂SO₄. Plates were read at 450 nm. Figures 10 and 11 show the geometric mean absorbance values at 6 and 8 weeks, respectively, for each of the two groups of pigs at 4 different serum dilutions. These data demonstrate marked enhancement of antibody titers to the hepatitis B core antigen following use of CT adjuvant vectors pPJV2006 and pPJV2003.

In addition to elevated humoral responses to the hepatitis B core antigen, a measurable elevation of antibody responses specific for the hepatitis B surface antigen was observed in the adjuvant test group as well. Surface antigen-specific antibodies were quantified in serum samples from individual animals using a commercial assay kit (AUSAB, Abbott Laboratories). This kit allows for the quantification of antibody responses in terms of milli-international unit (mIU/ml) using a standard panel. The geometric mean surface antigen antibody titers in the control group (Formulation #1) and adjuvant test group (Formulation #2) were 285 and 662 mIU/ml, respectively, demonstrating the ability of the present adjuvant plasmids (pPJV2006 and pPJV2003) to augment immune responses to an antigen encoded by a separate vector.

### Example 7

### Enhancement of Cellular Th1-Like Immune Response to a DNA Vaccine Using Plasmid Vectors Encoding CT or LT Subunits.

The pM2-FL DNA vaccine vector encoding the M2 protein of influenza A virus was employed to test the adjuvant effects of the pPJV2002, pPJV2003, pPJV2004, pPJV2005, pPJV2006 and pPJV2007 adjuvant vectors in the context of particle-mediated DNA vaccination. Particle-mediated DNA vaccination was performed by precipitating the M2 DNA vaccine vector, either with our without various combinations of the adjuvant vectors, onto microscopic gold particles and accelerating the coated gold particles into the epidermis of mice using a PowderJect® XR-1 particle delivery device (PowderJect Vaccines, Inc. Madison, WI). Construction of the pM2-FL DNA plasmid vector is described herein above in Example 3.

As above, the pM2-FL plasmid was precipitated onto 2 micron gold particles as single vector, or mixed vector plus adjuvant vector samples. Specifically, plasmid DNA (single pM2-FL vector or pM2-FL vector plus one or more adjuvant vectors) was mixed with 2 micron gold particles (Degussa) in a small centrifuge tube containing spermidine. Precipitation was carried out following the methodologies of Example 3, and the coated gold particles were then coated onto the inside surface of a TEFZEL® tube as also described in Example 3. The tubing was then cut into 0.5 inch cartridges suitable for loading into the particle delivery device.

The following DNA-gold formulations were generated for a mouse DNA vaccine adjuvant trial.
Formulation #1: pM2-FL DNA vector combined with the pWRG7054 empty plasmid vector prior to precipitation onto the same batch of gold, 2.1 µg total DNA per mg gold (0.1 µg pM2-FL and 2.0 µg pWRG7054), 0.5 mg gold per cartridge;
Formulation #2: pM2-FL DNA vector combined with the CTA and CTB (pPJV2002 and pPJV2003) DNA vectors prior to precipitation onto a single batch of gold, 2.1 µg total DNA per mg gold (1.0 µg of each DNA adjuvant vector per mg gold, 0.1 µg pM2-FL), 0.5 mg gold per cartridge;
Formulation #3: pM2-FL DNA vector combined with the CTA -KDEL and CTB (pPJV2006 and pPJV2003) DNA vectors all coprecipitated onto single batch of gold, 2.1 µg total DNA per mg gold (1.0 µg of each DNA adjuvant vector per mg gold, 0.1 µg pM2-FL), 0.5 mg gold per cartridge;
Formulation #4: pM2-FL DNA vector combined with the CTA -KDEL (pPJV2006) DNA vector and supplemented with the pWRG7054 empty plasmid vector all combined and coprecipitated onto single batch of gold, 2.1 µg total DNA per mg gold (1.0 µg pPJV2006, 1.0 µg pWRG7054, 0.1 µg pM2-FL), 0.5 mg gold per cartridge;
Formulation #5: pM2-FL DNA vector combined with the CTA (pPJV2002) DNA vector and supplemented with the pWRG7054 empty plasmid vector, all DNAs combined and coprecipitated onto single batch of gold, 2.1 µg total DNA per mg gold (1.0 µg pPJV2002, 1.0 µg pWRG7054, 0.1 µg pM2-FL), 0.5 mg gold per cartridge;
Formulation #6: pM2-FL DNA vector combined with the CTB (pPJV2003) DNA vector and supplemented with the pWRG7054 empty plasmid vector, all DNAs combined and coprecipitated onto single batch of gold, 2.1 µg total DNA per mg gold (1.0 µg pPJV2003, 1.0 µg pWRG7054, 0.1 µg pM2-FL), 0.5 mg gold per cartridge;
Formulation #7: pM2-FL DNA vector combined with the LTA and LTB (pPJV2004 and pPJV2005) DNA vectors prior to precipitation onto a single batch of gold, 2.1 µg total DNA per mg gold (1.0 µg of each DNA adjuvant vector per mg gold, 0.1 µg pM2-FL), 0.5 mg gold per cartridge;
Formulation #8: pM2-FL DNA vector combined with the LTA -RDEL and LTB (pPJV2007 and pPJV2005) DNA vectors all coprecipitated onto single batch of gold, 2.1 µg total DNA per mg gold (1.0 µg of each DNA adjuvant vector per mg gold, 0.1 µg pM2-FL), 0.5 mg gold per cartridge;
Formulation #9: pM2-FL DNA vector combined with the LTA -RDEL (pPJV2007) DNA vector and supplemented with the pWRG7054 empty plasmid vector all combined and coprecipitated onto single batch of gold, 2.1 µg total DNA per mg gold (1.0 µg pPJV2007, 1.0 µg pWRG7054, 0.1 µg pM2-FL), 0.5 mg gold per cartridge;
Formulation #10: pM2-FL DNA vector combined with the LTA (pPJV2004) DNA vector and supplemented with the pWRG7054 empty plasmid vector, all DNAs combined and coprecipitated onto single batch of gold, 2.1 µg total DNA per mg gold (1.0 µg pPJV2004, 1.0 µg pWRG7054, 0.1 µg pM2-FL), 0.5 mg gold per cartridge; and
Formulation #11: pM2-FL DNA vector combined with the LTB (pPJV2005) DNA vector and supplemented with the pWRG7054 empty plasmid vector, all DNAs combined and coprecipitated onto single batch of gold, 2.1 µg total DNA per mg gold (1.0 µg pPJV2005, 1.0 µg pWRG7054, 0.1 µg pM2-FL), 0.5 mg gold per cartridge.

These DNA vaccine formulations were then administered to eleven groups of mice as follows. Each experimental group contained 8 animals and each animal received two immunizations with their respective formulation with a 4 week resting period between immunizations. Each immunization consisted of two tandem deliveries to the abdominal epidermis (one cartridge per delivery) using a PowderJect® XR-1 particle delivery device (PowderJect Vaccines Inc., Madison, WI) at a helium pressure of 400 p.s.i.. Serum samples were collected two weeks following the second or booster immunization.

Individual serum samples were assayed for M2-specific antibody, responses for both IgG1 and IgG2a subclasses using the ELISA assay of Example 3 above for the determination of total IgG titer, except that a secondary antibody conjugate specific for either IgG1 or IgG2a was employed. The goat anti-mouse IgG1-biotin conjugated antibody was obtained from Southern Biotechnology Associates, Inc. (catalogue #1070-08, 0.5 mg/ml concentration) and used at a 1/8000 dilution. The goat anti-mouse IgG2a-biotin conjugated antibody was obtained from the same source (catalogue #1080-08, 0.5 mg/ml concentration) and was also used at a 1/8000 dilution.

Geometric mean antibody titers for M2 antigen-specific IgG1 and IgG2a were determined for each experimental group, and the IgG1-to-IgG2a ratios were calculated. These data are reported below in Table 5.

**TABLE 5**

| **Formulation #** | **IgG1-to-IgG2a Ratio** |
|---|---|
| 1 | 160.99 |
| 2 | 3.44 |
| 3 | 13.59 |
| 4 | 63.20 |
| 5 | 2.63 |
| 6 | 30.97 |
| 7 | 0.02 |
| 8 | 0.50 |
| 9 | 9.00 |
| 10 | 4.53 |
| 11 | 27.00 |

As can be seen with reference to Table 5, addition of either of the A or B subunits, or the various combinations of A and B subunits to the M2 DNA vaccine formulation resulted in significant reductions in the IgG1-to-IgG2a ratio otherwise elicited with the M2 DNA vaccine (pM2-FL) in the absence of adjuvant (Formulation #1). The greatest ratio reduction resulted from use of the LTA plus LTB, and LTA -RDEL plus LTB vector combinations (Formulations #7 and #8, respectively). In both of these formulations, use of the polynucleotide adjuvants of the present invention resulted in an abundance of M2 antigen-specific IgG2a titers over IgG1, which characteristic is a hallmark of a Th1-like immune response in mice.

The results from experimental groups receiving Fonnulations #1, #2 and #7 were plotted as the log IgG1-to-IgG2a ratio in Figure 12. As can be seen in that figure, adjuvanting the pM2-FL DNA vaccine composition with the CTA plus CTB adjuvant vectors (Formulation #2) caused a 2 order of magnitude drop in the IgG1-to-IgG2a ratio relative to the pM2-FL vaccine composition without adjuvant (Formulation #1). In addition, a further 2 order of magnitude drop in this ratio was observed when the pM2-FL DNA vaccine was adjuvanted with the LTA plus LTB adjuvant vectors (Formulation #7).

### Example 8

### Addition of Signal Peptide Coding Sequences to Adjuvant Vectors Encoding CT or LT.

The vector constructs containing the CTA, CTB, LTA and LTB toxin subunits (pPJV2002, pPJV2003, pPJV2004, and pPJV2005, respectively) were modified to remove the tpa signal peptide coding sequences, and a dual DNA vaccine vector encoding both the Hepatitis B surface and core antigens was constructed for use in the following experiments.

The standard PCR conditions that were used for the construction/modification of the vectors were as follows: 1x PCR core buffer with 1.5 mM MgCl₂ (Promega Corp., Madison, WI); 0.400 µM of each primer; 200 µM of each dNTP (USB Inc., Cleveland, OH); 2.5 µg Taq polymerase (Promega Corp., Madison, WI); 1.0 ng template DNA; water to 100 µl; and a mineral oil (Aldrich Chemical Inc., Milwaukee WI) overlay. A PTC-200 thermocycler (MJ Research Inc., Waltham, MA) was programmed to run the following routine: 4 minutes @ 95°C; 30 cycles of (1 minute @ 95°C/ 1 minute 15 seconds @ 55°C/ 1 minute @ 72°C); 10 minutes @ 72°C; 4°C hold. The amplification products were removed from the PCR reaction using the QIAquick® PCR Purification Kit (Qiagen Inc., Valencia CA) prior to cutting with restriction enzymes (New England Biolabs, Beverly, MA). All PCR products were sequenced after cloning to ensure fidelity of the amplification.

More specifically, a dual DNA vaccine vector encoding both the Hepatitis B surface and core antigens was constructed as follows. The pWRG7128 construct (Example 4) which contains the surface antigen coding sequence was modified using a series of standard molecular biology techniques to provide the dual (surface/core antigen) construct. Initially, the pWRG7128 construct was modified to remove the bovine growth hormone polyadenylation region and replace the same with the rabbit beta-globin polyadenylation region. A first insertion fragment containing the CMV promoter with exon 1 and exon 2 sequences, and a second insertion fragment containing a second rabbit beta-globin polyadenlyation region were ligated into the modified pWRG7128 construct, and an adaptor constructed by annealing systentic oligonucleotides was inserted between *Sph*1 and *Pst*1 sites located immediately upstream of the inserted CMV promoter.

Plasmid mpSmpCC (GlaxoSmithKline, UK) was PCRed with the following primers: 5'—GCC GCT AGC ATG GAC ATT GAC CCT TAT AAA GA—3' (SEQ ID NO:23) and 5'—CCA GGA TCC TTA ACA TTG AGA TTC C—3' (SEQ ID NO:24) to generate a Hepatitis B-adw2 core antigen coding sequence. This PCR product was cut with *Nhe*1 and *Bam*H1 to generate an insertion fragment, which fragment was then modified to include a downstream *Bgl*2 site and inserted into a cloning vector. The cloning vector was cut with *Pst*1 and *Eco*R1 to generate a core antigen insertion fragment; the modified pWRG7128 plasmid was cut with *Pst*1 and *Mfe*1 to generate a vector fragment, and the core antigen insertion fragment was ligated into the vector fragment, resulting in the dual surface/core antigen plasmid construct.

The vector constructs containing the CTA, CTB, LTA and LTB toxin subunits (pPJV2002, pPJV2003, pPJV2004, and pPJV2005, respectively) were modified to remove the tpa signal peptide coding sequences by merely excising the tpa coding sequences using restriction enzymes to produce the following constructs: CTA w/o TPA, CTB w/o TPA, LTA w/o TPA, and LTB w/o TPA, respectively.

The dual surface/core antigen plasmid was combined with irrelevant plasmid DNA (for non-adjuvanted control) or with the toxin subunit vector constructs and precipitated onto 2 micron gold particles. Specifically, plasmid DNA (dual surface/core antigen plasmid vector) plus two adjuvant vectors (to provide a CTA/CTB or LTA/LTB combination) was mixed with 2 micron gold particles (Degussa) in a small centrifuge tube containing spermidine. Precipitation was carried out following the methodologies of Example 3, and the coated gold particles were then coated onto the inside surface of a TEFZEL® tube as also described in Example 3. The tubing was then cut into 0.5 inch cartridges suitable for loading into the particle delivery device.

The following DNA-gold formulations were thus generated for a mouse DNA vaccine adjuvant trial:
Formulation #1: ("no adjuvant") 1 µg dual surface/core antigen DNA plasmid vector, 1 µg irrelevant DNA plasmid vector;
Formulation #2: ("CT") 1 µg dual surface/core antigen DNA plasmid vector, 0.5 µg pPJV2002 (CTA) DNA plasmid vector, 0.5 µg pPJV2003 (CTB) DNA plasmid vector;
Formulation #3: ("CT w/o TPA") 1 µg dual surface/core antigen DNA plasmid vector, 0.5 µg CTA w/o TPA DNA plasmid vector, 0.5 µg CTB w/o TPA DNA plasmid vector;
Formulation #4: ("CTA") 1 µg dual surface/core antigen DNA plasmid vector, 1 µg pPJV2002 (CTA) DNA plasmid vector;
Formulation #5: ("CTA w/o TPA") 1 µg dual surface/core antigen DNA plasmid vector, 1 µg CTA w/o TPA DNA plasmid vector;
Formulation #6: ("CTB") 1 µg dual surface/core antigen DNA plasmid vector, 1 µg pPJV2003 (CTB) DNA plasmid vector;
Formulation #7: ("CTB w/o TPA") 1 µg dual surface/core antigen DNA plasmid vector, 1 µg CTB w/o TPA DNA plasmid vector;
Formulation #8: ("LT") 1 µg dual surface/core antigen DNA plasmid vector, 0.5 µg pPJV2004 (LTA) DNA plasmid vector, 0.5 µg pPJV2005 (LTB) DNA plasmid vector;
Formulation #9: ("LT w/o TPA") 1 µg dual surface/core antigen DNA plasmid vector, 0.5 µg LTA w/o TPA DNA plasmid vector, 0.5 µg LTB w/o TPA DNA plasmid vector;
Formulation #10: ("LTA") 1 µg dual surface/core antigen DNA plasmid vector, 1 µg pPJV2004 (LTA) DNA plasmid vector;
Formulation #11: ("LTA w/o TPA") 1 µg dual surface/core antigen DNA plasmid vector, 1 µg LTA w/o TPA DNA plasmid vector;
Formulation #12: ("LTB") 1 µg dual surface/core antigen DNA plasmid vector, 1 µg pPJV2005 (LTB) DNA plasmid vector; and
Formulation #13: ("LTB w/o TPA") 1 µg dual surface/core antigen DNA plasmid vector, 1 µg LTB w/o TPA DNA plasmid vector.

In a first study, various of the above-described DNA vaccine formulations were administered to five groups of mice using the PowderJect® XR-1 particle delivery device (PowderJect Vaccines Inc., Madison, WI). Each experimental group contained 5 animals, and each animal received two immunizations with the respective formulation with a four week resting period between immunizations. The Formulations that were tested were as follows: Formulation #1 (no adjuvant); Formulation #2 (CT); Formulation #3 (CT w/o TPA); Formulation #8 (LT); and Formulation #9 (LT w/o TPA). All animals were sacrificed two weeks after the second immunization, and the spleens harvested for use in IFN-γ and IL-4 ELISPOT assays. For the cellular immune assays, single cell suspensions of splenocytes from the spleens of the immunized animals were cultured *in vitro* in the presence of a peptide corresponding to a known T cell epitope (from either the surface or the core antigen) in the mice. The peptide was dissolved in DMSO (10 mg/ml) and diluted to 10 ug/ml in culture.

For the ELISPOT assays, Millipore Multiscreen membrane filtration plates were coated with 50 µl of the appropriate antiserum (15 µg/ml anti-IFN-yor -IL-4 antiserum, Pharmingen) in sterile 0.1 M carbonate buffer (pH 9.6) overnight at 4°C. Plates were washed 6 times with sterile PBS and then blocked with tissue culture medium containing 10% fetal bovine serum (FBS) for 1-2 hr at RT. The medium was removed and spleen cells dispensed into the wells with a total of 1x10⁶ cells per well. For wells in which less than 1x10⁶ cells from immunized animals was added, cells from naive animals were used to bring the total to 1x10⁶. Cells were incubated overnight in a tissue culture incubator in the presence of the peptide as described above. The plates were then washed 2 time with PBS and 1 time with distilled water. This was followed by 3 washes with PBS. Biotinylated anti-IFN-γ or anti-IL-4 monoclonal antibody (Pharmingen) was added to the plate (50 µl of a 1 µg/ml solution in PBS) and incubated for 2 hr at RT. The plates were washed 6 times with PBS after which 50 µl of a Streptavidin Alkaline phosphatase conjugate (1:1000 in PBS, Pharmingen) was added and incubated for 2 hr at RT. The plates were washed 6 times with PBS and an alkaline phosphatase color substrate (BioRad) was added and the reaction was allowed to proceed until dark spots appeared. The reaction was stopped by washing with water 3 times. Plates were air dried and spots counted under a microscope.

The adjuvant effect of the secreted or non-secreted toxin subunit coding sequences was assessed by determining IFN-γ and IL-4 ELISPOT responses to both the Hepatitis B surface and core antigens ("sAg" and "cAg") encoded by the dual surface/core antigen construct, and these results were compared as reported in Figures 13A-13D. As can be seen, the secreted (signal sequence containing) CT and LT adjuvant vectors (Formulations #2 and #8, respectively) produced significant increases (P ≤ 0.05) in IFN-γ responses to both the surface and core antigens, and in the IL-4 response to surface antigen (see Figures 13A, 13B and 13C). With respect to the IL-4 responses to the core antigen, the lack of adjuvant effect by the LT vectors (Formulations #8 and #9) is consistent with observations that the LT toxin is more of a Th1 adjuvant than the CT toxin. Most importantly, the CT and LT vectors lacking the signal sequences (Formulations #3 and #9, respectively) exhibited weaker adjuvant effect, particularly as seen in the IFN-γ ELISPOT data for both the surface and core antigens (see Figures 13A and 13C), where there was a statistically significant drop in adjuvant activity by virtue of deletion of the signal sequences.

Finally, the clearly observable difference in adjuvant effect between the secreted (Formulations #2 and #8) and the non-secreted (Formulations #3 and #9) helps establish that the observed adjuvant effects are not due to CpG motifs within the adjuvant vectors since the signal-containing and non signal-containing vectors do not have any difference in bacterial DNA (CpG) content yet exhibit significant differences in their ability to augment surface antigen-specific IFN-γ responses.

In a second study, the above-described DNA vaccine formulations were administered to eight groups of mice using the PowderJect® XR-1 particle delivery device (PowderJect Vaccines Inc., Madison, WI). Each experimental group contained 5 animals, and each animal received two immunizations with the respective formulation with a four week resting period between immunizations. The Formulations that were tested were as follows: Formulation #1 (no adjuvant); Formulation #2 (CT); Formulation #4 (CTA); Formulation #5 (CTA w/o TPA); Formulation #6 (CTB); Formulation #7 (CTB w/o TPA); Formulation #8 (LT); Formulation #10 (LTA); Formulation #11 (LTA w/o TPA); Formulation #12 (LTB); and Formulation #13 (LTB w/o TPA). All animals were sacrificed two weeks after the second immunization, and the spleens harvested for use in IFN-γ and IL-4 ELISPOT assays described herein above.

As a result of this second study (data not shown), it was again observed that there was no discernable adjuvant effect that could be attributed to CpG content in the various adjuvant plasmids. Although for the most part no statistically relevant adjuvant effect was observed with the various toxin subunit adjuvant vectors, the LT subunit vectors (Formulations #10-13) did show adjuvant effect in the IFN-γ and IL-4 response to surface antigen (sAg) that was influenced by the presence/absence of the secretion signal sequence.

### Example 9

### Adjuvant Plasmid Vectors Encoding CTA/CTB or LTA/LTB Subunit Peptides in a Viral Challenge Study

In order to assess the ability of the adjuvant plasmid vectors of the present invention to provide for protective effect in a Herpes Simplex Virus type 2 (HSV-2) viral challenge model, the following study was carried out. A DNA vaccine encoding an HSV-2 antigen was constructed and then combined with various combinations of the present adjuvant plasmid vectors to provide vaccine compositions. After immunization, the immunized animals were challenged with HSV-2 virus, and the protective effect of the various vaccine compositions was determined.

With respect to the construction of the DNA antigen plasmid, standard PCR techniques were used to construct the plasmid. The standard PCR conditions that were used for the construction of the vector were as follows: 1x PCR core buffer with 1.5 mM MgCl₂ (Promega Corp., Madison, WI); 0.400 µM of each primer; 200 µM of each dNTP (USB Inc., Cleveland, OH); 2.5 µg Taq polymerase (Promega Corp., Madison, WI); 1.0 ng template DNA; water to 100 µl; and a mineral oil (Aldrich Chemical Inc., Milwaukee WI) overlay. A PTC-200 thermocycler (MJ Research Inc., Waltham, MA) was programmed to run the following routine: 4 minutes @ 95°C; 30 cycles of (1 minute @ 95°C/ 1 minute 15 seconds @ 55°C/ 1 minute @ 72°C); 10 minutes @ 72°C; 4°C hold. The amplification products were removed from the PCR reaction using the QIAquick® PCR Purification Kit (Qiagen Inc., Valencia CA) prior to cutting with restriction enzymes (New England Biolabs, Beverly, MA). All PCR products were sequenced after cloning to ensure fidelity of the amplification.

More specifically, a DNA vaccine plasmid vector encoding the HSV-2 early ICP27 antigen was constructed as follows. HSV is a double-stranded DNA virus having a genome of about 150-160 kbp. The viral genome is packaged within an icosahedral nucleocapsid which is enveloped in a membrane. The membrane (or envelope) includes at least 10 virus-encoded glycoproteins, the most abundant of which are gB, gC, gD, and gE. The viral genome also encodes over 70 other proteins, including a group of approximately five ICP antigens. These early proteins are synthesized early in the viral replication cycle, in contrast to the envelope glycoproteins which are only made late in the life cycle of the virus. For a review of the molecular structure and organization of HSV, see, for example, Roizman and Sears (1996) "Herpes simplex viruses and their replication" in Fields Virology, 3rd ed., Fields et al. eds., Lippincott-Raven Publishers, Philadelphia, PA. The HSV-2 ICP27 antigen can be readily obtained from the HSV-2 genome, for example the genomic region spanning from approximately nucleotide 114589 to 134980 of the HSV-2 genome, or an *Eco*RI fragment that spans nucleotides 110931 to 139697 of the HSV-2 genome. The sequence of the HSV-2 genome is available form published sources, for example the sequence deposited with GenBank under Accession Number NC_001798.

In order to construct the ICP27 vector used in the present study, the ICP27 coding region was PCRed from the HSV-2 genome using the following primers: 5'—GCC ACT CTC TTC CGA CAC—3' (SEQ ID NO:25) and 5'—CAA GAA CAT CAC ACG GAA C—3' (SEQ ID NO:26) to obtain a nucleotide fragment containing nucleotide sequences 114523-116179 (GenBank) of HSV-2 which correspond to the ICP27 coding region. The ICP27 fragment was then cloned into the multiple cloning region of the pTarget vector (Promega Corp., Madison, WT).

The adjuvant plasmid vector constructs containing the CTA, CTB, LTA and LTB toxin subunits (pPJV2002, pPJV2003, pPJV2004, and pPJV2005, respectively) were combined to provide CTA/CTB (pPJV2002 + pPJV2003) and LTA/LTB (pPJV2004 + pPJV2005) adjuvant. The ICP27 antigen plasmid was combined with with the toxin subunit vector construct pairs and precipitated onto 2 micron gold particles. Specifically, plasmid DNA (ICP27 antigen plasmid vector) plus two adjuvant vectors (to provide a CTA/CTB or LTA/LTB combination) was mixed with 2 micron gold particles (Degussa) in a small centrifuge tube containing spermidine. Precipitation was carried out following the methodologies of Example 3, and the coated gold particles were then coated onto the inside surface of a TEFZEL® tube as also described in Example 3. The tubing was then cut into 0.5 inch cartridges suitable for loading into the particle delivery device.

The following DNA-gold formulations were thus generated for the HSV-2 challenge study:
Formulation #1: (no adjuvant) 2 µg ICP27 antigen DNA plasmid vector; Formulation #2: ("High CT") 900 ng ICP27 antigen DNA plasmid vector, 50 ng of pPJV2002 (CTA), 50 ng of pPJV2003 (CTB);
Formulation #3: ("Low CT") 500 ng ICP27 antigen DNA plasmid vector, 250 ng of pPJV2002 (CTA), 250 ng of pPJV2003 (CTB);
Formulation #4: ("High LT") 900 ng ICP27 antigen DNA plasmid vector, 50 ng of pPJV2004 (LTA), 50 ng of pPJV2005 (LTB); and
Formulation #5: ("Low LT") 500 ng ICP27 antigen DNA plasmid vector, 250 ng of pPJV2004 (LTA), 250 ng of pPJV2005 (LTB).

In the study, the above-described DNA vaccine formulations were administered to five different groups of mice using the PowderJect® XR-1 particle delivery device (PowderJect Vaccines Inc., Madison, WI). Each experimental group contained 12 animals, and each animal received two immunizations (single shot applied to the abdomen) with the respective formulation with a four week resting period between immunizations. A sixth group of mice was established as a negative (naive) control, and did not receive any vaccinations. 4 mice from each group were sacked 2 weeks after the second immunization and used for IFN-γ ELISPOT assays (data not shown).

Two weeks post second immunization, all remaining mice (8/group) were challenged with 1x10⁶ PFU of HSV-2 virus, strain MS, via intra-nasal instillation. The survival graph depicting the results of the challenge study is depicted in Figure 14. As can be seen, 100% of the naive animals succumbed within 4 days post challenge. The naive animals are depicted on the graph by the (●) curve. In addition, 100% of the animals receiving the ICP27 antigen plasmid vector alone (Formulation #1) died within 7 days post challenge. The animals receiving Formulation #1 are depicted on the graph by the (▼) curve. In marked contrast, the 25% (2/8) of the animals receiving the ICP27 plasmid adjuvanted with the low dose CT (Formulation #3) were protected from the viral challenge, and 38% (3/8) of the animals receiving the ICP27 adjuvanted with the high dose CT (Formulation #2) were protected from the viral challenge. The animals receiving Formulation #3 are depicted on the graph by the (■) curve. The animals receiving Formulation #2 are depicted on the graph by the (◆) curve. Finally, both the low dose LT-adjuvanted (Formulation #5) and the high dose LT-adjuvanted (Formulation #4) ICP27 vaccine provided complete (100%) protection in the immunized animals. The animals receiving Formulation #5 are depicted on the graph by the (▲) curve. The animals receiving Formulation #4 are depicted on the graph by the (O) curve.

Accordingly, novel polynucleotide adjuvant molecules, compositions comprising those adjuvant molecules, and conventional and nucleic acid immunization techniques have been described.

## Claims

1. An adjuvant composition comprising: (i) a first nucleic acid sequence; (ii) a second nucleic acid sequence; and (iii) core carriers, wherein: (a) said first nucleic acid sequence is a truncated A subunit coding region obtained or derived from a bacterial ADP-ribosylating exotoxin; (b) said second nucleic acid sequence is a truncated B subunit coding region obtained or derived from a bacterial ADP-ribosylating exotoxin; (c) said first and second nucleic acid sequences are coated onto the core carriers and are operably linked to, or are each operably linked to, a promoter active in a mammalian cell; and (d) each of said truncated subunit coding regions has a 5' deletion, encodes a subunit peptide not having an amino terminal bacterial signal peptide and is operably linked to a leader sequence for secretion from a mammalian cell.

2. A composition according to claim 1, wherein the truncated A subunit coding region has been further genetically modified so as to delete a C-terminal KDEL or RDEL motif in the subunit peptide encoded thereby.

3. An adjuvant composition comprising: (i) a first nucleic acid sequence; (ii) a second nucleic acid sequence; and (iii) core carriers, wherein: (a) said first nucleic acid sequence is a modified A subunit coding region obtained or derived from a bacterial ADP-ribosylating exotoxin; (b) said second nucleic acid sequence is a B subunit coding region obtained or derived from a bacterial ADP-ribosylating exotoxin; (c) said first and second nucleic acid sequences are coated onto the core carriers and are operably linked to, or are each operably linked to, a promoter active in a mammalian cell; and (d) each of said modified A subunit coding region and said B subunit coding region each encode a mature subunit peptide, and with the further proviso that the modified A subunit coding region has been genetically modified so as to delete a C-terminal KDEL or RDEL motif in the subunit peptide encoded thereby and is operably linked to a leader sequence for secretion from a mammalian cell.

4. A composition according to claim 3, wherein the modified A subunit coding region and the B subunit coding region have each been truncated by a 5' deletion whereby each of said truncated subunit coding regions encodes a subunit peptide not having an amino terminal bacterial signal peptide.

5. A composition according to any one of the preceding claims, wherein the bacterial ADP-ribosylating exotoxin is an *E*. *coli* heat labile enterotoxin (LT).

6. A composition according to any one of the preceding claims further comprising an antigen of interest.

7. A composition according to any one of claims 1 to 5, further comprising a third nucleic acid sequence that encodes an antigen of interest.

8. A composition according to claim 6 or 7, wherein the antigen is from a bacterial pathogen, a viral pathogen, a parasitic pathogen, an allergen or a tumour specific antigen.

9. A composition according to claim 8 wherein the antigen is from an influenza virus, Herpes Simplex Virus (HSV), Hepatitis virus, or a papilloma virus.

10. A composition according to any one of the preceding claims, further comprising a pharmaceutically acceptable vehicle or excipient.

11. A composition according to any one of the preceding claims wherein the particulate composition is suitable for transdermal delivery via a particle delivery device.

12. A composition according to any one of the preceding claims, wherein the core carrier particle has an average diameter of 0.1 to 10 µm.

13. A composition according to any one of the preceding claims, wherein the core carrier particle comprises a metal.

14. A composition according to claim 13 wherein the metal is gold.

15. An adjuvant composition according to any one of claims 1 to 5 for use in enhancing an immune response in a vertebrate subject against an antigen of interest by administering the antigen of interest and the composition to the subject.

16. An adjuvant composition according to claim 15, wherein the antigen of interest and the composition are administered to the same site in the subject.

17. An adjuvant composition according to claim 15 or 16, wherein the antigen of interest and the composition are administered concurrently.

18. An adjuvant composition according to claim 15, wherein the composition further comprises the antigen of interest.

19. An adjuvant composition according to claim 15, wherein the composition comprises a third nucleic acid sequence which encodes the antigen of interest.

20. An adjuvant composition according to any one of claims 15 to 19, wherein the antigen of interest is from a bacterial pathogen, a viral pathogen, a parasitic pathogen, an allergen or a tumor specific antigen.

21. An adjuvant composition according to claim 20, wherein the antigen is from an influenza virus , Herpes Simplex Virus (HSV), Hepatitis virus, or a papilloma virus.

22. An adjuvant composition according to any one of claims 15 to 21, wherein the a core carrier particles are administered to the subject using a particle-mediated delivery technique.

23. An adjuvant composition according to any one of claims 15 to 22, wherein the subject is human.

24. Products containing a composition according to any one of claims 1 to 5 and an antigen of interest as a combined preparation for simultaneous, separate or sequential use for enhancing an immune response in a vertebrate subject against said antigen.

25. A particle delivery device for transdermal delivery which is loaded with a particulate composition as defined in any one of claims 1 to 13.

26. A device according to claim 25 which is a needleless syringe.

27. A hermetically sealed single unit dosage or multidose container adapted for use in a particle delivery device, said container comprising an adjuvant composition according to any one of claims 1 to 13.

28. Use of a first and second nucleic acid sequences as defined in any one of claims 1 to 5 for the manufacture of a medicament for enhancing an immune response in a vertebrate subject against an antigen of interest by administering the antigen of interest and the medicament to the subject.

## Patentansprüche

1. Adjuvanszusammensetzung, umfassend: (i) eine erste Nukleinsäuresequenz; (ii) eine zweite Nukleinsäuresequenz und (iii) Core-Träger, wobei: (a) die erste Nukleinsäuresequenz eine trunkierte A-Untereinheit-codierende Region, erhalten aus oder abgeleitet von einem bakteriellen ADP-ribosylierenden Exotoxin, ist; (b) die zweite Nukleinsäuresequenz eine trunkierte B-Untereinheit-codierende Region, erhalten aus oder abgeleitet von einem bakteriellen ADP-ribosylierenden Exotoxin, ist; (c) die erste und zweite Nukleinsäuresequenz auf die Core-Träger aufgetragen sind und funktionell verknüpft sind oder jeweils funktionell verknüpft sind mit einem Promotor, der in einer Säugerzelle aktiv ist; und (d) jede der trunkierte Untereinheit-codierenden Regionen eine 5'-Deletion aufweist, für ein Untereinheit-Peptid codiert, das kein aminoterminales bakterielles Signalpeptid aufweist, und funktionell verknüpft ist mit einer Leader-Sequenz zur Sekretion aus einer Säugerzelle.

2. Zusammensetzung gemäß Anspruch 1, wobei die trunkierte A-Untereinheit-codierende Region des Weiteren genetisch modifiziert worden ist, um ein C-terminales KDEL- oder RDEL-Motiv in dem **dadurch** codierten Untereinheit-Peptid zu deletieren.

3. Adjuvanszusammensetzung, umfassend: (i) eine erste Nukleinsäuresequenz; (ii) eine zweite Nukleinsäuresequenz und (iii) Core-Träger, wobei: (a) die erste Nukleinsäuresequenz eine modifizierte A-Untereinheit-codierende Region, erhalten aus oder abgeleitet von einem bakteriellen ADP-ribosylierenden Exotoxin, ist; (b) die zweite Nukleinsäuresequenz eine B-Untereinheit-codierende Region, erhalten aus oder abgeleitet von einem bakteriellen ADP-ribosylierenden Exotoxin, ist; (c) die erste und zweite Nukleinsäuresequenz auf die Core-Träger aufgetragen sind und funktionell verknüpft sind oder jeweils funktionell verknüpft sind mit einem Promotor, der in einer Säugerzelle aktiv ist; und (d) jede der modifizierte A-Untereinheit-codierenden Region und der B-Untereinheit-codierenden Region jeweils für ein reifes Untereinheit-Peptid codiert, und mit der weiteren Maßgabe, dass die modifizierte A-Untereinheit-codierende Region genetisch modifiziert worden ist, um ein C-terminales KDEL- oder RDEL-Motiv in dem **dadurch** codierten Untereinheit-Peptid zu deletieren, und funktionell verknüpft ist mit einer Leader-Sequenz zur Sekretion aus einer Säugerzelle.

4. Zusammensetzung gemäß Anspruch 3, wobei die modifizierte A-Untereinheit-codierende Region und die B-Untereinheit-codierende Region jeweils mittels einer 5'-Deletion trunkiert worden sind, wodurch jede der trunkierte Untereinheit-codierenden Regionen für ein Untereinheit-Peptid codiert, das kein aminoterminales bakterielles Signalpeptid aufweist.

5. Zusammensetzung gemäß einem der vorstehenden Ansprüche, wobei das bakterielle ADP-ribosylierende Exotoxin ein hitzelabiles E.-coli-Enterotoxin (LT) ist.

6. Zusammensetzung gemäß einem der vorstehenden Ansprüche, die des Weiteren ein Antigen von Interesse umfasst.

7. Zusammensetzung gemäß einem der Ansprüche 1 bis 5, die des Weiteren eine dritte Nukleinsäuresequenz, die für ein Antigen von Interesse codiert, umfasst.

8. Zusammensetzung gemäß Anspruch 6 oder 7, wobei das Antigen aus einem bakteriellen Pathogen, einem viralen Pathogen, einem parasitischen Pathogen, einem Allergen oder einem Tumor-spezifischen Antigen ist.

9. Zusammensetzung gemäß Anspruch 8, wobei das Antigen aus einem Influenzavirus, Herpes-simplex-Virus (HSV), Hepatitisvirus oder einem Papillomvirus ist.

10. Zusammensetzung gemäß einem der vorstehenden Ansprüche, die des Weiteren ein pharmazeutisch verträgliches Vehikel oder Exzipiens umfasst.

11. Zusammensetzung gemäß einem der vorstehenden Ansprüche, wobei die partikuläre Zusammensetzung für transdermale Abgabe über eine Partikelabgabevorrichtung geeignet ist.

12. Zusammensetzung gemäß einem der vorstehenden Ansprüche, wobei das Core-Trägerpartikel einen mittleren Durchmesser von 0,1 bis 10 µm aufweist.

13. Zusammensetzung gemäß einem der vorstehenden Ansprüche, wobei das Core-Trägerpartikel ein Metall umfaßt.

14. Zusammensetzung gemäß Anspruch 13, wobei das Metall Gold ist.

15. Adjuvanszusammensetzung gemäß einem der Ansprüche 1 bis 5 für die Verwendung bei der Verstärkung einer Immunreaktion bei einem Vertebraten-Subjekt gegen ein Antigen von Interesse durch Verabreichung des Antigens von Interesse und der Zusammensetzung an das Subjekt.

16. Adjuvanszusammensetzung gemäß Anspruch 15, wobei das Antigen von Interesse und die Zusammensetzung an der gleichen Stelle bei dem Subjekt verabreicht werden.

17. Adjuvanszusammensetzung gemäß Anspruch 15 oder 16, wobei das Antigen von Interesse und die Zusammensetzung gleichzeitig verabreicht werden.

18. Adjuvanszusammensetzung gemäß Anspruch 15, wobei die Zusammensetzung des Weiteren das Antigen von Interesse umfasst.

19. Adjuvanszusammensetzung gemäß Anspruch 15, wobei die Zusammensetzung eine dritte Nukleinsäuresequenz umfasst, die für das Antigen von Interesse codiert.

20. Adjuvanszusammensetzung gemäß einem der Ansprüche 15 bis 19, wobei das Antigen von Interesse aus einem bakteriellen Pathogen, einem viralen Pathogen, einem parasitischem Pathogen, einem Allergen oder einem Tumor-spezifischen Antigen ist.

21. Adjuvanszusammensetzung gemäß Anspruch 20, wobei das Antigen aus einem Influenzavirus, Herpes-simplex-Virus (HSV), Hepatitisvirus oder einem Papillomvirus ist.

22. Adjuvanszusammensetzung gemäß einem der Ansprüche 15 bis 21, wobei die Core-Trägerpartikel dem Subjekt unter Verwendung einer Partikel-vermittelten Abgabetechnik verabreicht werden.

23. Adjuvanszusammensetzung gemäß einem der Ansprüche 15 bis 22, wobei das Subjekt menschlich ist.

24. Erzeugnisse, enthaltend eine Zusammensetzung gemäß einem der Ansprüche 1 bis 5, und ein Antigen von Interesse, als eine kombinierte Zubereitung für die simultane, getrennte oder sequentielle Verwendung zum Verstärken einer Immunreaktion bei einem Vertebraten-Subjekt gegen das Antigen.

25. Partikelabgabevorrichtung zur transdermalen Abgabe, die mit einer partikulären Zusammensetzung, wie in einem der Ansprüche 1 bis 13 definiert, geladen ist.

26. Vorrichtung gemäß Anspruch 25, die eine nadellose Spritze ist.

27. Hermetisch verschlossener Einzel-Einheitsdosierungs- oder Mehrfachdosis-Behälter, der für die Verwendung in einer Partikelabgabevorrichtung angepasst ist, wobei der Behälter eine Adjuvanszusammensetzung gemäß einem der Ansprüche 1 bis 13 umfasst.

28. Verwendung einer ersten und zweiten Nukleinsäuresequenz, wie in einem der Ansprüche 1 bis 5 definiert, zur Herstellung eines Medikaments zum Verstärken einer Immunreaktion bei einem Vertebraten-Subjekt gegen ein Antigen von Interesse durch Verabreichung des Antigens von Interesse und des Medikaments an das Subjekt.

## Revendications

1. Composition adjuvante comprenant : (i) une première séquence d'acides nucléiques ; (ii) une seconde séquence d'acides nucléiques ; et (iii) des supports formant noyaux, composition dans laquelle : (a) ladite première séquence d'acides nucléiques est une région codant pour une sous-unité A tronquée obtenue à partir ou dérivant d'une exotoxine ADP-ribosylante bactérienne ; (b) ladite seconde séquence d'acides nucléiques est une région codant pour une sous-unité B tronquée obtenue à partir ou dérivant d'une exotoxine ADP-ribosylante bactérienne ; (c) lesdites première et seconde séquences d'acides nucléiques sont fixées sur les supports formant noyaux et sont liées de manière opérationnelle, ou sont chacune liées de manière opérationnelle, à un promoteur actif dans une cellule mammalienne ; et (d) chacune desdites régions codant pour une sous-unité tronquée présente une délétion en 5', code pour un peptide de sous-unité n'ayant pas de peptide signal bactérien amino-terminal et est liée de manière opérationnelle à une séquence de tête en vue d'une sécrétion depuis une cellule mammalienne.

2. Composition selon la revendication 1, dans laquelle la région codant pour une sous-unité A tronquée a en outre été génétiquement modifiée de manière à supprimer un motif KDEL ou RDEL C-terminal dans le peptide de sous-unité qu'elle code.

3. Composition adjuvante comprenant : (i) une première séquence d'acides nucléiques ; (ii) une seconde séquence d'acides nucléiques ; et (iii) des supports formant noyaux, composition dans laquelle : (a) ladite première séquence d'acides nucléiques est une région codant pour une sous-unité A modifiée obtenue à partir ou dérivant d'une exotoxine ADP-ribosylante bactérienne ; (b) ladite seconde séquence d'acides nucléiques est une région codant pour une sous-unité B obtenue à partir ou dérivant d'une exotoxine ADP-ribosylante bactérienne ; (c) lesdites première et seconde séquences d'acides nucléiques sont fixées sur les supports formant noyaux et sont liées de manière opérationnelle à, ou sont chacune liées de manière opérationnelle à, un promoteur actif dans une cellule mammalienne ; et (d) ladite région codant pour une sous-unité A modifiée et ladite région codant pour une sous-unité B codent chacune pour un peptide de sous-unité mature, étant entendu en outre que la région codant pour une sous-unité A modifiée a été génétiquement modifiée de manière à supprimer un motif KDEL ou RDEL C-terminal dans le peptide de sous-unité qu'elle code et est liée de manière opérationnelle à une séquence de tête en vue d'une sécrétion depuis une cellule mammalienne.

4. Composition selon la revendication 3, dans laquelle la région codant pour une sous-unité A modifiée et la région codant pour une sous-unité B ont chacune été tronquées par une délétion en 5', suite à laquelle chacune desdites régions codant pour une sous-unité tronquée code pour un peptide de sous-unité n'ayant pas de peptide signal bactérien amino-terminal.

5. Composition selon l'une quelconque des revendications précédentes, dans laquelle l'exotoxine. ADP-ribosylante bactérienne est une entérotoxine thermolabile de *E. coli* (LT).

6. Composition selon l'une quelconque des revendications précédentes, comprenant en outre un antigène d'intérêt.

7. Composition selon l'une quelconque des revendications 1 à 5, comprenant en outre une troisième séquence d'acide nucléique codant pour un antigène d'intérêt.

8. Composition selon la revendication 6 ou 7, dans laquelle l'antigène est sélectionné parmi un pathogène bactérien, un pathogène viral, un pathogène parasitaire, un allergène et un antigène spécifique d'une tumeur.

9. Composition selon la revendication 8, dans laquelle l'antigène est sélectionné parmi un virus influenza, le virus de l'herpès simplex (HSV), le virus d'une hépatite et un papillomavirus.

10. Composition selon l'une quelconque des revendications précédentes, comprenant en outre un véhicule ou excipient pharmaceutiquement acceptable.

11. Composition selon l'une quelconque des revendications précédentes, dans laquelle la composition particulaire convient pour l'administration transdermique via un dispositif d'administration de particules.

12. Composition selon l'une quelconque des revendications précédentes, dans laquelle la particule support formant noyau a un diamètre moyen compris entre 0,1 et 10 µm.

13. Composition selon l'une quelconque des revendications précédentes, dans laquelle la particule support formant noyau comprend un métal.

14. Composition selon la revendication 13, dans laquelle le métal est l'or.

15. Composition adjuvante selon l'une quelconque des revendications 1 à 5, en vue d'une utilisation permettant d'améliorer une réponse immunitaire chez un sujet vertébré vis-à-vis d'un antigène d'intérêt en administrant l'antigène d'intérêt et la composition au sujet.

16. Composition adjuvante selon la revendication 15, dans laquelle l'antigène d'intérêt et la composition sont administrés au même endroit chez le sujet.

17. Composition adjuvante selon la revendication 15 ou 16, dans laquelle l'antigène d'intérêt et la composition sont administrés simultanément.

18. Composition adjuvante selon la revendication 15, celle-ci comprenant en outre l'antigène d'intérêt.

19. Composition adjuvante selon la revendication 15, celle-ci comprenant une troisième séquence d'acides nucléiques codant pour l'antigène d'intérêt.

20. Composition adjuvante selon l'une quelconque des revendications 15 à 19, dans laquelle l'antigène d'intérêt est sélectionné parmi un pathogène bactérien, un pathogène viral, un pathogène parasitaire, un allergène et un antigène spécifique d'une tumeur.

21. Composition adjuvante selon la revendication 20, dans laquelle l'antigène est sélectionné parmi un virus influenza, le virus de l'herpès simplex (HSV), le virus d'une hépatite et un papillomavirus.

22. Composition adjuvante selon l'une quelconque des revendications 15 à 21, dans laquelle les particules d'un support formant noyau sont administrées au sujet en utilisant une technique d'administration médiée par des particules.

23. Composition adjuvante selon l'une quelconque des revendications 15 à 22, dans laquelle le sujet est humain.

24. Produits contenant une composition selon l'une quelconque des revendications 1 à 5 et un antigène d'intérêt sous la forme d'une préparation combinée en vue d'une utilisation simultanée, séparée ou successive permettant d'améliorer une réponse immunitaire chez un sujet vertébré vis-à-vis dudit antigène.

25. Dispositif d'administration de particules pour administration transdermique, qui est chargé d'une composition particulaire telle que définie dans l'une quelconque des revendications 1 à 13.

26. Dispositif selon la revendication 25, qui est une seringue sans aiguille.

27. Récipient à dose unitaire individuelle ou multidose hermétiquement fermé, adapté à une utilisation dans un dispositif d'administration de particules, ledit récipient comprenant une composition adjuvante selon l'une quelconque des revendications 1 à 13.

28. Utilisation d'une première et d'une seconde séquences d'acides nucléiques telles que définies dans l'une quelconque des revendications 1 à 5, pour la fabrication d'un médicament permettant d'améliorer une réponse immunitaire chez un sujet vertébré vis-à-vis d'un antigène d'intérêt par administration de l'antigène d'intérêt et du médicament au sujet.
